# EUROPEAN PATENT APPLICATION

(11) **EP 4 238 466 A1**
(43) Date of publication of application: **06.09.2023**
(21) Application number: 21885594.8
(22) Date of filing: 31.05.2021
(51) Int. Cl.: A47K 10/48

(54) **HAND-DRYING APPARATUS AND HAND-DRYING SYSTEM**

(30) Priority: 28.10.2020 WO PCT/JP2020/040489
(71) Applicant: MITSUBISHI ELECTRIC CORPORATION, Chiyoda-ku Tokyo 100-8310 (JP)
(72) Inventor: ASAHI, Yohei, Tokyo 100-8310 (JP); SHIMIZU, Eigo, Tokyo 100-8310 (JP); TAKANO, Koshiro, Tokyo 100-8310 (JP); NAGATA, Shigeyuki, Tokyo 100-8310 (JP); KOBAYASHI, Takaki, Tokyo 100-8310 (JP); SAWABE, Kenji, Tokyo 100-8310 (JP); FUKANO, Manabu, Tokyo 100-8310 (JP); KASHIWABARA, Hideaki, Tokyo 100-8310 (JP); SHIGA, Akira, Tokyo 100-8310 (JP)
(74) Representative: Pfenning, Meinig & Partner mbB
(86) International application number: PCT/JP2021/020698
(87) International publication number: WO 2022/091470

(57) **Abstract**

A hand dryer (1) includes a hand insertion section (3), a blower (10), and a UV irradiator (23). The hand insertion section (3) defines a hand insertion space (5). The hand insertion section (3) has an air hole (6) that opens to the hand insertion space (5). The blower (10) is in fluid communication with the air hole (6). The blower (10) is configured to blow air from the air hole (6) into the hand insertion space (5) or to suck air from the hand insertion space (5) into the air hole (6). The UV irradiator (23) irradiates the hand insertion space (5) with ultraviolet light generated by a light source.

## Description

### Field

The present disclosure relates to a hand dryer and a hand-drying system. Background

Hand dryers which dry hands by jetting an airflow onto wet hands after hand washing are being widely used (for example, refer to PTL 1).

### Citation List

### Patent Literature

[PTL 1] Japanese Patent No. 6552749

### Summary

### Technical Problem

When removing water adhering to a hand by an airflow, there is a possibility that water droplets containing bacteria or viruses may scatter inside an enclosure of a hand dryer or scatter to the outside of the enclosure. In addition, there is a possibility that such water droplets adhering to the inside of the enclosure may scatter to the outside of the enclosure when the hand dryer is used by a next person and an airflow is jetted.

The present disclosure has been made in order to solve such problems and an object thereof is to improve hygiene of a hand dryer and a hand-drying system.

### Solution to Problem

A hand dryer according to the present disclosure, comprises: a hand insertion section defining a hand insertion space and having an air hole that opens to the hand insertion space; a blower in fluid communication with the air hole, the blower being configured to blow air from the air hole into the hand insertion space or configured to suck air from the hand insertion space into the air hole; and a UV irradiator including a light source, the UV irradiator being configured to irradiate the hand insertion space with ultraviolet light produced by the light source.

Also, a hand-drying system according to the present disclosure, comprises: the hand dryer being arranged in a user region that is a region which a user enters; and presence/absence detection means for detecting whether or not a person is present in the user region.

Also, a hand-drying system according to the present disclosure, comprises: the hand dryer; a cover section capable of covering at least one face of the hand insertion space of the hand dryer; a switching section capable of switching between a closed state where the cover section covers the at least one face of the hand insertion space and an open state where a hand can be inserted into the hand insertion space; and proximity detection means capable of detecting that a hand or a person is in proximity to the hand insertion section in the closed state, the hand-drying system being configured to enter the open state when the proximity detection means detects that a hand or a person is in proximity to the hand insertion section.

Also, a hand-drying system according to the present disclosure, comprises: the hand dryer being arranged in a user region being a region which a user enters; and human count detection means for detecting a number of people in the user region, the UV irradiator being configured to emit the ultraviolet light when the number of people is zero, the hand-drying system being configured to stop emission of the ultraviolet light by the UV irradiator when the number of people is one or more.

Also, a hand-drying system according to the present disclosure, comprises: the hand dryer; human recognition means for recognizing a same user; human storage means for counting a use frequency by a same user; and output variable means which changes an output of emission of the ultraviolet light by the UV irradiator in accordance with a daily use frequency by a same user.

Also, a hand-drying system according to the present disclosure, comprises: the hand dryer; and a sterilizer which discharges a sterilizing liquid being a liquid with a sterilization effect.

Also, a hand-drying system according to the present disclosure, comprises: the hand dryer in plurality, the hand-drying system cooperatively operating the plurality of hand dryers.

Also, a hand-drying system according to the present disclosure, comprises: the hand dryer; and a terminal used by a manager who manages the hand dryer, the hand-drying system transmitting data detected by the hand dryer to the terminal.

### Advantageous Effects of Invention

According to the present disclosure, hygiene of a hand dryer and a hand-drying system can be improved.

### Brief Description of the Drawings

Fig. 1 is a perspective view showing a hand dryer according to a first embodiment.
Fig. 2 is a front view of the hand dryer according to the first embodiment.
Fig. 3 is a side view of the hand dryer according to the first embodiment.
Fig. 4 is a top view of the hand dryer according to the first embodiment.
Fig. 5 is a side view showing a state where a user is using the hand dryer according to the first embodiment.
Fig. 6 is a sectional side view of the hand dryer according to the first embodiment.
Fig. 7 is a perspective view showing a first modification of the hand dryer according to the first embodiment.
Fig. 8 is a perspective view showing a second modification of the hand dryer according to the first embodiment.
Fig. 9 is a diagram of an upper part of the hand dryer according to the first embodiment as viewed by a gaze from a right side toward a left side.
Fig. 10 is a functional block diagram of the hand dryer according to the first embodiment.
Fig. 11 is a diagram showing an example of a hardware configuration of a processing circuit of the hand dryer according to the first embodiment.
Fig. 12 is a diagram of an upper part of the hand dryer according to a modification of the first embodiment as viewed by a gaze from a right side toward a left side.
Fig. 13 is a schematic view for explaining a principle of a mutual-capacitance type capacitance sensor which constitutes a hand detection section of the hand dryer according to the first embodiment.
Fig. 14 is a schematic view for explaining the principle of the mutual-capacitance type capacitance sensor which constitutes the hand detection section of the hand dryer according to the first embodiment.
Fig. 15 is a schematic view for explaining the principle of the mutual-capacitance type capacitance sensor which constitutes the hand detection section of the hand dryer according to the first embodiment.
Fig. 16 is a schematic view for explaining the principle of the mutual-capacitance type capacitance sensor which constitutes the hand detection section of the hand dryer according to the first embodiment.
Fig. 17 is a perspective view of a UV irradiator included in the hand dryer according to the first embodiment.
Fig. 18 is an exploded perspective view of the UV irradiator included in the hand dryer according to the first embodiment.
Fig. 19 is a front view of the UV irradiator included in the hand dryer according to the first embodiment.
Fig. 20 is a rear view of the UV irradiator included in the hand dryer according to the first embodiment.
Fig. 21 is a sectional view of the UV irradiator included in the hand dryer according to the first embodiment taken along line B-B in Fig. 19.
Fig. 22 is a sectional view of the UV irradiator included in the hand dryer according to the first embodiment.
Fig. 23 is a sectional view showing a modification of the UV irradiator included in the hand dryer according to the first embodiment.
Fig. 24 is a sectional view showing another modification of the UV irradiator included in the hand dryer according to the first embodiment.
Fig. 25 is a sectional view showing another modification of the UV irradiator included in the hand dryer according to the first embodiment.
Fig. 26 is a sectional view showing another modification of the UV irradiator included in the hand dryer according to the first embodiment.
Fig. 27 is a sectional view showing another modification of the UV irradiator included in the hand dryer according to the first embodiment.
Fig. 28 is a sectional view of a reflection section.
Fig. 29 is a perspective view showing an example of an assembly corresponding to another aspect of the reflection section.
Fig. 30 is a development view showing a sheet prior to three-dimensionally assembling the assembly 37 shown in Fig. 29.
Fig. 31 is a development view showing another example of an assembly sheet.
Fig. 32 is a diagram schematically showing an example of a structure for maintaining a state where the assembly sheet has been assembled as the assembly.
Fig. 33 is a diagram schematically showing an example of a structure for maintaining a state where the assembly sheet has been assembled as the assembly.
Fig. 34 is a development view showing a modification of the assembly sheet shown in Fig. 31.
Fig. 35 is a schematic sectional view of a hand dryer according to a second embodiment.
Fig. 36 is a side view of an upper part of a hand dryer according to a third embodiment.
Fig. 37 is a side view of an upper part of a hand dryer according to a fourth embodiment.
Fig. 38 is a side view of an upper part of a hand dryer according to a fifth embodiment.
Fig. 39 is a schematic top view of a hand dryer according to a sixth embodiment.
Fig. 40 is a side view of a hand dryer according to a seventh embodiment.
Fig. 41 is a perspective view of a hand dryer according to an eighth embodiment.
Fig. 42 is a sectional side view of the hand dryer according to the eighth embodiment.
Fig. 43 is a functional block diagram of a hand dryer according to a ninth embodiment.
Fig. 44 is a functional block diagram of a hand dryer according to a tenth embodiment.
Fig. 45 is a functional block diagram of a hand-drying system according to an eleventh embodiment.
Fig. 46 is a flowchart showing an example of processing to be executed by the hand-drying system according to the eleventh embodiment.
Fig. 47 is a functional block diagram of a hand-drying system according to a twelfth embodiment.
Fig. 48 is a flowchart showing an example of processing to be executed by the hand-drying system according to the twelfth embodiment.
Fig. 49 is a functional block diagram of a hand-drying system according to a thirteenth embodiment.
Fig. 50 is a flowchart showing an example of processing to be executed by the hand-drying system according to the thirteenth embodiment.
Fig. 51 is a flowchart showing another example of processing to be executed by the hand-drying system according to the thirteenth embodiment.
Fig. 52 is a functional block diagram of a hand-drying system according to a fourteenth embodiment.
Fig. 53 is a functional block diagram of a hand-drying system according to a fifteenth embodiment.
Fig. 54 is a functional block diagram of a hand-drying system according to a sixteenth embodiment.
Fig. 55 is a perspective views of an example of a chloride storage section included in the hand-drying system according to the sixteenth embodiment.
Fig. 56 is a perspective views of the example of the chloride storage section included in the hand-drying system according to the sixteenth embodiment.
Fig. 57 is a top view of the chloride storage section shown in Fig. 56.
Fig. 58 is a side view of the chloride storage section shown in Fig. 56.

### Description of Embodiments

Hereinafter, embodiments will be described with reference to the drawings. Common or corresponding elements in each drawing will be denoted by same reference signs and descriptions thereof will be simplified or omitted. In case where reference is made to an angle in the present disclosure, when there is a major angle and a minor angle whose sum is 360 degrees, an angle being the minor angle will be referred to as a general rule, and when there is an acute angle and an obtuse angle whose sum is 180 degrees, an angle being the acute angle will be referred to as a general rule. In addition, configurations shown in the following embodiments represent examples of technical ideas according to the present disclosure and can be combined with other known techniques, or a plurality of technical ideas described in the present disclosure may be combined with one another. Furthermore, a part of the configurations can be omitted or modified without departing from the spirit and scope of the present disclosure.

### First embodiment

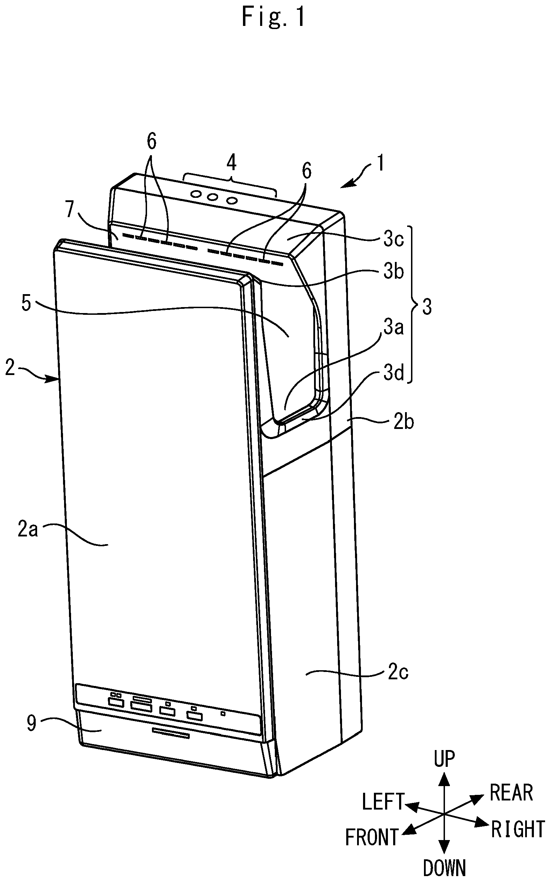

Fig. 1 is a perspective view of a hand dryer 1 according to a first embodiment. In the following description, as a general rule, directions of "front", "rear", "left", "right", "up", and "down" will be specified with respect to the hand dryer 1 as indicated by arrows in Fig. 1. "Front" corresponds to a direction of a near side to a user directly opposing the hand dryer 1. "Rear" corresponds to a direction of a far side to the user. "Left" corresponds to a direction to the left of the user. "Right" corresponds to a direction to the right of the user. "Up" corresponds to vertically upward. "Down" corresponds to vertically downward. In the present embodiment, a "near side", a "front-face side", a "user side", and a "side close to the user" refer to the front side. A "rear-face side", a "far side", a "wall side", and a "side far from the user" refer to the rear side. In addition, the left side and the right side may be collectively referred to as a "lateral side". For example, the front-face side of the hand dryer 1 is the near side in Fig. 1.

In addition, terms in the description below will be defined as follows. A front view refers to a point of view when viewed from a front side toward a rear side. In addition, a front view refers to a point of view when viewed from a front side by a gaze in a direction perpendicular to a left-right direction. Furthermore, a side view refers to a point of view from a left side toward a right side or a point of view from a right side toward a left side. In addition, a side view refers to a point of view when viewed from a left side or a right side by a gaze in a direction perpendicular to a front-rear direction. Furthermore, a bottom view refers to a point of view when viewed from a lower side toward an upper side. In addition, a bottom view refers to a point of view when viewed from a lower side by a gaze in a direction perpendicular to any of a front-rear direction and a left-right direction. Furthermore, a top view refers to a point of view when viewed from an upper side toward a lower side. In addition, a top view refers to a point of view when viewed from an upper side by a gaze in a direction perpendicular to any of a front-rear direction and a left-right direction.

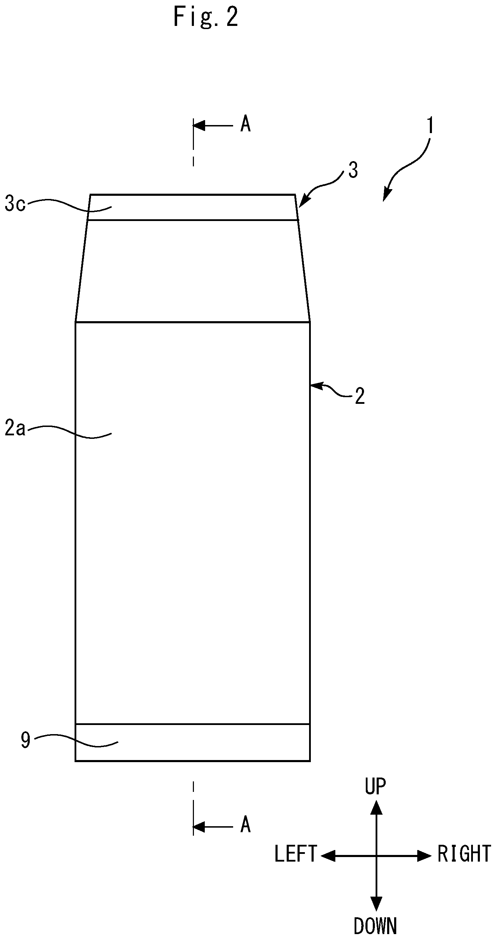

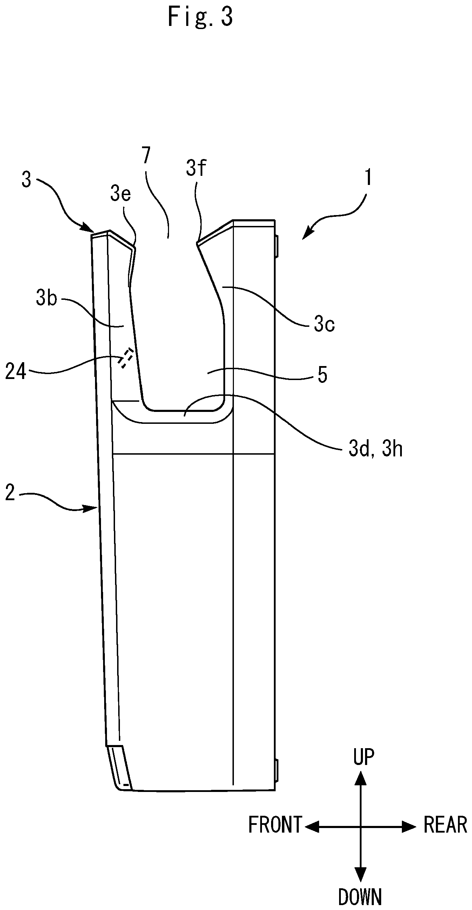

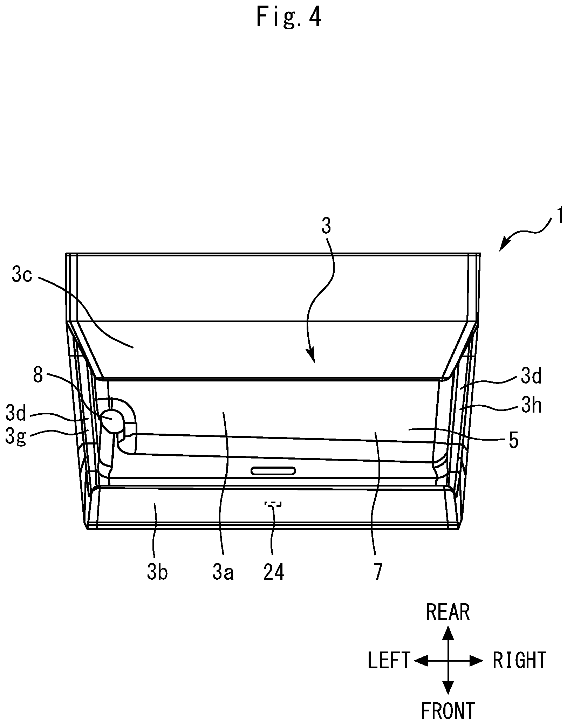

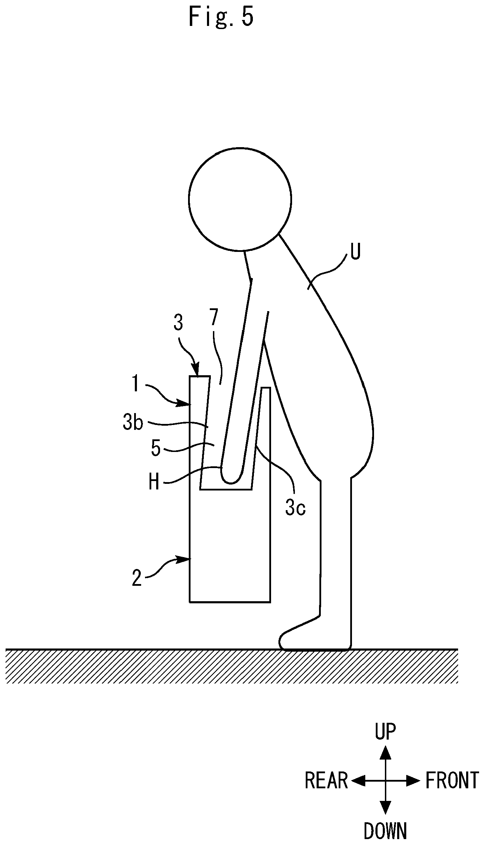

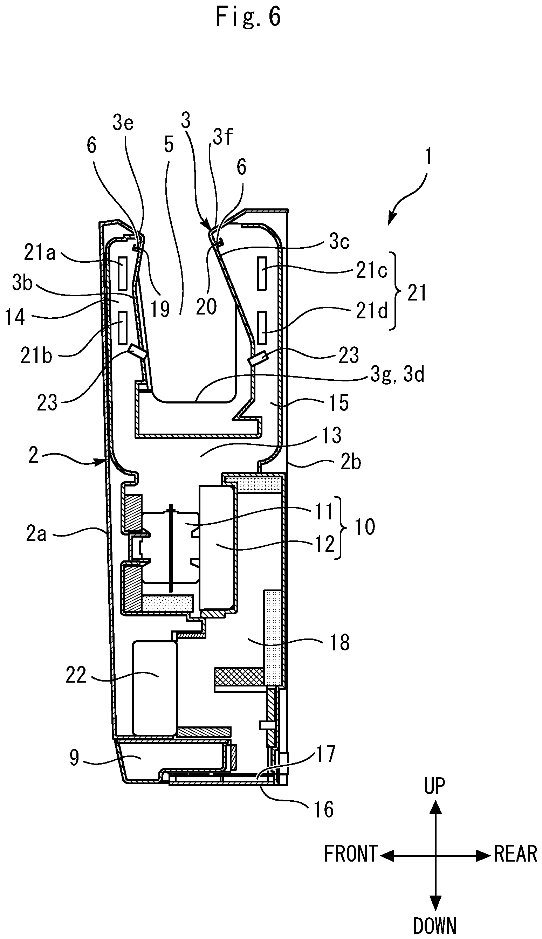

Fig. 2 is a front view of the hand dryer 1 according to the first embodiment. Fig. 3 is a side view of the hand dryer 1 according to the first embodiment. Fig. 4 is a top view of the hand dryer 1 according to the first embodiment. Fig. 5 is a schematic side view showing a state where a user U is using the hand dryer 1 according to the first embodiment. Fig. 6 is a sectional side view of the hand dryer 1 according to the first embodiment and is a sectional view taken along line A-A in Fig. 2.

The hand dryer 1 shown in Figs. 1 to 6 includes a main body enclosure 2 which forms an outer shell and a housed section being housed in the main body enclosure 2. The hand dryer 1 has a function of drying wet hands. The main body enclosure 2 defines an outer appearance of the hand dryer 1. Note that a part of the main body enclosure 2 may be arranged inside the main body enclosure 2 and a part of the housed section may be visible to the outside.

The main body enclosure 2 includes a cover section, a hand insertion section 3, a display section 4, and an operating section. The cover section is a design portion being exposed to the outer appearance. The hand insertion section 3 defines a hand insertion space 5. The hand insertion space 5 is a space into which a hand is to be inserted. The hand insertion space 5 according to the present embodiment is a single space. Both hands of the user U can be inserted into the hand insertion space 5 being a single space. For example, the hand insertion space 5 extends from an upper end to a lower end of the hand insertion section 3. For example, the hand insertion space 5 extends from a front end to a rear end of the hand insertion section 3. For example, the hand insertion space 5 extends from a left end to a right end of the hand insertion section 3. For example, the display section 4 may have a display which displays information to be notified or have an indicator lamp. The operating section is a portion to be operated by a user, a cleaner, or a manager.

The hand insertion section 3 has an air hole 6 that opens to the hand insertion space 5. In the present embodiment, an example in which air is blown from the air hole 6 into the hand insertion space 5 will be described. According to the present embodiment, since water adhering to a hand can be blasted away by an airflow that is blown from the air hole 6, the hand can be dried in a shorter period of time. The hand dryer according to the present disclosure is not limited to this example and may be configured to suck air from the hand insertion space 5 into the air hole 6. In this case, an airflow is generated in the hand insertion space 5 due to air being sucked into the air hole 6 and a hand can be dried by the airflow.

The hand insertion section 3 is arranged in an upper part of the hand dryer 1. The hand insertion section 3 includes an insertion peripheral section and an insertion bottom section 3a. The insertion peripheral section includes an insertion front section 3b, an insertion rear section 3c, and an insertion side section 3d. The insertion peripheral section is the hand insertion section 3 excluding the insertion bottom section 3a. In the hand insertion section 3, the insertion front section 3b is arranged on a front side, the insertion rear section 3c is arranged on a rear side, the insertion side section 3d is arranged on a left side and a right side, and the insertion bottom section 3a is arranged on a front side of the hand insertion section 3 on a lower side.

As modifications, the hand insertion section 3 may not include the insertion side section 3d. In addition, the hand insertion section 3 may not include the insertion front section 3b. Furthermore, the hand insertion section 3 may include an insertion upper section (not illustrated) that is arranged on an upper side. In addition, a conceivable combination of the above may be provided. For example, the hand insertion section 3 may not include the insertion front section 3b but may include an insertion upper section. Alternatively, the hand insertion section 3 may only include the insertion bottom section 3a or only include the insertion upper section. In other words, while insertion generally means "to insert inside", insertion in the present embodiment may include the use of the word "insertion" with respect to an object that only has a structure on one side or one face. In addition, for example, while the insertion bottom section 3a and the insertion front section 3b can be more or less clearly distinguished from each other in the present embodiment, each wall surface of the hand insertion section 3 need not necessarily be clearly distinguishable. For example, the hand insertion section 3 may be hemispherically formed.

The hand insertion section 3 according to the present embodiment defines the hand insertion space 5 with the insertion bottom section 3a, the insertion front section 3b, the insertion rear section 3c, and the insertion side section 3d. A hand can be arranged in the hand insertion space 5. A length of the hand insertion space 5 in the front-rear direction and a length of the hand insertion space 5 in the left-right direction are lengths that enable a hand to be arranged.

An upper end part of the insertion side section 3d is arranged at a lower position than an upper end part of the insertion front section 3b and an upper end part of the insertion rear section 3c. An hand insertion opening 7 is defined in an upper part of the main body enclosure 2 or an upper part of the hand insertion section 3. The hand insertion space 5 is defined below the hand insertion opening 7 in an upper part of the main body enclosure 2. The hand insertion space 5 is a concave space that covers a hand of a user inserted from the hand insertion opening 7.

The hand insertion section 3 defines the concave hand insertion space 5 by having a roughly U-shape in a side view shown in Fig. 3 or a side view (Fig. 6) in a section taken along line A-A in Fig. 2. As shown in Fig. 5, the user U can insert, downward from above, a hand H into the hand insertion space 5 from the hand insertion opening 7.

In the side view in Fig. 3, a surface of the insertion front section 3b facing the hand insertion space 5 is slightly inclined from an upper part toward a lower part and from a front side toward a rear side. In addition, a surface of the insertion rear section 3c facing the hand insertion space 5 is slightly inclined from an upper part toward a lower part and from a front side toward a rear side.

As shown in Fig. 5, for example, the user U stands in front of the hand dryer 1 and inserts the hand H into the hand insertion space 5. The hand insertion section 3 is installed at a position of at roughly the height of the waist of an adult male or female. Providing the insertion front section 3b and the insertion rear section 3c with the inclines described above enables the hand H to be readily inserted when the user U inserts the hand H into the hand insertion space 5 from above.

In a case of the hand dryer with a different insertion direction of a hand, the hand dryer may be installed at a height that differs from the example shown in Fig. 5. For example, in a case of a hand dryer of a type in which a hand is inserted from a near side toward a far side, the hand insertion section 3 is installed at roughly the height of an elbow of an adult male or female.

The insertion peripheral section has opposing faces. The insertion front section 3b is arranged at a position opposing the insertion rear section 3c. The hand insertion opening 7 is provided above the insertion bottom section 3a. As shown in Fig. 4, the insertion side section 3d includes an insertion left section 3g and an insertion right section 3h. The insertion left section 3g is arranged on a left side of the insertion front section 3b in a front view. The insertion right section 3h is arranged on a right side of the insertion front section 3b in a front view. The insertion left section 3g is arranged at a position opposing the insertion right section 3h.

As shown in Fig. 3, the insertion front section 3b includes a front projecting section 3e. The insertion rear section 3c includes a rear projecting section 3f. The front projecting section 3e is a portion that is projected rearward from a front-face side or a side close to the user or, in other words, from the insertion front section 3b. The rear projecting section 3f is a portion that is projected forward from a rear-face side or a side far from the user or, in other words, from the insertion rear section 3c. The front projecting section 3e is provided on an upper side among the insertion front section 3b. The rear projecting section 3f is provided on an upper side among the insertion rear section 3c.

The insertion front section 3b and the insertion rear section 3c are connected to the insertion bottom section 3a provided in a lowermost part of the hand insertion section 3. In this manner, the hand insertion section 3 has a bottomed cross-sectional U-shape with an open top part in a side view or a side view in an A-A cross-section. The insertion bottom section 3a may be inclined with respect to a horizontal plane. A surface facing the hand insertion space 5 of the insertion front section 3b and the insertion rear section 3c may have a folded shape or a curved shape in a side view.

An upper end part of the insertion side section 3d is arranged at a position closer to the insertion bottom section 3a than the upper end part of the insertion front section 3b and the upper end part of the insertion rear section 3c. The upper end part of the insertion side section 3d is arranged at a position closer to a lowermost part of the insertion bottom section 3a than the upper end part of the insertion front section 3b and the upper end part of the insertion rear section 3c. As described above, since the insertion side section 3d is arranged at a low position, both sides in the left-right direction of the hand insertion section 3 are opened. The hand insertion section 3 according to the present embodiment configured as described above enables a hand of a user to be freely inserted into and withdrawn from the hand insertion space 5 from an up-down direction or a left-right direction.

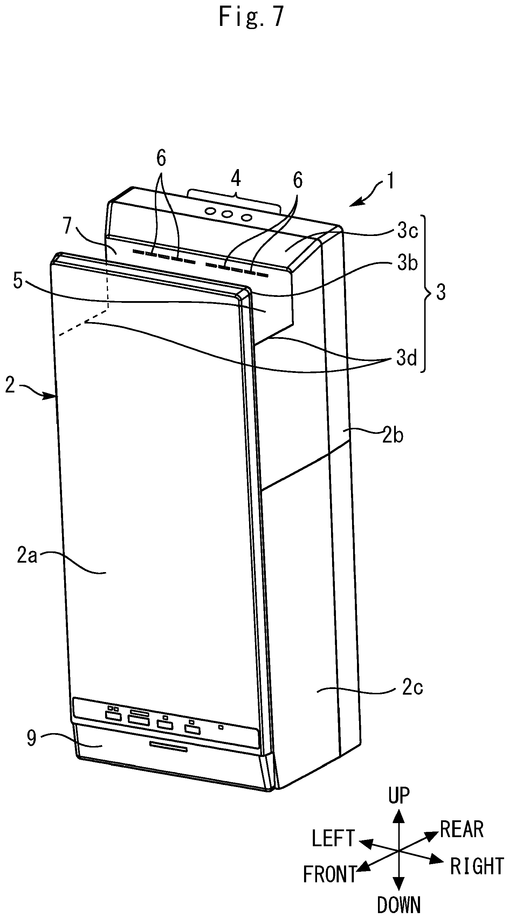

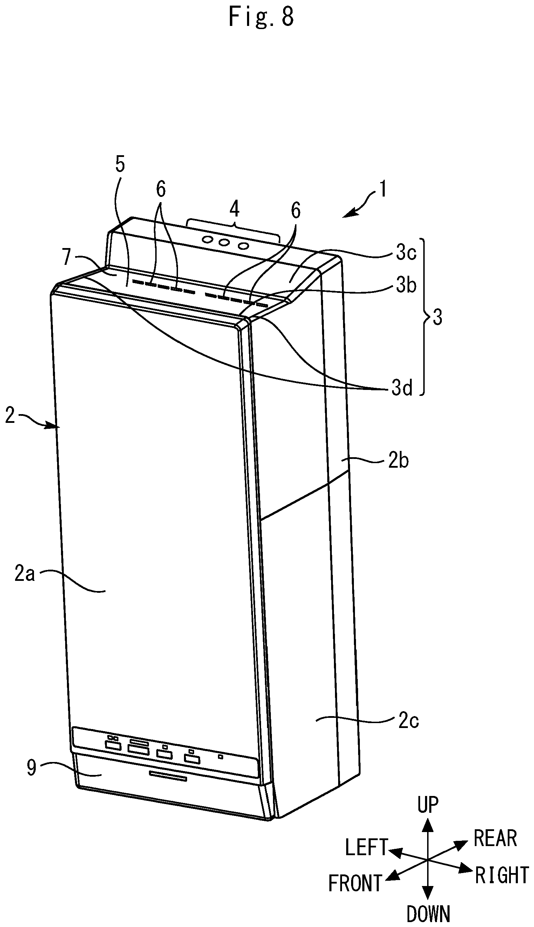

Modifications of the hand dryer 1 according to the first embodiment will now be described. Fig. 7 is a perspective view showing a first modification of the hand dryer 1 according to the first embodiment. Fig. 8 is a perspective view showing a second modification of the hand dryer 1 according to the first embodiment. As shown in the modifications, the hand insertion section 3 may have a box-like shape of which an upper face is opened. As shown in Figs. 7 and 8, a structure may be adopted in which the upper end part of the insertion side section 3d is arranged at a position closer to the upper end part of the insertion front section 3b and the upper end part of the insertion rear section 3c than an upper end part or a lower end part of the insertion bottom section 3a. In this case, water can be prevented from scattering in the left-right direction. In addition, as shown in Fig. 8, an upper end of the insertion side section 3d may be arranged at a height equivalent to an upper end of the insertion front section 3b or the upper end of the insertion side section 3d. Furthermore, when the height of the upper end of the insertion front section 3b differs from a height of an upper end of the insertion rear section 3c, an upper face of the insertion side section 3d may be inclined. The insertion front section 3b and the insertion side section 3d are desirably connected to each other. The insertion side section 3d and the insertion rear section 3c are desirably connected to each other. Accordingly, water does not leak to the outside from a gap between the insertion front section 3b and the insertion side section 3d. When the hand dryer 1 is viewed in a horizontal direction at a position lower than upper parts of the insertion front section 3b, the insertion rear section 3c, and the insertion side section 3d, the hand insertion space 5 is covered by the hand insertion section 3. Therefore, the hand insertion space 5 cannot be visually recognized. When the height of the insertion side section 3d is high such as when the height of the insertion side section 3d is equivalent to the height of the insertion front section 3b or the insertion rear section 3c, the hand insertion space 5 cannot be visually recognized even when viewed from both horizontal directions.

The height of the upper end of the insertion front section 3b may be lower than the height of the upper end of the insertion rear section 3c. When the height of the upper end of the insertion rear section 3c and the height of the upper end of the insertion front section 3b are equivalent heights, the hand insertion opening 7 is parallel to a horizontal plane in a side view. On the other hand, when the height of the upper end of the insertion front section 3b is lower than the height of the upper end of the insertion rear section 3c as described above, inclining the hand insertion opening 7 with respect to a horizontal plane in a side view causes the hand insertion opening 7 to face a direction of a user. Accordingly, the user can more readily insert a hand.

In addition, the hand dryer 1 may include insertion side section adjustment means capable of adjusting the height of the upper end of the insertion side section 3d. For example, as the insertion side section adjustment means, a mechanism that makes the insertion side section 3d movable so that the insertion side section 3d slides in a vertical direction may be provided. Alternatively, as the insertion side section adjustment means, the insertion side section 3d may be configured so that the insertion side section 3d can be detached and re-attached at a position of a different height. A structure that enables the insertion side section 3d to be detached and re-attached may be a structure using fixing by screws or a structure using snap fit which does not use screws. The insertion side section adjustment means may be capable of adjusting the height of the upper end of the insertion side section 3d in stages. For example, the insertion side section adjustment means may be capable of adjusting the height of the upper end of the insertion side section 3d in three stages of "high", "medium", and "low". "High" may correspond to a height such as that shown in Fig. 8, "medium" may correspond to a height such as that shown in Fig. 7, and "low" may correspond to a height such as that shown in Fig. 1. Alternatively, the insertion side section adjustment means may be capable of adjusting the height of the upper end of the insertion side section 3d in a stepless manner. Reducing the height of the upper end of the insertion side section 3d has an advantage of making it easy for the user to insert a hand into the hand insertion space 5 from a side of the hand insertion section 3. Increasing the height of the upper end of the insertion side section 3d has an advantage of making it difficult for water droplets blasted off a hand from being further scattered to the outside of the hand insertion section 3. Changing the height of the upper end of the insertion side section 3d using the insertion side section adjustment means in accordance with the season or a risk of infection is more advantageous in terms of realizing both safety and convenience.

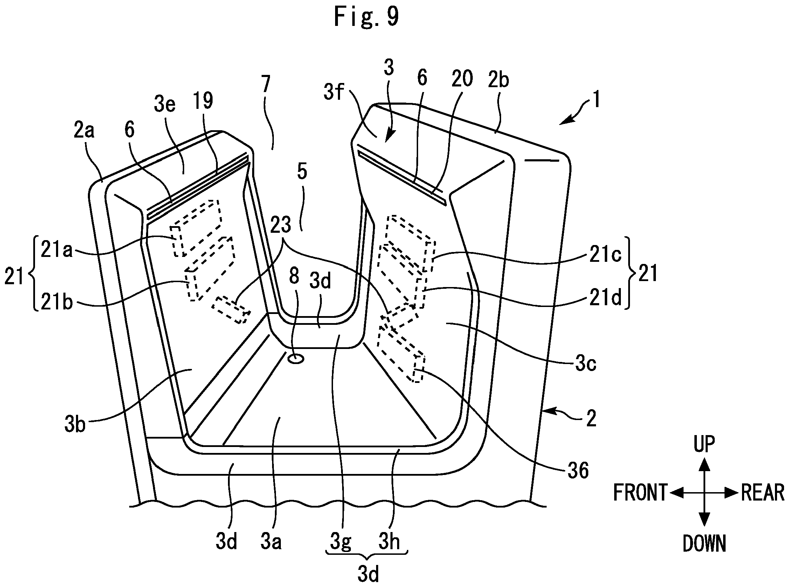

Fig. 9 is a diagram of an upper part of the hand dryer 1 according to the first embodiment as viewed by a gaze from a right side toward a left side. Fig. 9 shows a partially-changed scale for the purpose of facilitating understanding of the configuration of the hand dryer 1. Fig. 9 is expressed using a perspective method.

As shown in Fig. 9, a part of the insertion bottom section 3a is provided with a drain outlet 8 for discharging water in the insertion bottom section 3a. For example, the drain outlet 8 is provided at a low position among the insertion bottom section 3a. Accordingly, water is less likely to accumulate in the insertion bottom section 3a and is readily discharged. In other words, the insertion bottom section 3a may be inclined toward the drain outlet 8. For example, the drain outlet 8 may be provided in plurality. An upper end part of a drainage path (not illustrated) which extends in an up-down direction in the main body enclosure 2 is mounted to the drain outlet 8. A drain tank 9 arranged in a bottom part of the main body enclosure 2 is connected to a lower end part of the drainage path. The drain tank 9 is for accumulating water having been drained through the drainage path. The drain tank 9 is attachably and detachably mounted to the bottom part of the main body enclosure 2. For example, the drain tank 9 can be detached from the hand dryer 1 by pulling the bottom part of the main body enclosure 2 toward the front. In other words, a part of the drain tank 9 is exposed to the outer appearance. The drain outlet 8 is provided with a gradient so that water flows downward, and water adhering to the insertion bottom section 3a flows through the drainage path and accumulates in the drain tank 9.

For example, the hand insertion section 3 may be constructed using a material such as an acrylonitrile butadiene styrene copolymerized resin (ABS resin) or polypropylene. For example, a major portion of the hand insertion section 3 may be constructed using a material such as an ABS resin or polypropylene. For example, a portion defining the outer appearance of the hand insertion section 3 may be constructed using a material such as an ABS resin or polypropylene.

As shown in Fig. 1, the cover section of the main body enclosure 2 includes a front cover 2a, a rear cover 2b, and a side cover 2c. The front cover 2a is arranged in the front. The rear cover 2b is arranged in the rear. The side cover 2c is arranged to the rear of the front cover 2a but to the front of the rear cover 2b. The front cover 2a is arranged to the front of the rear cover 2b. The front cover 2a is arranged to the front of the insertion front section 3b. The rear cover 2b is arranged to the rear of the insertion rear section 3c. The side cover 2c is arranged to the side. The side cover 2c is arranged to the side of the front cover 2a and the rear cover 2b. The cover section of the main body enclosure 2 is arranged on an outer side of the hand insertion section 3. In the present embodiment, at least a part of the housed section is arranged between the cover section and the hand insertion section 3.

As shown in Fig. 6, inside the main body enclosure 2, a blower 10 that generates a high-speed airflow is installed below the hand insertion section 3. The blower 10 is constituted of a high-pressure airflow generator including an electrically-actuated motor 11 and a turbo fan 12 that is rotated by the motor 11. The blower 10 is arranged so that an intake side constitutes a rear face and an exhaust side constitutes a front face. Alternatively, the blower 10 may be arranged so that an intake side constitutes a lower face and an exhaust side constitutes an upper face. The motor 11 is, for example, a brushless motor. Using a brushless motor enables output to be readily adjusted.

The hand dryer 1 includes a blowout port section, a blowout air path, an air inlet 16, and an inlet air path 18. For example, the blowout port section blows out air from the air hole 6 of the hand insertion section 3. For example, the blowout port section is constituted of a plurality of nozzles. The blowout port section according to the present embodiment includes a first blowout port section 19 and a second blowout port section 20. The nozzle has a hollow cylindrical shape or a hollow square columnar shape. The nozzle may be bent. A tip of the nozzle has a circular or rectangular shape. For example, the first blowout port section 19 is arranged in the insertion front section 3b. For example, blowout air from the first blowout port section 19 is blown out so that the air reaches the insertion rear section 3c if the air does not strike a hand. For example, the second blowout port section 20 is arranged in the insertion rear section 3c. For example, blowout air from the second blowout port section 20 is blown out so that the air reaches the insertion front section 3b if the air does not strike a hand. The first blowout port section 19 is arranged to the front of the second blowout port section 20. While the blowout port section is provided in plurality such as the first blowout port section 19 and the second blowout port section 20 in the present embodiment, the hand dryer according to the present disclosure may only include one blowout port section. For example, only the second blowout port section 20 may be provided without providing the first blowout port section 19. In addition, the first blowout port section 19 may have only one air hole 6 or may include a plurality of the air holes 6. Furthermore, the second blowout port section 20 may have only one air hole 6 or may include a plurality of the air holes 6. In addition, the hand dryer according to the present disclosure may include three or more blowout port sections and may include, for example, a third blowout port section.

A blowout air path 13 is provided on an exhaust side of the blower 10. The blowout air path 13 is an air path which guides an airflow generated by the blower 10 to the blowout port section. The blowout port section and the blowout air path 13 may include one air path or a plurality of air paths. In the example shown in Fig. 6, the blowout air path 13 includes a front blowout air path 14 and a rear blowout air path 15. The front blowout air path 14 is arranged to the front of the rear blowout air path 15. For example, at least a part of the blowout air path 13 is formed along the up-down direction. For example, a major portion of the blowout air path 13 is formed along the up-down direction.

The air inlet 16 is an opening for taking in air outside the hand dryer 1 into the hand dryer 1. For example, the air inlet 16 is formed in a grid shape or a slit shape. For example, the air inlet 16 is provided in the bottom part of the main body enclosure 2. The air inlet 16 may be provided with a filter section 17. For example, the filter section 17 may be arranged inside the main body enclosure 2 and in a vicinity of the air inlet 16. Accordingly, outside air can be taken into the inlet air path 18 through the filter section 17 while preventing penetration by foreign objects. The inlet air path 18 is provided on an exhaust side of the blower 10. The inlet air path 18 is an air path which fluidically connects the blower 10 and the air inlet 16 to each other.

The inlet air path 18 is defined on a rear-face side and formed in the up-down direction inside the main body enclosure 2. An upper part of the inlet air path 18 is in fluid communication with the blower 10. A lower part of the inlet air path 18 is in fluid communication with the air inlet 16. A part of the inlet air path 18 is arranged on a rear side of the drain tank 9.

An exhaust-side of the blower 10 communicates with a portion below the front blowout air path 14 and the rear blowout air path 15 which are successively arranged in the up-down direction and which are defined so as to branch toward a front-face side and a rear-face side inside the main body enclosure 2. High-pressure air having been pressurized by the blower 10 is discharged to the front blowout air path 14 and the rear blowout air path 15 provided connected to the blower 10. A configuration may be adopted in which a heater is built into the front blowout air path 14 and the rear blowout air path 15 at a position before the branch to the front-face side and the rear-face side in order to raise a temperature of passing high-pressure air.

The first blowout port section 19 is provided in an upper part of the front blowout air path 14. The second blowout port section 20 is provided in an upper part of the rear blowout air path 15. The first blowout port section 19 is provided in the front projecting section 3e on an upper side of the insertion front section 3b. The second blowout port section 20 is provided in the rear projecting section 3f on an upper side of the insertion rear section 3c. The first blowout port section 19 and the second blowout port section 20 oppose each other across the hand insertion space 5. The first blowout port section 19 and the second blowout port section 20 may include a plurality of air holes 6 opened facing obliquely downward in a slight wave shape. Each air hole 6 may be horizontally arranged in a single row along the left-right direction of the hand dryer 1.

The first blowout port section 19 and the second blowout port section 20 convert high-pressure air generated by the blower 10 into a high-speed airflow and jet the high-speed airflow from the air hole 6 toward the hand insertion space 5 as an operating air flow. The operating air flow is jetted from the first blowout port section 19 and the second blowout port section 20 in a direction opposing the hand insertion space 5 at an angle inclined slightly downward relative to the horizontal direction. The operating air flow blasts, downward in the hand insertion space 5, water adhering to a wrist, a palm of a hand, or a back of a hand inserted by the user into the hand insertion space 5.

As shown in Figs. 6 and 9, a hand detection section 21 is built into the insertion front section 3b and the insertion rear section 3c. The hand detection section 21 is an example of hand detection means. Preferably, the hand detection section 21 is built into a position lower than the first blowout port section 19 and a position lower than the second blowout port section 20. As the user causes a wet hand to enter the hand insertion opening 7 and move toward a far side of the hand insertion space 5, the hand detection section 21 detects the inserted hand. When the hand detection section 21 detects that a hand of the user has been inserted into the hand insertion space 5 as described above, the hand detection section 21 outputs a hand detection signal to the effect that a hand of the user has been detected to a control section 22 to be described later. Details of the hand detection section 21 will be provided later.

As shown in Fig. 6, the control section 22 which controls an operation of the blower 10 in accordance with a detection of a hand by the hand detection section 21 is provided in a lower portion inside the main body enclosure 2. The control section 22 controls the operation of the blower 10 based on information in a hand detection signal output from the hand detection section 21 and causes an airflow to be jetted into the hand insertion space 5 from the first blowout port section 19 and the second blowout port section 20.

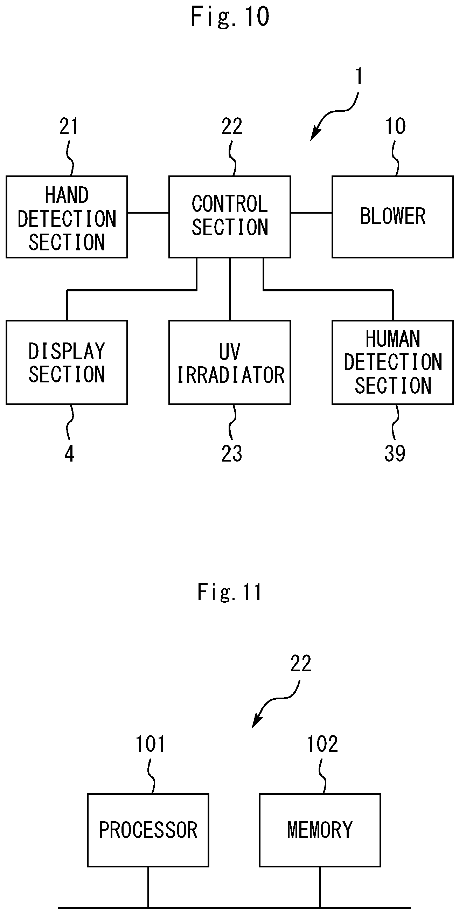

Fig. 10 is a functional block diagram of the hand dryer 1 according to the first embodiment. Fig. 11 is a diagram showing an example of a hardware configuration of a processing circuit of the hand dryer 1 according to the first embodiment. For example, functions of the control section 22 may be achieved by the processing circuit of the hardware configuration shown in Fig. 11. For example, the functions of the control section 22 may be achieved by a processor 101 shown in Fig. 11 by executing a program stored in a memory 102. Alternatively, the functions of the control section 22 may be achieved through cooperation of a plurality of processors and a plurality of memories. Alternatively, a part of the functions of the control section 22 may be implemented as an electronic circuit and other parts may be achieved using the processor 101 and the memory 102.

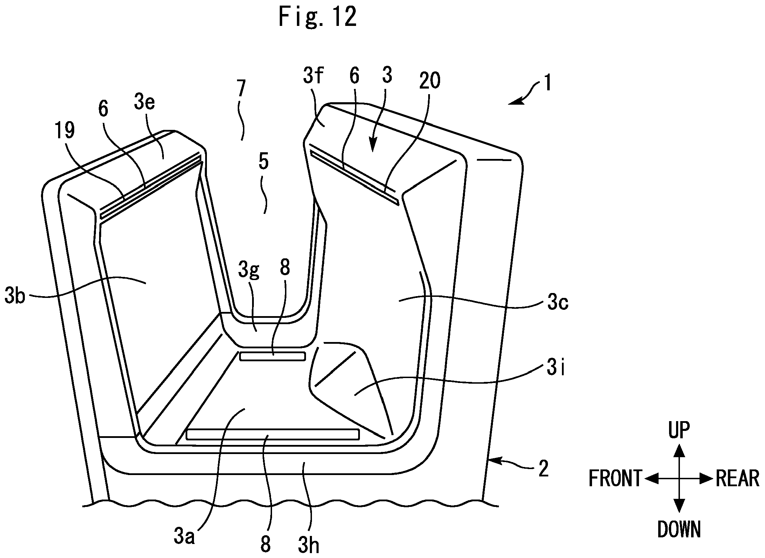

Fig. 12 is a diagram of an upper part of the hand dryer 1 according to a modification of the first embodiment as viewed by a gaze from a right side toward a left side. Fig. 12 is expressed using a perspective method. Hereinafter, the modification shown in Fig. 12 will be described. The drain outlet 8 is respectively provided near a left end of the insertion bottom section 3a and near a right end of the insertion bottom section 3a. The hand insertion section 3 further includes a crest section 3i. The crest section 3i is formed between the insertion bottom section 3a and the insertion rear section 3c. A surface of the crest section 3i is highest at a center in the left-right direction and is inclined such that the further toward a left direction from the center, the lower the surface, and the further toward a right direction from the center, the lower the surface. Accordingly, water having dripped from a hand can be smoothly guided to the drain outlets 8 on both left and right sides along the incline of the crest section 3i.

Next, the hand detection section 21 will be described. A capacitance sensor adopting a mutual capacitance system is used as the hand detection section 21. Note that the hand detection section according to the present disclosure may use detection means adopting other systems. The capacitance sensor includes a plurality of electrodes and a circuit section (not illustrated) which is connected to each electrode and which detects a change in capacitance between the electrodes. As shown in Fig. 9, an electrode 21a and an electrode 21b which constitute the capacitance sensor are built into the insertion front section 3b. The electrode 21b is arranged on a lower side of the electrode 21a. An electrode 21c and an electrode 21d which constitute the capacitance sensor are built into the insertion rear section 3c. The electrode 21d is arranged on a lower side of the electrode 21c. The electrode 21a and the electrode 21c are arranged so as to oppose each other. In other words, the electrode 21a and the electrode 21c are arranged in a state where respective main surfaces oppose each other across the hand insertion space 5. The electrode 21b and the electrode 21d are arranged so as to oppose each other. In other words, the electrode 21b and the electrode 21d are arranged in a state where respective main surfaces oppose each other across the hand insertion space 5. In this case, a "state where respective main surfaces oppose each other" refers to a state where respective main surfaces of the electrodes are opposing each other. In this case, a main surface refers to a primary surface of which an area is larger than other surfaces of each electrode.

The electrode 21a and the electrode 21b are arranged in a state of a positional relationship in which respective main surfaces are positioned above and below on a plane. The electrode 21c and the electrode 21d are arranged in a state of a positional relationship in which respective main surfaces are positioned above and below on a plane. In other words, in the present first embodiment, two pairs of electrodes are arranged in a state where a first electrode and a second electrode of each electrode pair oppose each other across the hand insertion space 5 in a depth direction of the main body enclosure 2 and, in each electrode pair, one of the electrodes is arranged in a state where the electrode is adjacent to an electrode with a different polarity in the other electrode pair in the up-down direction on a plane.

In this case, "respective main surfaces being arranged on a plane" refers to a state where, for example, in the electrode 21c and the electrode 21d, respective main surfaces are parallel to each other while respective side surfaces oppose each other. In the present first embodiment, the electrode 21a, the electrode 21b, the electrode 21c, and the electrode 21d have rectangular parallelepiped shapes with a same shape and same dimensions. However, the shape and the dimensions of each electrode can be changed as appropriate.

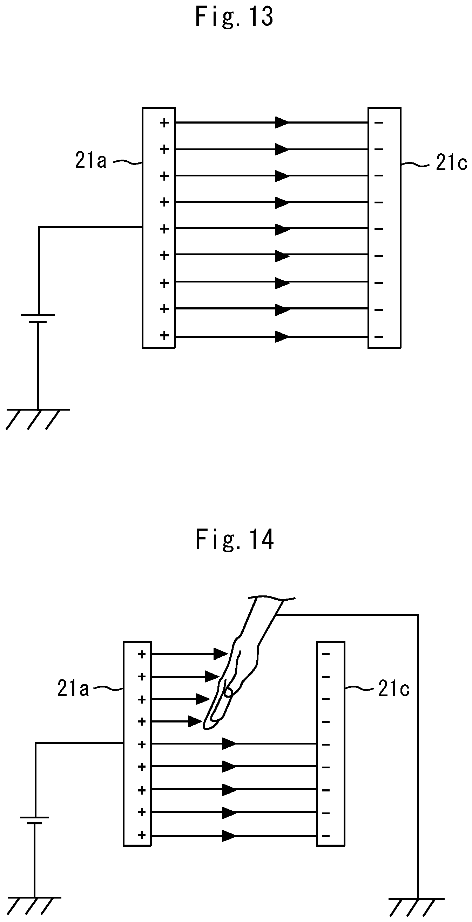

Fig. 13 is a schematic view for explaining a principle of a capacitance sensor adopting a mutual capacitance system which constitutes the hand detection section 21 of the hand dryer 1 according to the first embodiment. A capacitance sensor adopting a mutual capacitance system includes a circuit section which detects a change in capacitance between electrodes, applies voltage to a transmission electrode from the circuit section, and forms an electric field between the transmission electrode and a reception electrode. When a fingertip approaches, a part of the electric field is transferred to a fingertip side, the electric field detected by the reception electrode decreases, and capacitance also decreases. By detecting a change in the capacitance between the electrodes with the circuit section, the decrease in capacitance is captured and the approach by the fingertip is detected. The circuit section stores a capacitance between the electrodes when a fingertip is not approaching.

Fig. 13 shows a state of a capacitance sensor adopting a mutual capacitance system including two electrodes 21a and 21b which form a pair and which are arranged in a state where respective main surfaces oppose each other when a hand is not approaching the capacitance sensor. Fig. 14 is a schematic view for explaining a principle of a capacitance sensor adopting a mutual capacitance system which constitutes the hand detection section 21 of the hand dryer 1 according to the first embodiment. Fig. 14 shows a state of a capacitance sensor adopting a mutual capacitance system including two electrodes 21a and 21c which form a pair and which are arranged in a state where respective main surfaces oppose each other when a hand approaches the capacitance sensor.

As shown in Fig. 13, voltage is applied to the electrode 21a, an electric field is formed between the electrode 21a and the electrode 21c, and capacitance formed between the electrode 21a and the electrode 21c is measured. By arranging the electrode 21a and the electrode 21c in an opposing state and measuring the capacitance between the electrode 21a and the electrode 21c, this configuration can be used as a sensor which detects a hand inserted between the electrode 21a and the electrode 21c. When a potential difference is created between the electrode 21a and the electrode 21c, an electric field due to capacitive coupling is formed between the electrode 21a and the electrode 21c. Since a magnitude of the capacitance is inversely proportional to a distance between the electrode 21a and the electrode 21c, the capacitance increases when a conductor such as a metal is inserted between the electrode 21a and the electrode 21c. In addition, even when a dielectric or a substance such as water with a higher permittivity than air is inserted, the capacitance between the electrode 21a and the electrode 21c increases.

Furthermore, when a conductor that is a part of a human body such as a hand is brought close to or inserted between the electrode 21a and the electrode 21c as shown in Fig. 14, a part of the electric field is induced into the human body and the capacitance between the electrode 21a and the electrode 21c decreases. In other words, since the human body in proximity to or inserted between the electrode 21a and the electrode 21c can be considered being grounded, a state of electrostatic screening is created and the capacitance between the electrode 21a and the electrode 21c decreases. Therefore, by measuring the capacitance between the electrode 21a and the electrode 21c on a regular basis and detecting a change in the capacitance, a hand can be detected.

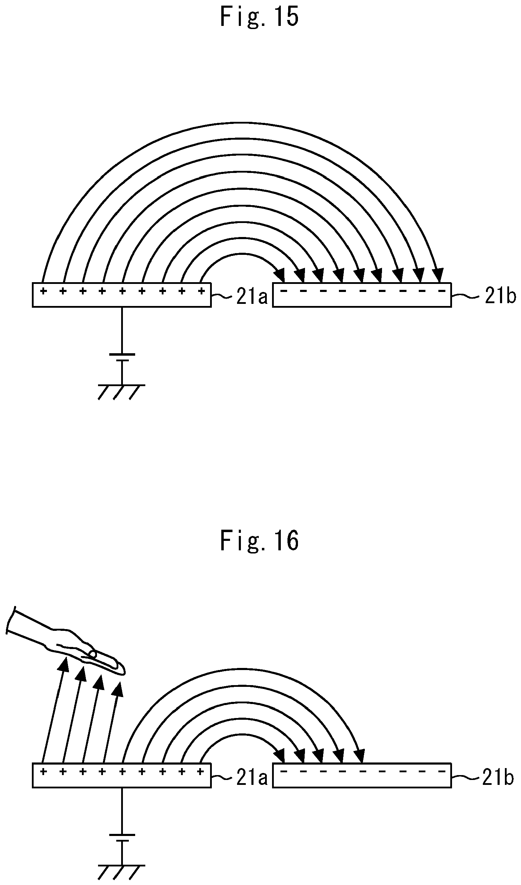

Fig. 15 is a schematic view for explaining a principle of a capacitance sensor adopting a mutual capacitance system which constitutes the hand detection section 21 of the hand dryer 1 according to the first embodiment. Fig. 15 shows a state of a capacitance sensor adopting a mutual capacitance system including two electrodes 21a and 21b which form a pair and of which respective main surfaces are arranged on a plane when a hand is not approaching the capacitance sensor. Fig. 16 is a schematic view for explaining a principle of a capacitance sensor adopting a mutual capacitance system which constitutes the hand detection section 21 of the hand dryer 1 according to the first embodiment. Fig. 16 shows a state of a capacitance sensor adopting a mutual capacitance system including two electrodes 21a and 21b which form a pair and of which respective main surfaces are arranged on a plane when a hand approaches the capacitance sensor. In this case, "respective main surfaces being arranged on a plane" refers to a state where, in the electrode 21a and the electrode 21b, respective main surfaces are parallel to each other while respective side surfaces oppose each other.

As shown in Fig. 15, voltage is applied to the electrode 21a, an electric field is formed between the electrode 21a and the electrode 21b, and capacitance formed between the electrode 21a and the electrode 21b is measured. By arranging the electrode 21a and the electrode 21b in a state where planar directions are parallel to each other and measuring the capacitance between the electrode 21a and the electrode 21b, the electrode 21a and the electrode 21b can be used as a sensor which detects a hand in proximity to the electrode 21a and the electrode 21b. When a potential difference is created between the electrode 21a and the electrode 21b, an electric field due to capacitive coupling is formed between the electrode 21a and the electrode 21b. Since a magnitude of the capacitance is inversely proportional to a distance between the electrode 21a and the electrode 21b, the capacitance increases when a conductor such as a metal is in proximity to the electrode 21a and the electrode 21b. In addition, even when a dielectric such as water with a higher permittivity than air is in proximity to the electrode 21a and the electrode 21b, the capacitance between the electrode 21a and the electrode 21b increases.

As shown in Fig. 16, when a conductor that is a part of a human body such as a hand is brought close to the electrode 21a and the electrode 21b, a part of the electric field is induced into the human body and the capacitance between the electrode 21a and the electrode 21b decreases. In other words, since the human body in proximity to between the electrode 21a and the electrode 21b can be considered being grounded, a state of electrostatic screening is created and the capacitance between the electrode 21a and the electrode 21b decreases. Therefore, by detecting the capacitance between the electrode 21a and the electrode 21b on a regular basis and detecting a change in the capacitance, a hand can be detected. In other words, the hand detection section 21 includes, at different positions, a plurality of electrode pairs made up of two electrodes with different polarities and detects a hand inserted into the hand insertion space 5 based on a change in capacitance between electrodes of each electrode pair.

As described earlier, with a capacitance sensor adopting a mutual capacitance system, when a dielectric or a substance such as water with higher permittivity than air adheres to a surface of at least one of the two electrodes, capacitance increases. On the other hand, when a part of a human body such as a hand comes close to or is inserted between the two electrodes, capacitance decreases. Accordingly, a capacitance sensor adopting a mutual capacitance system can distinguish a difference between a state where a hand comes close to or is inserted between the two electrodes and a state where a dielectric such as water adheres to a surface of at least one of the two electrodes and can make a determination. Accordingly, a capacitance sensor adopting a mutual capacitance system can accurately discriminate between a case where a hand comes close to or is inserted between the two electrodes and a case where a dielectric such as water adheres to a surface of at least one of the two electrodes.

The hand detection section 21 using such a capacitance sensor adopting a mutual capacitance system detects a hand by detecting a capacitance between two electrodes among the electrode 21a, the electrode 21b, the electrode 21c, and the electrode 21d. In other words, by sequentially changing and switching a combination of two electrodes for detecting a change in capacitance to four patterns, the hand detection section 21 determines an insertion of a hand and an insertion position thereof in the hand insertion space 5. The hand detection section 21 detects a change in capacitance between two electrodes in the four detection patterns from pattern 1 to pattern 4.

In pattern 1, the hand detection section 21 detects a change in capacitance between the electrode 21a and the electrode 21b arranged in a state of being adjacent to each other in the up-down direction on a plane as shown in Figs. 15 and 16 by regularly detecting, at predetermined intervals, the capacitance between the electrode 21a and the electrode 21b, and detects a presence or absence of an insertion of a hand into a near side in the hand insertion space 5 or, in other words, a presence or absence of an insertion of a hand into a side of the front projecting section 3e in the hand insertion space 5.

In pattern 2, the hand detection section 21 detects a change in capacitance between the electrode 21c and the electrode 21d arranged in a state of being adjacent to each other in the up-down direction on a plane by regularly detecting, at predetermined intervals, the capacitance between the electrode 21c and the electrode 21d, and detects a presence or absence of an insertion of a hand into a far side in the hand insertion space 5 or, in other words, a presence or absence of an insertion of a hand into a side of the rear projecting section 3f in the hand insertion space 5. In this case, the electrode 21c and the electrode 21d function in a similar manner to the electrode 21a and the electrode 21b shown in Figs. 15 and 16.

In pattern 3, the hand detection section 21 detects a change in capacitance between the electrode 21a and the electrode 21c arranged in a state of opposing each other across the hand insertion space 5 as shown in Figs. 13 and 14 by regularly detecting, at predetermined intervals, the capacitance between the electrode 21a and the electrode 21c, and detects a presence or absence of an insertion of a hand into an upper side in the hand insertion space 5 or, in other words, a presence or absence of an insertion of a hand into a side of the hand insertion opening 7 in the hand insertion space 5.

In pattern 4, the hand detection section 21 detects a change in capacitance between the electrode 21b and the electrode 21d arranged in a state of opposing each other across the hand insertion space 5 by regularly detecting, at predetermined intervals, the capacitance between the electrode 21b and the electrode 21d, and detects a presence or absence of an insertion of a hand into a lower side in the hand insertion space 5 or, in other words, a presence or absence of an insertion of a hand into a side of the insertion bottom section 3a in the hand insertion space 5. In this case, the electrode 21b and the electrode 21d function in a similar manner to the electrode 21a and the electrode 21c shown in Figs. 13 and 14.

In the present first embodiment, in the four detection patterns from pattern 1 to pattern 4, the electrode 21a and the electrode 21d are used as positive-side electrodes and the electrode 21b and the electrode 21c are used as negative-side electrodes. In addition, when detecting a change in capacitance between electrodes in the four detection patterns described above, the hand detection section 21 switches among combinations of electrodes that perform detection of the change in capacitance. Accordingly, when detecting the capacitance between respective electrodes with the four electrode combinations described above, a polarity of each electrode need not be changed or, in other words, a change of the polarity of each electrode to a positive side or to a negative side need not be performed and a detection time of a hand can be reduced.

By detecting the presence or absence of an insertion of a hand in the hand insertion space 5 at four points of a near side, a far side, an upper side, and a lower side in the hand insertion space 5 as described above, an insertion position of the hand in the hand insertion space 5 can be determined in two-dimensional directions of a depth direction and an up-down direction. Accordingly, a detailed insertion position of the hand in the hand insertion space 5 can be detected.

A capacitance sensor adopting a mutual capacitance system requires electrodes to form a pair, and in order to detect capacitance at four points, eight electrodes of 4 × 2 = 8 poles are required. In the present first embodiment, since combinations of electrodes to form a pair are switched, the capacitance of four points can be detected by four electrodes and an effect of preventing the number of electrodes from increasing is obtained.

In addition, since the hand detection section 21 uses a capacitance sensor adopting a mutual capacitance system, the capacitance between electrodes increases when a dielectric such as water adheres to a surface of the electrodes but the capacitance decreases when a part of a human body such as a hand is inserted between the electrodes. Therefore, the hand detection section 21 can accurately discriminate between a case where a hand comes close to or is inserted between the two electrodes and a case where a dielectric such as water adheres to a surface of at least one of the two electrodes.

The control section 22 controls the operation of the blower 10 based on information in a hand detection signal output from the hand detection section 21. The control section 22 operates the blower 10 when the hand detection section 21 detects that a hand has been inserted into the hand insertion space 5. In addition, the control section 22 stops the blower 10 when the hand detection section 21 detects that a hand is not inserted into both the lower side and the upper side of the hand insertion space 5.

A high-pressure airflow generated by the blower 10 is guided to the first blowout port section 19 that is a hand-drying nozzle provided on a front-face side wall surface of the hand insertion section 3 and the second blowout port section 20 that is a hand-drying nozzle provided on a rear-face side wall surface of the hand insertion section 3, converted into a high-speed airflow, and jetted into the hand insertion space 5 from the first blowout port section 19 and the second blowout port section 20. In addition, the high-pressure airflow jetted into the hand insertion space 5 dries a hand inserted into the hand insertion space 5 by blasting away moisture adhering to the hand. The water having been blasted away adheres to the hand insertion section 3. For example, water adhering to the insertion front section 3b, the insertion rear section 3c, and the insertion side section 3d drips down to the insertion bottom section 3a. Water in the insertion bottom section 3a flows from the drain outlet 8 to the drain tank 9.

The control section 22 controls an allocation of supply of the high-pressure airflow to the first blowout port section 19 and the second blowout port section 20 based on a combination of the two electrodes having detected a hand in the hand insertion space 5. The control section 22 determines the combination of the two electrodes having detected a hand by processing information in a hand detection signal output from the hand detection section 21 and determines an insertion position of the hand in the hand insertion space 5. When an insertion of a hand into a near side in the hand insertion space 5 is detected during an operation of the blower 10, the control section 22 reduces the high-pressure airflow to the first blowout port section 19 and increases the high-pressure airflow to the second blowout port section 20. Conversely, when an insertion of a hand into a far side in the hand insertion space 5 is detected during an operation of the blower 10, the control section 22 increases the high-pressure airflow to the first blowout port section 19 and reduces the high-pressure airflow to the second blowout port section 20. By changing an allocation of supply of the high-pressure airflow to the first blowout port section 19 and the second blowout port section 20 depending on an insertion position of a hand, regardless of the position at which a hand is inserted in the depth direction in the hand insertion space 5, a rear side and a front side of the hand can be uniformly and efficiently dried.

A method of reducing the high-pressure airflow to the first blowout port section 19 while increasing the high-pressure airflow to the second blowout port section 20 or a method of increasing the high-pressure airflow to the first blowout port section 19 while reducing the high-pressure airflow to the second blowout port section 20 is not particularly limited. In order to reduce the high-pressure airflow to the first blowout port section 19 while increasing the high-pressure airflow to the second blowout port section 20, the supply of the high-pressure airflow to the front blowout air path 14 may be reduced and the supply of the high-pressure airflow to the rear blowout air path 15 may be increased. Conversely, in order to increase the high-pressure airflow to the first blowout port section 19 while reducing the high-pressure airflow to the second blowout port section 20, the supply of the high-pressure airflow to the front blowout air path 14 may be increased and the supply of the high-pressure airflow to the rear blowout air path 15 may be reduced.

The control described above may be realized by, for example, providing a mobile inductive plate for guiding the high-pressure airflow generated by the blower 10 and having the control section 22 control an orientation of the inductive plate to adjust supplies of the high-pressure airflow to the front blowout air path 14 and the rear blowout air path 15. Alternatively, the control section 22 may perform control of closing a part of the front blowout air path 14 or a part of the rear blowout air path 15 to adjust supplies of the high-pressure airflow to the front blowout air path 14 and the rear blowout air path 15. Alternatively, a blower 10 for the front blowout air path 14 and a blower 10 for the rear blowout air path 15 may be individually provided and the control section 22 may control a generation amount of a high-pressure airflow by each of the blowers 10.

As described above, the hand dryer 1 according to the present first embodiment includes the electrode 21a on an upper side as viewed from a front-face side and includes the electrode 21b on a lower side as viewed from the front-face side on a front-face side wall surface of the hand insertion section 3. In addition, the hand dryer 1 includes the electrode 21c on an upper side as viewed from the front-face side and includes the electrode 21d on a lower side as viewed from the front-face side on a rear-face side wall surface of the hand insertion section 3. Furthermore, the hand detection section 21 switches combinations of two electrodes for detecting a change in capacitance to detect the presence or absence of an insertion of a hand in the hand insertion space 5 at the four points of the near side, the far side, the upper side, and the lower side in the hand insertion space 5.

In other words, the hand dryer 1 detects a change in capacitance between two electrodes arranged in a state where respective main surfaces oppose each other across the hand insertion space 5 and a change in capacitance between two electrodes arranged in a state of being adjacent in the up-down direction on a plane to detect the presence or absence of an insertion of a hand in the hand insertion space 5. Accordingly, the hand dryer 1 can determine an insertion position of the hand in the hand insertion space 5 in two-dimensional directions of the depth direction and the up-down direction. Accordingly, a detailed insertion position of the hand in the hand insertion space 5 can be detected.

In addition, the control section 22 controls the operation of the blower 10 in accordance with the position of the hand in the hand insertion space 5 as detected by the hand detection section 21. In other words, by changing an allocation of supplies of the high-pressure airflow to the first blowout port section 19 and the second blowout port section 20 depending on the insertion position of a hand in the hand insertion space 5, regardless of the position at which the hand is inserted in the depth direction in the hand insertion space 5, the control section 22 can dry a rear side and a front side of the hand in an uniform and efficient manner.

Furthermore, since the hand dryer 1 switches combinations of electrodes to form a pair for detecting a change in capacitance, capacitance of four points in the hand insertion space 5 can be detected by four electrodes and an effect of preventing the number of electrodes from increasing is obtained.

Therefore, the hand dryer 1 according to the present first embodiment enables an easy-to-use hand dryer to be provided which suppresses an increase in the number of electrodes, which prevents malfunctions due to adhesion of water while avoiding an increase in apparatus size and an increase in cost, and which enables optimal operation control in accordance with an insertion position of a hand.

The hand dryer 1 further includes an UV irradiator 23. For example, the UV irradiator 23 is arranged in the hand insertion section 3. The UV irradiator 23 includes a light source 24 which generates light. The UV irradiator 23 irradiates the hand insertion space 5 with light generated by the light source 24. In other words, the UV irradiator 23 irradiates a surface of the hand insertion section 3 facing the hand insertion space 5 with light generated by the light source 24. In some cases, a hand may be irradiated with light radiated from the UV irradiator 23.

The UV irradiator 23 emits ultraviolet light. UV stands for ultraviolet. In other words, the UV irradiator 23 is an apparatus which emits ultraviolet light. Generally, ultraviolet light is a collective term for light with a wavelength shorter than visible light and represents electromagnetic waves with a wavelength ranging from approximately 1 nm to 400 nm. In addition, generally, a wavelength range from 100 nm to 280 nm is referred to as UVC, a wavelength range from 280 nm to 315 nm is referred to as UVB, and a wavelength range from 315 nm to 400 nm is referred to as UVA.

In the present disclosure, "microorganisms" include at least one of bacteria and viruses. Some microorganisms are hazardous to humans. Ultraviolet light acts on microorganisms. In the present disclosure, sterilization by ultraviolet light is defined as causing light energy to act on deoxyribo nucleic acid (hereinafter, referred to as "DNA") itself of microorganisms to create an inactivated state where the microorganisms stop growing or to reduce the number of microorganisms. In addition, an expression of inactivation ≈ sterilization may be used in the present disclosure. Generally, it is said that UVB has a greater capability of inactivating microorganisms than UVA and that UVC has an even greater capability of inactivating microorganisms than UVB. In particular, a wavelength of UVC is highly capable of directly destroying DNA and, accordingly, capable of inactivating microorganisms at a fast pace. Furthermore, among the UVC range, wavelengths ranging from 200 nm to 285 nm have particularly high germicidal power. More specifically, it is said that wavelengths centered on 222 nm and 260 nm have high germicidal power.

A dominant wavelength of the UV irradiator 23 is ultraviolet light. In other words, among light beams emitted by the UV irradiator 23, a wavelength with a highest output or, in other words, highest radiant intensity is ultraviolet light. According to the present embodiment, irradiating a surface of the hand insertion section 3 with ultraviolet light from the UV irradiator 23 enables microorganisms adhering to the surface of the hand insertion section 3 to be sterilized. Therefore, even when water droplets or an aerosol has scattered outside of the hand insertion section 3 due to an airflow when drying a hand, scattering of microorganisms to a periphery can be reliably prevented. As a result, hygiene improves.

The light source 24 of the UV irradiator 23 according to the present embodiment is a light-emitting diode (LED). In other words, the light source 24 is an LED which generates ultraviolet light and will be hereinafter referred to as a UV-LED. The UV-LED does not contain mercury. Generally, mercury is toxic and adversely affects the environment. Since the UV-LED does not contain mercury, the UV-LED provides a high level of safety and a risk of adversely affecting the environment is low. For example, the UV-LED provides high output of a single wavelength. A wavelength with highest radiant intensity among light generated by the light source 24 is hereinafter referred to as a "dominant wavelength". The dominant wavelength of light generated by the light source 24 may be present in any band including UVA, UVB, and UVC. In particular, the dominant wavelength of light generated by the light source 24 is desirably present in the UVC range with high germicidal power.

The dominant wavelength of light generated by the light source 24 preferably ranges from 220 nm to 280 nm. More preferably, the dominant wavelength of light generated by the light source 24 desirably ranges from 220 nm to 225 nm or from 250 nm to 285 nm. Even more preferably, the dominant wavelength of light generated by the light source 24 desirably ranges from 255 nm to 280 nm. With the wavelength range described above, since germicidal power is particularly high, sterilization can be performed in an efficient manner in a relatively short period of time, with relatively low output, or with a relatively small number of the light sources 24. Note that the preferable wavelength range similarly applies to a UV-lamp to be described later.

The light source 24 of the UV irradiator 23 may be a lamp instead of an LED. In addition, the light source 24 may include mercury or may not include mercury (mercury-free). A lamp including mercury has high efficiency and high output and, therefore, high germicidal power. Furthermore, a mercury-free lamp provides a high level of safety and a risk of adversely affecting the environment is low.

Generally, ultraviolet light has an invisible wavelength. While a dominant wavelength of light generated by the light source 24 according to the present embodiment is a wavelength in the ultraviolet range, the light generated by the light source 24 may include wavelengths in the visible light range. For example, the light source 24 may generate blue or violet visible light together with ultraviolet light. Visible light refers to light visible to the eye. For example, when a light beam is emitted from the light source 24, the user can identify the color of the emitted light. For example, the user can identify the color of the emitted light when viewing the light source 24, when viewing a light beam, or when an object is irradiated with the light beam. For example, the user can identify whether the emitted light is red, blue, or violet. Accordingly, a determination can be made as to whether the light source 24 is turned on or off. For example, when it is found that the light source 24 is not turned on when the light source 24 is supposed to be turned on, a failure is suspected. In other words, a failure can be discovered at an early stage. In addition, since the UV irradiator 23 is harmful to humans depending on specifications, there may be cases where the light source 24 is not intended to be turned on during hand drying. In such specifications, a determination to abort use can be made when the light source 24 is turned on during hand drying. The advantageous effect described above can be achieved by differentiating a color of the hand insertion section 3 or a color of light emitted from the UV irradiator 23 when the light source 24 is turned on from the color of the hand insertion section 3 or the color of light emitted from the UV irradiator 23 when the light source 24 is turned off.

The hand dryer 1 includes one or more UV irradiators 23. The hand dryer 1 may include a plurality of UV irradiators 23. In addition, one UV irradiator 23 may include only one light source 24 or one UV irradiator 23 may include a plurality of light sources 24. Alternatively, a plurality of UV irradiators 23 may include a plurality of light sources 24. When the hand dryer 1 includes a plurality of light sources 24, each light source 24 may have a same dominant wavelength or each light source 24 may have a different dominant wavelength. When using a plurality of light sources 24 with the same dominant wavelength such as a plurality of light sources 24 with same specifications, a unit price can possibly be reduced. In addition, when using a plurality of light sources 24 with different dominant wavelengths, a sterilization rate can possibly be increased. For example, it is said that using three light sources 24 with dominant wavelengths of 260 nm, 265 nm, and 275 nm, respectively, increases the sterilization rate as compared to using three light sources 24 with the same dominant wavelength of 265 nm. Accordingly, sterilization can be performed even more efficiently.

When the hand dryer 1 includes a plurality of UV irradiators 23, each UV irradiator 23 may be arranged at each of the portions of the hand insertion section 3 which oppose each other. For example, the UV irradiators 23 may be arranged in each of the insertion front section 3b and the insertion rear section 3c which opposes the insertion front section 3b. In addition, the UV irradiators 23 may be arranged in each of the insertion right section 3h and the insertion left section 3g which opposes the insertion right section 3h. An irradiation range from one UV irradiator 23 is limited. For example, a major portion of light emitted from the UV irradiator 23 is emitted toward a surface that opposes the UV irradiator 23. For example, at least a part of light emitted from the UV irradiator 23 is emitted toward a surface that opposes the UV irradiator 23. For example, when light is emitted from the UV irradiator 23 from the insertion front section 3b toward the insertion rear section 3c, illuminance of the insertion front section 3b ends up being lower than illuminance of the insertion rear section 3c if reflectance of the insertion rear section 3c is low. By comparison, illuminance of the insertion front section 3b is increased when the insertion rear section 3c is also provided with the UV irradiator 23. In this manner, by arranging the UV irradiator 23 in each of the portions of the hand insertion section 3 which oppose each other, illuminance of the entire hand insertion section 3 can be increased.

At least a part of the UV irradiator 23 is exposed to the hand insertion space 5. For example, the light source 24 is arranged at a position close to a position where the hand insertion space 5 is defined among the hand insertion section 3. The light source 24 is arranged to the rear of the front cover 2a. The light source 24 is arranged on an inner side of four corners of the front cover 2a. The light source 24 is arranged at a position covered by the front cover 2a in a front view.

Various housing sections such as the drain tank 9 and the insertion bottom section 3a are arranged below the light source 24. The light source 24 may be arranged at a position covered by the insertion side section 3d in a side view. In addition, an outer appearance of a structure in which the shape of the insertion front section 3b or the insertion rear section 3c is folded or bent may be provided so that the light source 24 is covered by the insertion front section 3b or the insertion rear section 3c in a side view.

A part of the insertion front section 3b may protrude rearward above the light source 24. For example, when the light source 24 is arranged in the insertion front section 3b, the front projecting section 3e may protrude more rearward than the light source 24.

A part of the insertion rear section 3c may protrude forward above the light source 24. For example, when the light source 24 is arranged in the insertion rear section 3c, the rear projecting section 3f may protrude more forward than the light source 24.

A part of the insertion side section 3d may protrude toward a center side above the light source 24. For example, when the light source 24 is arranged in the insertion side section 3d, a part of the insertion side section 3d may protrude toward the center side above the light source 24.

A member for transmitting a light beam including ultraviolet light emitted from the light source 24 is referred to as a window 26. Details of the window 26 will be provided later. Both the light source 24 and the window 26 or any one of the light source 24 and the window 26 are arranged at a position that cannot be visually recognized in a front view of the outer appearance of the hand dryer 1. Both the light source 24 and the window 26 or any one of the light source 24 and the window 26 are arranged at a position that cannot be visually recognized in a side view of the outer appearance of the hand dryer 1. Both the light source 24 and the window 26 or any one of the light source 24 and the window 26 are arranged at a position that cannot be visually recognized in a top view of the outer appearance of the hand dryer 1. Both the light source 24 and the window 26 or any one of the light source 24 and the window 26 are arranged at a position that cannot be visually recognized in a bottom view of the outer appearance of the hand dryer 1. In the present disclosure, both the light source 24 and the window 26 or any one of the light source 24 and the window 26 not being visually recognizable means that both the light source 24 and the window 26 or any one of the light source 24 and the window 26 are arranged at a position covered by the main body enclosure 2. In the present embodiment, in the outer appearance of the hand dryer 1, due to both the light source 24 and the window 26 or any one of the light source 24 and the window 26 being covered by the main body enclosure 2, both the light source 24 and the window 26 or any one of the light source 24 and the window 26 are not visually recognizable in any of a front view, a rear view, a side view, a top view, and a bottom view. Accordingly, the possibility that the user directly views both the light source 24 and the window 26 or any one of the light source 24 and the window 26 can be reduced, a risk of a light beam entering an eye can be reduced, and a high level of safety is provided.

Both the light source 24 and the window 26 or any one of the light source 24 and the window 26 may be arranged at a position that cannot be visually recognized when viewed from a horizontal direction. In addition, when the hand insertion section 3 has a box-like shape in which an upper face of the hand insertion section 3 is opened as shown in Fig. 7 or 8, both the light source 24 and the window 26 or any one of the light source 24 and the window 26 may be arranged at a position that cannot be visually recognized when viewed from any direction from 0 degrees to 360 degrees relative to the horizontal direction. In other words, both the light source 24 and the window 26 or any one of the light source 24 and the window 26 may be configured to be covered by the main body enclosure 2 even when the hand dryer 1 is viewed from any direction that is perpendicular to a vertical line. Accordingly, the possibility that the user directly views both the light source 24 and the window 26 or any one of the light source 24 and the window 26 can be reduced, a risk of a light beam entering an eye can be reduced, and a high level of safety is provided.

In addition, in the hand dryer 1 according to the present embodiment, the light source 24 and the UV irradiator 23 are covered by the main body enclosure 2 so that the light source 24 and the UV irradiator 23 are not visually recognized when the hand dryer 1 is viewed by a gaze in all directions in a three-dimensional space from outside of the main body enclosure 2. In other words, the light source 24 and the UV irradiator 23 are covered by the main body enclosure 2 so that, when it is assumed that light beams are emitted in all directions in a three-dimensional space from the light source 24 or the UV irradiator 23, there is no light beam that directly exits the main body enclosure 2 from the light source 24. Accordingly, the risk of a light beam from the light source 24 entering an eye can be more reliably reduced and a higher level of safety can be provided.

In addition, in the hand dryer 1 according to the present embodiment, the light source 24 is covered by the main body enclosure 2 so that the light source 24 is not visually recognized when the hand dryer 1 is viewed by a gaze in all directions in a three-dimensional space from outside of the main body enclosure 2. In other words, the light source 24 is covered by the main body enclosure 2 so that, when it is assumed that light beams are emitted in all directions in a three-dimensional space from the light source 24, there is no light beam that directly exits the main body enclosure 2 from the light source 24. Accordingly, the risk of a light beam from the light source 24 entering an eye can be more reliably reduced and a higher level of safety can be provided.

Note that both the light source 24 and the window 26 or any one of the light source 24 and the window 26 need not necessarily be arranged at a position that cannot be visually recognized when viewed from all of the directions described above. However, both the light source 24 and the window 26 or any one of the light source 24 and the window 26 are desirably arranged at a position that is not visually recognizable as much as possible.

In the present embodiment, for example, the light source 24 has a rectangular parallelepiped shape. The shape of the light source 24 in the present disclosure is not particularly limited and the light source 24 may have a cylindrical shape or a bombshell shape. For example, a bombshell shape is a shape that combines a hemispherical shape with a cylindrical shape.

The hand insertion section 3 is made of a material such as an ABS resin or polypropylene. In other words, the hand insertion section 3 may be made of a material with low ultraviolet transmittance. When transmittance is low, a light beam is mainly reflected or absorbed. The light source 24 is arranged between the hand insertion section 3 and the cover section. For example, when the light source 24 is arranged on a surface on a side of the insertion front section 3b, the light source 24 is arranged on a front side of the insertion front section 3b and on a rear side of the front cover 2a. In other words, the light source 24 is arranged at a position far from the hand insertion space 5 than the hand insertion section 3. Therefore, when the hand insertion section 3 made of a material with low transmittance is present at a position close to the side of the hand insertion space 5 than the light source 24, a major portion of a light beam emitted from the light source 24 is reflected or absorbed by the hand insertion section 3 close to the side of the hand insertion space 5. Specifically, when the light source 24 is arranged on a surface on a side of the insertion front section 3b, a major portion of a light beam emitted from the light source 24 is reflected or absorbed by the insertion front section 3b. In this case, for example, a light beam from the light source 24 is hardly emitted toward a side opposing the side where the light source 24 is present. Therefore, the hand insertion section 3 on a side where the light source 24 is arranged and a region irradiated with a light beam emitted from the light source 24 are desirably not provided with a material or, in other words, provided with an opening, or a material with as high an optical transparency as possible is arranged. A member for transmitting a light beam including ultraviolet light emitted from the light source 24 as described above is referred to as the window 26. In the present embodiment, the hand insertion section 3 is provided with an opening and the window 26 is fitted into the opening or the window 26 is arranged in proximity to the opening.

The light source 24 emits an infinite number of light beams in a radial pattern centered on an optical axis. For example, when the light source is a rectangular parallelepiped, the optical axis represents a direction parallel to a thickness direction of the rectangular parallelepiped. When a rectangular parallelepiped is made up of three directions of a width direction, a depth direction, and a thickness direction, the thickness direction refers to a direction with a smallest dimension. For example, a beam angle of the light source 24 may range from 30 degrees to 150 degrees or may be 360 degrees. The beam angle of the light source 24 preferably ranges from 50 degrees to 140 degrees. For example, a beam angle of 30 degrees is an orientation that is inclined by 15 degrees to each side from the optical axis when viewed from a direction perpendicular to the optical axis. A beam angle is, for example, an angle in which radiant intensity is 50% when radiant intensity in the direction of the optical axis is assumed to be 100%. For example, a beam angle of 30 degrees indicates that radiant intensity at a position inclined by 15 degrees to one side from the optical axis when viewed from a direction perpendicular to the optical axis is 50% of radiant intensity in the optical axis direction. When the beam angle is too small, there is a possibility that only a part of the hand insertion section 3 can be irradiated. When the beam angle is too wide, there is a possibility that an irradiation amount to the outside of the hand insertion space 5 becomes excessively large. Therefore, the beam angle is desirably not too small and not too large.

The optical axis of the light source 24 or an imaginary extension line of the optical axis intersects with a surface of the hand insertion section 3 that faces the hand insertion space 5. A large number of light beams generated by the light source 24 irradiate the surface of the hand insertion section 3 that faces the hand insertion space 5. The imaginary extension line of the optical axis of the light source 24 passes a plane that faces the light source 24. The light source 24 may be arranged so that a thickness direction or a central axis thereof is parallel to the horizontal direction. In addition, the light source 24 may be arranged so that a thickness direction or a central axis thereof is not parallel to the horizontal direction. In this case, the light source 24 is desirably arranged in an inclined posture so that a portion on an upper side of the light source 24 is positioned farther to the side of the hand insertion space 5 than a portion on a lower side of the light source 24. For example, when the light source 24 is arranged at a position close to a surface of the insertion front section 3b, the posture of the light source 24 is desirably inclined so that a portion on the upper side of the light source 24 is positioned farther to a rear side than a portion on the lower side of the light source 24. Since such a posture enables ultraviolet light to be emitted toward a slightly lower side of the hand insertion section 3, upward leakage of a light beam can be more reliably suppressed. In addition, the insertion bottom section 3a with a large amount of adhesion of water droplets can be irradiated with ultraviolet light, an amount of microorganisms contained in water droplets scattered from the insertion bottom section 3a can be reduced, and hygiene is improved.

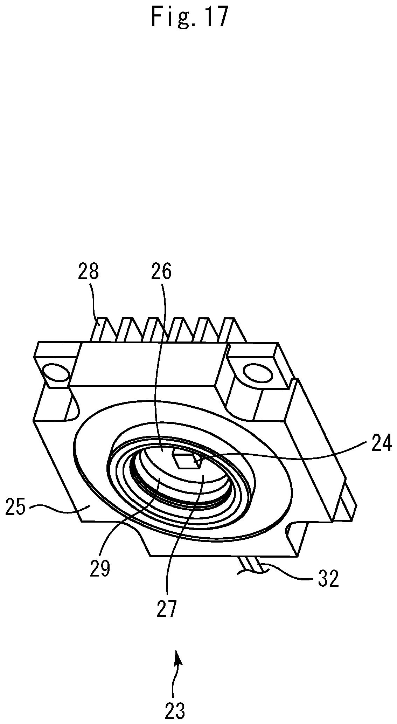

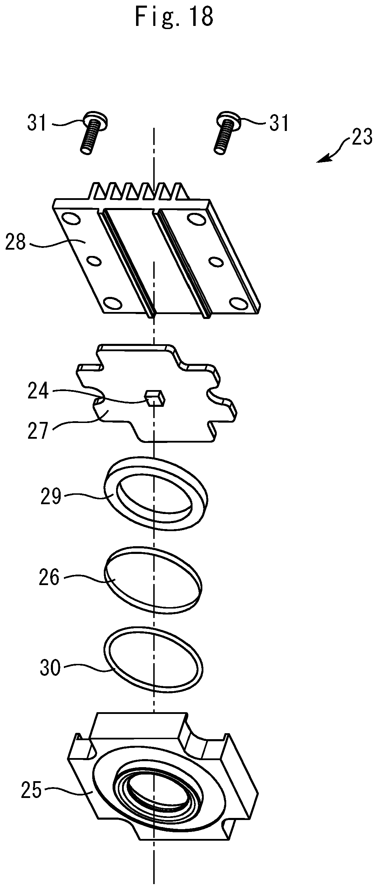

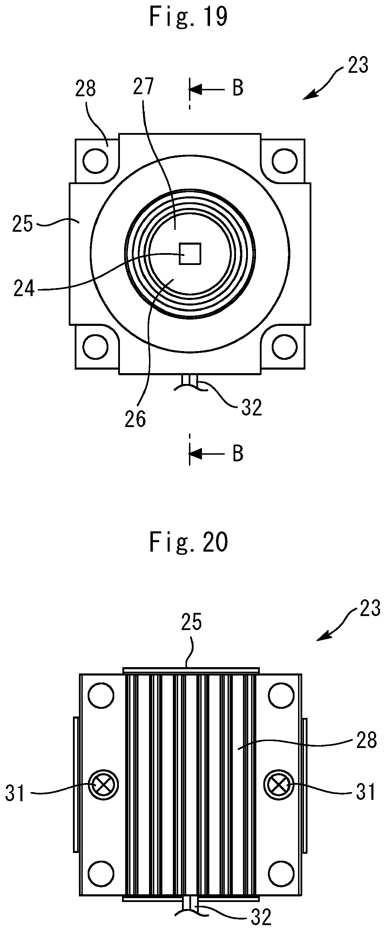

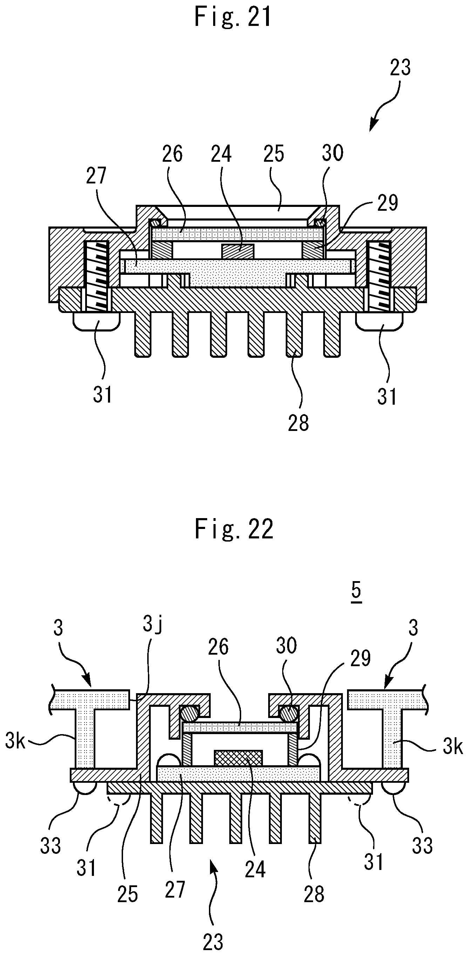

Fig. 17 is a perspective view of the UV irradiator 23 included in the hand dryer 1 according to the first embodiment. Fig. 18 is an exploded perspective view of the UV irradiator 23 included in the hand dryer 1 according to the first embodiment. Fig. 19 is a front view of the UV irradiator 23 included in the hand dryer 1 according to the first embodiment. Fig. 20 is a rear view of the UV irradiator 23 included in the hand dryer 1 according to the first embodiment. Fig. 21 is a sectional view of the UV irradiator 23 included in the hand dryer 1 according to the first embodiment taken along line B-B in Fig. 19.

As shown in Fig. 18, in addition to the light source 24, the UV irradiator 23 includes a case 25, the window 26, a substrate 27, a heat sink 28, a spacer 29, a sealing member 30, a fixing member 31, and a wiring 32. Note that components of the UV irradiator 23 are not limited to the above and may be omitted, added, or replaced as deemed appropriate.

The case 25 is a part that constitutes an outer appearance of the UV irradiator 23. The case 25 has an opening at a position where the window 26 is to be mounted. The light source 24 is installed on the substrate 27. The light source 24 emits light when power is supplied from the substrate 27 to the light source 24. The window 26 protects the light source 24. The window 26 covers the light source 24 from an opposite side to the substrate 27. Light generated by the light source 24 passes through the window 26 and is subsequently emitted to the hand insertion space 5 and a surface of the hand insertion section 3 facing the hand insertion space 5.

The heat sink 28 is provided for dissipating heat of the light source 24 and the substrate 27 having been heated by the generation of light. The heat sink 28 of the illustrated example has fins for expanding a surface area. The spacer 29 is provided for maintaining a distance between the window 26 and the light source 24. The spacer 29 is arranged between the substrate 27 and the window 26.

The sealing member 30 is a member for maintaining airtightness and liquid-tightness by, for example, sealing a gap between the case 25 and the window 26. The fixing member 31 is a member for fixing positions or a positional relationship of a plurality of members. The fixing member 31 is preferably attachable to and detachable from the UV irradiator 23 or the hand insertion section 3. The fixing member 31 may be a member for fixing the UV irradiator 23 to the hand dryer 1 or the hand insertion section 3. The fixing member 31 may be a member for fixing at least one of the case 25, the heat sink 28, and the substrate 27 to the hand dryer 1 or the hand insertion section 3. The fixing member 31 may be a member for fixing positions of the case 25, the substrate 27, and the heat sink 28 by fastening the case 25, the substrate 27, and the heat sink 28. The fixing member 31 may be a member for fixing positions of the case 25 and the heat sink 28 by fastening the case 25 and the heat sink 28. For example, the fixing member 31 may be a screw as in the illustrated example. The wiring 32 is wiring for connecting the substrate 27 to a power supply section.

The UV irradiator 23 may include a cooling section in place of the heat sink 28 or in addition to the heat sink 28. For example, the cooling section is a blower such as a fan.

As shown in Fig. 21, the window 26 is arranged with respect to the light source 24 across a gap. For example, the gap may be a distance of around 0.1 mm to 50 mm. The light source 24 is protected by the window 26.

The window 26 in the illustrated example has a disk-like or circular shape. As a modification, for example, the window 26 may have a rectangular parallelepiped shape or a lens-like shape. According to the window 26 having a lens-like shape, light radiated from the light source 24 can be focused and a relatively small beam angle can be achieved.

The window 26 is configured with reduced thickness. However, the window 26 is preferably thick enough to withstand shock which may possibly be generated when the hand dryer 1 is used by the user or during cleaning or maintenance of the hand dryer 1. Generally, when the thickness of the window 26 increases, transmittance of the window 26 tends to decrease. Therefore, the thickness of the window 26 is, for example, around 0.5 mm to 3 mm. Preferably, the thickness of the window 26 is around 1 mm to 2 mm.

The window 26 includes an incidence plane, an exit plane, and a peripheral plane. The incidence plane is a surface on which a light beam from the light source 24 is incident. The exit plane is a surface on an opposite side to the incidence plane. The exit plane is a surface which causes light incident on the incidence plane to be emitted toward an opposing surface of the hand insertion section 3 or toward the hand insertion space 5. The peripheral plane is a surface positioned to the side of the incidence plane. The peripheral plane is a surface positioned to the side of the exit plane. The incidence plane may be parallel to the exit plane. The peripheral plane may be perpendicular to the incidence plane and the exit plane. A direction from the incidence plane to the exit plane is the thickness direction. For example, a dimension of the window 26 with respect to the thickness direction is preferably within the ranges described above (0.5 mm to 3 mm or 1 mm to 2 mm).

When the window 26 has a lens-like shape, surfaces having curved surfaces of the lens shape are the incidence plane and the exit plane. In addition, when the window 26 has a lens-like shape, the window 26 has a central axis that passes through a projecting portion of the lens-like shape. The central axis of the window 26 or an imaginary extension line of the central axis intersects with the light source 24. In other words, the window 26 with the lens shape and the light source 24 are arranged on a straight line. The central axis of the window 26 or an imaginary extension line of the central axis may pass through another window. In other words, the window 26 with the lens shape and the other window may be arranged on a straight line.

A light-emitting surface of the light source 24 is desirably parallel to the incidence plane of the window 26. When the light-emitting surface of the light source 24 is not parallel to the incidence plane of the window 26, transmittance may decline and illuminance of light emitted from the exit plane of the window 26 to the hand insertion section 3 or the hand insertion space 5 may decline. When the light-emitting surface of the light source 24 is parallel to the incidence plane of the window 26, a decline in transmittance can be reliably suppressed.

The light-emitting surface of the light source 24 is provided at a position in proximity to the incidence plane of the window 26. Since light beams from the light source 24 advance radially, when a distance between the light source 24 and the incidence plane of the window 26 increases, the number of light beams that are not incident on the window 26 may increase. As a result, illuminance of the irradiated hand insertion section 3 or the hand insertion space 5 may possibly decline. In order to make all of the light beams from the light source 24 incident on the window 26, the window 26 must be given a larger size as the distance between the light source 24 and the window 26 increases. When the distance between the light source 24 and the window 26 is short, since all of or most of the light beams from the light source 24 can be caused to be incident on the window 26 even when the size of the window 26 is small, illuminance of the hand insertion section 3 or the hand insertion space 5 can be increased.

The window 26 is made of a material that transmits at least ultraviolet light of a part of the wavelengths among ultraviolet light generated by the light source 24. The window 26 is desirably made of a material with high ultraviolet transmittance. Transmittance refers to a percentage of incident light with a particular wavelength passing through the window 26. Incident light not having passed through is reflected or absorbed by the window 26. A sum of transmittance, reflectance, and absorptance is 100%. For example, the transmittance of the window 26 with respect to wavelengths of UVA or UVB is preferably 80% or higher and more preferably 90% or higher. For example, the transmittance of the window 26 with respect to a major portion of wavelengths of UVA and UVB is preferably 80% or higher and more preferably 90% or higher. In addition, for example, the transmittance of the window 26 with respect to wavelengths of 200 nm or higher among UVC is preferably 80% or higher and more preferably 90% or higher. In addition, the transmittance of the window 26 with respect to a major portion of wavelengths of 200 nm or higher among UVC is preferably 80% or higher and more preferably 90% or higher. In addition, the transmittance of the window 26 with respect to wavelengths from 250 nm to 285 nm is preferably 80% or higher and more preferably 90% or higher. In addition, the transmittance of the window 26 with respect to a major portion of wavelengths from 250 nm to 285 nm is preferably 80% or higher and more preferably 90% or higher. In addition, the transmittance of the window 26 with respect to the dominant wavelength of the light source 24 is preferably 80% or higher and more preferably 90% or higher.

Fig. 22 is a sectional view of the UV irradiator 23 included in the hand dryer 1 according to the first embodiment. The hand dryer 1, the hand insertion section 3, or the UV irradiator 23 includes a screw 33. The screw 33 corresponds to fixing means for fixing the UV irradiator 23 to the hand insertion section 3. The UV irradiator 23 is attachably and detachably fixed to the hand insertion section 3 by attachable and detachable fixing means such as the screw 33. Note that the UV irradiator 23 may be fixed to the hand insertion section 3 by fixing means other than the screw 33 as long as the fixing means is attachable and detachable. In the example shown in Fig. 22, the UV irradiator 23 is installed so that the window 26 and the case 25 of a portion corresponding to a window frame of the window 26 are exposed to the hand insertion space 5 from an opening 3j formed in the hand insertion section 3. The hand insertion section 3 has a boss 3k that protrudes from a rear surface on an opposite side to a front surface facing the hand insertion space 5. The UV irradiator 23 is fixed to the hand insertion section 3 by fastening the case 25 to the boss 3k using the screw 33.

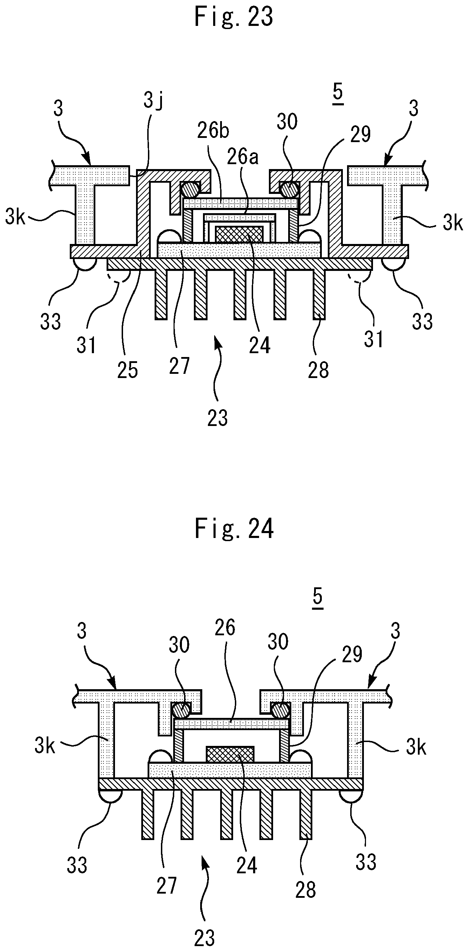

One UV irradiator 23 may include a plurality of windows 26. The plurality of windows 26 may be arranged parallel to each other or arranged approximately parallel to each other. Fig. 23 is a sectional view showing a modification of the UV irradiator 23 included in the hand dryer 1 according to the first embodiment. The UV irradiator 23 shown in Fig. 23 includes a first window 26a and a second window 26b. The first window 26a and the second window 26b correspond to a plurality of the windows 26. The first window 26a covers the light source 24. The second window 26b covers the first window 26a.

The light source 24, the first window 26a, and the second window 26b are arranged in a straight line. Preferably, the second window 26b is arranged parallel to the first window 26a. Preferably, an incidence plane of the first window 26a is arranged parallel to the light-emitting surface of the light source 24. For example, the first window 26a and the second window 26b are arranged at positions intersecting the optical axis of the light source 24 or an imaginary extension line of the optical axis. At least one of the plurality of windows 26 included in one UV irradiator 23 may have a lens-like shape. The first window 26a is arranged at a position closer to the light source 24 than the second window 26b. The second window 26b is arranged at a position farther from the light source 24 than the first window 26a. By cooperating with the sealing member 30, the second window 26b prevents water or foreign objects from penetrating into the UV irradiator 23 and into the main body enclosure 2. In addition, the second window 26b has a function of preventing a hand of a person or the like from coming into contact with the light source 24. The first window 26a has a function of preventing water, foreign objects, a hand of a person, or the like from coming into contact with the light source 24. In addition, the first window 26a has a function of protecting the light source 24.

As long as the sealing property described above is secured, a configuration in which one UV irradiator 23 includes only one window 26 is more preferable. The window 26 reflects or absorbs a part of a light beam. Therefore, the smaller the number of windows 26, the more efficiently the hand insertion section 3 or the hand insertion space 5 can be irradiated with ultraviolet light. In particular, when there is only one window 26, the hand insertion section 3 or the hand insertion space 5 can be even more efficiently irradiated with ultraviolet light.

The window 26 may have a property of not transmitting ultraviolet light with short wavelengths. In addition, the window 26 may have a property of not transmitting ultraviolet light with short wavelengths due to a filter or a band pass. For example, the window 26 may have a property of not transmitting wavelengths of 180 nm or less. In addition, the window 26 may have a property of not transmitting wavelengths of 150 nm or less. Ultraviolet light with short wavelengths may have an adverse effect on the human body. Using the window 26 with a property of not transmitting ultraviolet light with short wavelengths further increases the level of safety.

The window 26 is preferably made of a material with high UV transmittance. For example, the window 26 may be made of quartz glass. For example, the window 26 may be made of synthetic quartz glass. For example, the window 26 may be made of UV-protective glass which partially blocks UV. For example, the window 26 may be made of a resin material with high UV transmittance. For example, the window 26 may be made of a fluorine resin. Examples of a fluorine resin include PFA, FEP, ETFE, and PCTFE.

An antireflective film may be formed on at least one of the incidence plane and the exit plane of the window 26. An antireflective film prevents reflection of light incident on the incidence plane from a light source and is generally referred to as AR coating (Anti Reflection Coating). Since antireflective coating is known art, a description of a mechanism thereof will be omitted. Light not reflected is either transmitted or absorbed. For example, a major portion of light not reflected is transmitted and a part of the light not reflected is absorbed. For example, when transmittance of quartz glass with respect to the UVB range is 90%, applying processing due to antireflective coating on one side increases the transmittance to 94% and applying processing due to antireflective coating on both sides increases the transmittance to around 98%. Therefore, by providing the window 26 with antireflective coating, the hand insertion section 3 or the hand insertion space 5 can be more efficiently irradiated with ultraviolet light. Besides quartz glass, processing due to antireflective coating may be applied to materials to be used as the window 26. For example, processing due to antireflective coating may be applied to a fluorine resin of which transmittance is higher than general materials but lower than quartz glass. Accordingly, transmittance can be increased while suppressing cost.

For example, the window 26 is made of a transparent material, a translucent material, or a material with high transparency. For example, the window 26 is made of a material with higher UV transmittance than a major portion of the hand insertion section 3. For example, the window 26 is made of a material with higher UV transmittance than a major portion of a portion facing the hand insertion space 5 among the hand insertion section 3.

In addition, the window 26 may be subjected to filter processing for reducing radiant intensity of a specific wavelength. An example of a filter is a band-pass filter. For example, filter processing may be applied to the window 26 in order to reduce wavelengths that are harmful to the human body.

The window 26 is arranged at a position close to a surface facing the hand insertion space 5 among the hand insertion section 3. The window 26 may be configured to increase airtightness with the hand insertion section 3 by cooperating with the fixing member 31 or the case 25. The window 26 may be arranged so that a thickness direction or a central axis thereof is parallel to the horizontal direction. In addition, the window 26 may be arranged so that a thickness direction or a central axis thereof is not parallel to the horizontal direction. In this case, the window 26 is desirably arranged in an inclined posture so that an upper side of the window 26 is positioned farther to the side of the hand insertion space 5 than a lower side of the window 26. For example, when the window 26 is arranged at a position close to a surface of the insertion front section 3b, the posture of the window 26 is desirably inclined so that the upper side of the window 26 is positioned farther to a rear side than the lower side of the window 26. In addition, when inclining the window 26, the central axis of the window 26 is desirably arranged parallel to the optical axis of the light source 24. In other words, the optical axis of the light source 24 is desirably also inclined. In this case, the optical axis of the light source 24 is inclined downward relative to horizontal. Therefore, ultraviolet light can be emitted toward a slightly lower side of the hand insertion section 3 and upward leakage of a light beam can be suppressed. In addition, the insertion bottom section 3a with a large amount of adhesion of water droplets can be irradiated with ultraviolet light, an amount of microorganisms contained in water droplets scattered from the insertion bottom section 3a can be reduced, and hygiene is improved.

The window 26 may be fixed so as to be fitted into an opening provided in the hand insertion section 3. A surface of the hand insertion section 3 on which the window 26 is arranged and the window 26 may be arranged in an approximately same plane. The window 26 may be arranged at a position closer to the cover section than the hand insertion section 3 in a periphery of the window 26 or, in other words, at a position far from the hand insertion space 5. In other words, the window 26 may be arranged at a position that is recessed from the hand insertion section 3 in the periphery of the window 26 or a position that is recessed by one step.

For example, when the window 26 is arranged on a surface of the insertion front section 3b, the window 26 is arranged at a position closer to the front cover 2a than the insertion front section 3b in the periphery of the window 26 or to the front of the insertion front section 3b. In this manner, by arranging the window 26 at a position that is farther from the hand insertion space 5 than the hand insertion section 3 in a periphery of the window 26 or at a recessed position, a hand is less likely to come into contact with the window 26 when inserting the hand and the window 26 is less likely to be damaged. Accordingly, it is easier to prevent transmittance of the window 26 from declining due to damage sustained by the window 26 or the like. In addition, an edge of the opening of the hand insertion section 3 in the periphery of the window 26 may have a shape that protrudes toward the side of the hand insertion space 5 by one step. Accordingly, a hand is less likely to come into contact with the window 26 when inserting the hand and the window 26 is less likely to be damaged.

A distance between the window 26 and the hand insertion section 3 in which the window 26 is arranged is kept constant in order to increase adhesion. For example, the window 26 is separated from the hand insertion section 3 by a distance corresponding to the thickness of the sealing member 30. For example, when the thickness direction or the central axis of the window 26 is parallel to a horizontal direction or, in other words, when the window 26 extends along a vertical direction, the hand insertion section 3 in a periphery of the window 26 also extends along the vertical direction. On the other hand, when the upper side of the window 26 is closer to the side of the hand insertion space 5 than the lower side of the window 26 as described earlier, even in the hand insertion section 3 in the periphery of the window 26, the hand insertion section 3 at a position close to the upper side of the window 26 is arranged at a position closer to the hand insertion space 5 than the hand insertion section 3 at a position close to the lower side of the window 26. In other words, the hand insertion section 3 may have a shape that partially bulges toward the side of the hand insertion space 5. Accordingly, the adhesion between the window 26 and a periphery of the hand insertion section 3 in which the window 26 is arranged can be maintained at a higher level. A portion between the bulged position of the hand insertion section 3 and a periphery of the bulged position is desirably formed smoothly. Accordingly, water is less likely to pool at the bulged position.

The hand insertion section 3 in a periphery of and above the window 26 may include a projecting section which is projected toward the side of the hand insertion space 5. Accordingly, a light beam emitted from the light source 24 is prevented from being emitted directly upward. Accordingly, an amount of upward leakage of the light beam can be reduced.

The case 25 is a component which constitutes an outer appearance of the UV irradiator 23. The sealing member 30, the window 26, the light source 24, the spacer 29, and the substrate 27 are arranged between the case 25 and the heat sink 28. The case 25 is arranged so as to come into contact with the hand insertion section 3. The case 25 may be fixed so as to come into contact with the hand insertion section 3 via the fixing member 31. The case 25 may be fixed to the hand insertion section 3 without involving the fixing member 31. For example, the case 25 may be fixed to the hand insertion section 3 using a press-fitting or snap-fitting technique. Alternatively, the case 25 may be fixed to the hand insertion section 3 by screwing a male screw provided in an outer periphery of the case 25 to a female screw provided in an inner periphery of an opening formed in the hand insertion section 3.

The UV irradiator 23 need not necessarily include the case 25. A component of the UV irradiator 23 other than the case 25 may be fixed to the hand insertion section 3. The case 25 is arranged at a position equivalent to a surface of the hand insertion section 3 or arranged at a deeper position than the surface of the hand insertion section 3. For example, compared to the hand insertion section 3 in the periphery of the case 25, the case 25 is arranged at a position of equivalent distance from the hand insertion space 5 or a position of a slightly longer distance from the hand insertion space 5. Accordingly, a hand of the user is less likely to come into contact with the case 25 when the user inserts the hand in the hand insertion space 5 and is hygienic. The case 25 has an opening near a center thereof. The opening is for passing light emitted from the light source 24. The case 25 may include a groove for fixing the sealing member 30. The sealing member 30 is positioned in a circumferential direction of the sealing member 30 by fitting the sealing member 30 into the groove.

The fixing member 31 included in the UV irradiator 23 is, for example, a screw. An object other than a screw may be used as the fixing member 31. For example, the fixing member 31 is for fixing the UV irradiator 23 to the hand insertion section 3.

In the present disclosure, a sealing member for filling a gap between the hand insertion section 3 and the UV irradiator 23 may be provided. Hereinafter, the sealing member and the sealing member 30 described earlier will be collectively referred to as the "sealing member 30". For example, the sealing member 30 is made of a softer material than the hand insertion section 3. For example, the sealing member 30 is made of a softer material than a major portion of the hand insertion section 3. The sealing member 30 may be a component that is generally referred to as a packing, an O-ring, a gasket, or the like. For example, the sealing member 30 is a soft material such as rubber, silicon, or an elastomer. The sealing member 30 may be an integrated component having been insert-molded with respect to the hand insertion section 3. The UV irradiator 23 is fixed to the hand insertion section 3 via the fixing member 31 or the like. The sealing member 30 may be provided in plurality. For example, the sealing member 30 is interposed between the hand insertion section 3 and the case 25 in order to seal the hand insertion section 3 and the case 25. For example, the sealing member 30 is interposed between the hand insertion section 3 and the window 26 in order to seal the hand insertion section 3 and the window 26. For example, the sealing member 30 is interposed between the case 25 and the window 26 in order to seal the case 25 and the window 26. A cross section of the sealing member 30 is slightly squashed when the UV irradiator 23 is fixed by the fixing member 31. For example, a sectional area of the sealing member 30 decreases by around 10% to 20% due to compression. Sealing property is improved due to deformation of the sealing member 30 in this manner.

Water scattered from a wet hand adheres to the hand insertion section 3. When the sealing member 30 is absent, there is a possibility that water may penetrate into the main body enclosure 2 or the UV irradiator 23 from a small gap between the hand insertion section 3 and the UV irradiator 23. When the sealing member 30 is absent, there is a possibility that water may penetrate into the main body enclosure 2 or the UV irradiator 23 from a small gap between the case 25 and the window 26. When water adheres to the UV irradiator 23, a failure may occur. Using the sealing member 30 described above enables penetration of water to be suppressed.

The heat sink 28 is a component for dissipating heat of the substrate 27 and the light source 24 to cool the substrate 27 and the light source 24 and suppress temperature rises thereof. The heat sink 28 is mounted to a surface on an opposite side to the light source 24 or in a vicinity of the surface with respect to the substrate 27. The heat sink 28 is fixed so as to come into direct or indirect contact with the substrate 27. At least a part of the substrate 27 or a part of the heat sink 28 may be positioned so as to be exposed to a blowout air path or an exhaust air path. Accordingly, the substrate 27 or the heat sink 28 can be cooled by an airflow during actuation of the hand dryer 1. As a result, in addition to heat dissipation by the heat sink 28 due to natural convection during normal time, heat dissipation efficiency can be further increased by forced convection when the user uses the hand dryer 1. In addition, when a hand is not inserted, the substrate 27 or the heat sink 28 may be cooled by performing a breeze operation of the blower 10. A breeze operation may be an operation that produces an air volume or an air speed that is equivalent to a minimum air volume or a minimum air speed set by the hand dryer 1 in order to dry a hand by sending dry air to the hand or an operation that produces an air volume or an air speed that is even lower than the minimum air volume or the minimum air speed. Alternatively, a breeze operation may be an operation that produces an operating sound that is equivalent to an operation with a quietest operating sound among the operations set by the hand dryer 1 in order to dry a hand by sending dry air to the hand or an operation that produces an operating sound that is even quieter than the quietest operation. According to a breeze operation, the substrate 27 or the heat sink 28 can be cooled even when a hand is not inserted. As means for adjusting air volume, a motor of the blower 10 is desirably a brushless motor which enables air volume to be readily controlled.

The substrate 27 causes the light source 24 to emit light. The substrate 27 is electrically connected to the light source 24. The substrate 27 is electrically connected to the power supply section. The substrate 27 may have a plate-like shape. For example, the optical axis of the light source 24 is perpendicularly arranged with respect to the substrate 27. Other various electronic components or electric components may be connected to the substrate 27. The substrate 27 may be solid enough to not deform when an external force is applied such as when force is applied by one hand. Alternatively, the substrate 27 may be rigid enough to deform when force is applied by one hand. In addition, the substrate 27 may be rigid enough to bend under its own weight. For example, when thickness of a part of the substrate 27 is 1 mm or less, stiffness of the portion is low. For example, when the thickness of a part of the substrate 27 is 0.1 mm or less, stiffness of the portion is even lower. In addition, when the substrate 27 has a notched shape, stiffness declines. A part of the substrate 27 may have a film-like thin portion. Such a configuration enables the substrate 27 to readily deform and, at the same time, maintain a bent shape.

For example, when arranging two light sources 24 on one plate-like substrate 27, optical axes of the light sources 24 are to be arranged parallel to each other. However, in order to irradiate the entire hand insertion section 3 with ultraviolet light, it is more efficient to emit the ultraviolet light so that the optical axes of the two light sources 24 are not parallel to each other. In this case, by mounting one light source 24 to one substrate 27 and arranging two substrates 27 at different angles, the two light sources 24 are arranged so that respective optical axes are in a non-parallel state. For example, when the stiffness of the substrate 27 is low as described above, the substrate 27 can be used as a component of the UV irradiator 23 while maintaining a curved state. Accordingly, a plurality of light sources 24 can be arranged on a single substrate 27 and the optical axes of the plurality of light sources 24 can be arranged in different orientations. Accordingly, the number of substrates 27 can be reduced and space saving can be achieved.

The spacer 29 has a function of maintaining a constant distance between the substrate 27 and the window 26. For example, the spacer 29 is made of a resin material or a metal material. A shape of the spacer 29 may be a hollow cylindrical shape or a hollow square cylindrical shape. The spacer 29 may have an external shape equivalent to that of the window 26. For example, when the external shape of the window 26 is a circle, the shape of the spacer 29 may be a hollow cylindrical shape. In addition, when the external shape of the window 26 is a rectangular parallelepiped shape, the shape of the spacer 29 may be a hollow square cylindrical shape. The substrate 27 is in contact with a side of one end of the spacer 29. The window 26 is in contact with a side of another end of the spacer 29.

The spacer 29 has a thickness direction, a width direction, and a length direction. When the spacer 29 has a hollow cylindrical shape, a length of the spacer 29 in the width direction is equal to a length of the spacer 29 in the length direction. A length of the spacer 29 in the thickness direction is shorter than the length of the spacer 29 in the width direction and, at the same time, shorter than the length of the spacer 29 in the length direction. For example, among three mutually orthogonal axes in a x direction, a y direction, and a z direction, a dimension in a direction with a shortest length corresponds to a dimension of the spacer 29 in the thickness direction. The spacer 29 has opposing faces in the thickness direction. The length of the spacer 29 in the thickness direction is longer than a length of the light source 24 in the thickness direction. The spacer 29 has an axis along the thickness direction. The axis of the spacer 29 is desirably parallel to the optical axis of the light source 24. The axis of the spacer 29 may be a central axis. In addition, the light source 24 and the spacer 29 are preferably arranged so that an imaginary extension line of the optical axis of the light source 24 and an imaginary extension line of an axis in the thickness direction of the spacer 29 pass through the insertion bottom section 3a or an opposing surface. The spacer 29 may have a hollow shape and the light source 24 may be arranged inside the spacer 29 or, in other words, in the hollow portion of the spacer 29. The light source 24 and the spacer 29 are preferably arranged so that the thickness direction of the spacer 29 coincides with or approximately coincides with the thickness direction of the light source 24. A surface on one side in the thickness direction of the spacer 29 corresponds to a surface close to the hand insertion space 5, and a surface opposing the surface on the one side corresponds to a surface that is far from the hand insertion space 5. The spacer 29 and the light source 24 are in contact with the substrate 27. The surface that is far from the hand insertion space 5 in the thickness direction of the spacer 29 and the surface that is far from the hand insertion space 5 in the thickness direction of the light source 24 are in contact with the substrate 27. In other words, the surface that is far from the hand insertion space 5 in the thickness direction of the spacer 29 and the surface that is far from the hand insertion space 5 in the thickness direction of the light source 24 are arranged on a same plane or arranged on approximately the same plane. Due to a connection between this fact and the fact that the length of the spacer 29 in the thickness direction is longer than the length of the light source 24 in the thickness direction, the surface close to the hand insertion space 5 in the thickness direction of the spacer 29 is arranged at a position closer to the hand insertion space 5 than the surface close to the hand insertion space 5 in the thickness direction of the light source 24. Accordingly, a light beam emitted from the light source 24 strikes the spacer 29 and is reflected on a front side of the surface close to the hand insertion space 5 in the thickness direction of the light source 24.

The light source 24 is mounted to the surface of the substrate 27. The light source 24 is arranged so as to be surrounded by the substrate 27, the window 26, and the spacer 29. Accordingly, damage to the light source 24 due to shock from outside of the light source 24 can be more reliably prevented. The spacer 29 may be made of a material with high ultraviolet reflectance. The spacer 29 may be configured to reflect at least light of a dominant wavelength among light generated by the light source 24. For example, when the reflectance of the spacer 29 is low such as when the spacer 29 has high absorptance, a part of light beams is absorbed by the spacer 29 and illuminance of light beams reaching the hand insertion section 3 declines. In addition, for example, when the reflectance of the spacer 29 is low such as when the spacer 29 has high transmittance, the window 26 with a larger size is required in order to cause the hand insertion section 3 to be irradiated with light beams emitted across a wide range from the light source 24. When the window 26 is large, thickness of the window 26 must be increased in order to secure strength of the window 26. When the thickness of the window 26 increases, transmittance of the window 26 decreases. Using the spacer 29 with high reflectance with respect to wavelength of ultraviolet light generated by the light source 24 in use, light beams emitted across a wide angle can be reflected, the size of the window 26 can be suppressed, the thickness of the window 26 can be reduced, and a decline in illuminance can be prevented. Examples of materials with high reflectance include fluorine resin in the case of resins and aluminum and members subjected to surface treatment such as anodizing and evaporation coating in the case of metal. Reflectance being high means that, for example, reflectance with respect to the dominant wavelength of the light source 24 is 80% or higher or preferably 90% or higher. Alternatively, reflectance being high may mean that reflectance is relatively high when compared with other materials used in the hand insertion section 3.

Note that the spacer 29 need not have a hollow shape. Although the light source 24 is desirably surrounded by the spacer 29 over 360 degrees or across an entire periphery, the light source 24 is not limited to such a configuration. For example, the light source 24 may be surrounded by a plurality of spacers 29. For example, the light source 24 may be surrounded by a plurality of spacers 29 provided at intervals. For example, with the center and an optical axis of the light source 24 as center, the light source 24 may be surrounded by the spacer 29 across a range of 180 degrees or more or the light source 24 may be surrounded by the spacer 29 across a range of 270 degrees or more. Instead of a circular or square shape, for example, the spacer 29 may have a partially-missing columnar shape such as a C-shape, a U-shape, or an arch shape. However, the shape of the spacer 29 is preferably a hollow circular shape or a hollow square shape with four sides closed off. This is because the spacer 29 cooperates with the window 26 or the sealing member 30 to constitute a structure which discourages water or dust from entering the light source 24. For the same reason, when the spacer 29 has a partially-missing shape, the missing range of the shape is preferably as small as possible. In addition, a position of the missing portion of the spacer 29 is preferably a position on a lower side in the vertical direction. For example, the missing portion of the spacer 29 may be present in a range on a lower side in the vertical direction than the center of the light source 24. For example, the spacer 29 may be missing in a range on a lower side of a lower end of the light source 24 with respect to a position in the vertical direction. For example, the shape of the spacer 29 may be a hollow, square columnar, C-shape or a U-shape with a bottom side or, in other words, a lower-side portion missing. A part of light beams emitted from the light source 24 arranged inside or, in other words, in a hollow portion of the spacer 29 having a hollow cylindrical shape or a hollow square columnar shape strikes the spacer 29 and is reflected by the spacer 29. Light beams striking the spacer 29 just once and being reflected by the spacer 29 advance toward the side of the hand insertion section 3. At this point, a light beam striking a portion on a lower side in the vertical direction among the spacer 29 just once and being reflected by the spacer 29 and subsequently advancing toward the side of the hand insertion section 3 without striking the spacer 29 advances upward in the vertical direction. Such a light beam does not strike the hand insertion section 3 and ends up being emitted into a space where the hand dryer 1 is installed. By comparison, with the spacer 29 of which a portion on a lower side in the vertical direction is missing, light beams that strike the spacer 29 and are reflected by the spacer 29 to be emitted into the space where the hand dryer 1 is installed can be reduced. Accordingly, a risk of exposure to radiation for persons in the periphery can be reduced.

Fixation of the UV irradiator 23 is not limited to the fixing member 31. For example, the fixing member 31 may be a part of the hand dryer 1 or a part of the hand insertion section 3. The fixing member 31 may be a part of the case 25 or a part of the heat sink 28. In addition, the fixing member 31 need not be provided as long as the UV irradiator 23 can be fixed.

Figs. 24 to 27 are each a sectional view showing another modification of the UV irradiator 23 included in the hand dryer 1 according to the first embodiment. The modification of the UV irradiator 23 shown in each of Figs. 24 to 27 is an example that does not include the case 25. In the modification according to each of Figs. 24 to 27, the UV irradiator 23 is fixed to the hand insertion section 3 by fastening the heat sink 28 to the boss 3k of the hand insertion section 3 using the screw 33.

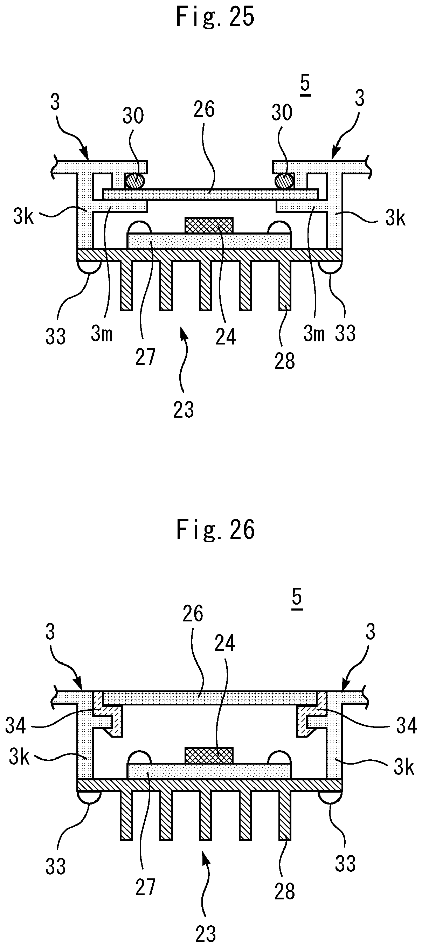

In the modification shown in Fig. 24 and the modification shown in Fig. 25, the sealing member 30 seals a gap between the window 26 and the hand insertion section 3.

The modification shown in each of Figs. 25 to 27 is an example that does not include the spacer 29. In these modifications, the window 26 is held by the hand insertion section 3.

In the modification shown in Fig. 25, the window 26 is supported by a projecting section 3m that projects from the boss 3k to an inner circumferential side.

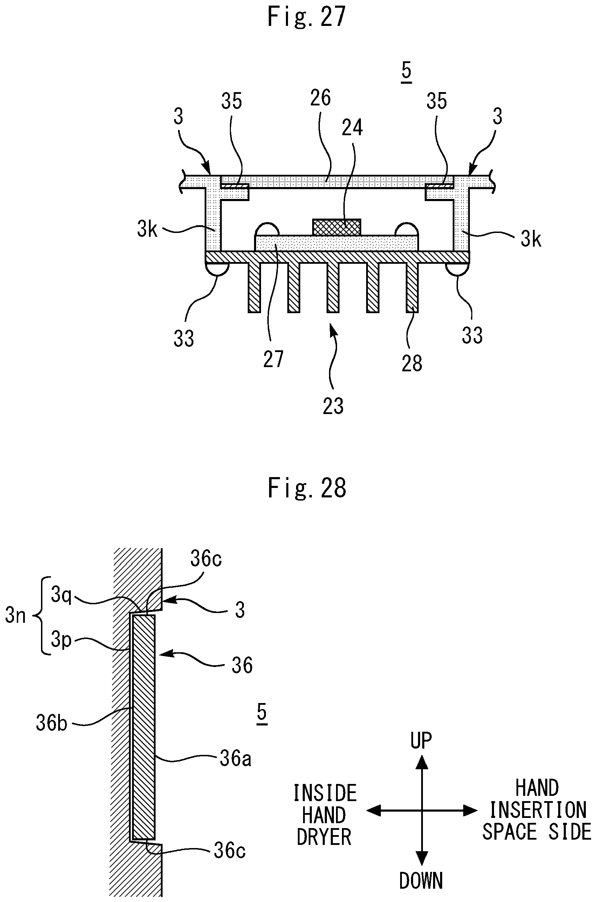

The modification shown in each of Figs. 26 and 27 is an example that does not include the sealing member 30.

In the modification shown in Fig. 26, the window 26 is mounted to the hand insertion section 3 using a bushing 34. An outer circumference section of the bushing 34 fits with the inner circumferential section of the opening formed in the hand insertion section 3. An outer circumference section of the window 26 fits with the inner circumferential section of the bushing 34. For example, the bushing 34 has a structure similar to that of a cable bushing. The bushing 34 may also have a function of sealing the gap between the window 26 and the hand insertion section 3. In this case, the window 26 is mounted by pushing the window 26 toward the light source 24 from the side of the hand insertion space 5.

The hand dryer 1 may be configured such that the UV irradiator 23 is detachable. In other words, the UV irradiator 23 may be configured to be detachable from the hand insertion section 3 of the hand dryer 1. In addition, the hand dryer 1 may be configured such that a main body portion excluding the UV irradiator 23 and the UV irradiator 23 among the hand dryer 1 can be separated from each other and the UV irradiator 23 can be detached from the main body portion. The hand dryer 1 may be configured such that the UV irradiator 23 is detachable and can be replaced with a new UV irradiator 23. Accordingly, when the light source 24 reaches the end of its lifetime and performance declines, the performance can be restored by replacing the UV irradiator 23 with a new UV irradiator 23. In addition, replacing with a new UV irradiator 23 also enables performance of the window 26, the sealing member 30, or the like to be restored. Furthermore, detaching the fixing member 31 enables the light source 24 or the window 26 to be replaced while keeping the case 25 or the sealing member 30 mounted to the hand dryer 1. In other words, the entirety of the UV irradiator 23 may be readily replaced or a part of the UV irradiator 23 may be readily replaced in a selective manner in accordance with a portion to be detached or as needed. In addition, by removing the screw 33 or the fixing member 31 as fixing means, a light source section including the light source 24 can be detached. In other words, the light source section can be detached according to a plurality of methods or by detaching a plurality of portions. As already described, a portion on an upper side of the light source 24 or the window 26 is preferably positioned farther to the side of the hand insertion space 5 than a portion on a lower side of the light source 24 or the window 26. In other words, with the UV irradiator 23 as a whole, the portion on an upper side may be positioned farther to the side of the hand insertion space 5 than the portion on a lower side. In this case, since the sealing member 30 is less likely to fall off when detaching the fixing member 31 and replacing the light source 24 or the window 26, work for attachment/detachment and replacement can be readily performed.

In the modification shown in Fig. 27, the window 26 is mounted to the hand insertion section 3 using a two-sided adhesive tape 35. A peripheral edge section of the window 26 is bonded to an edge section of the opening formed in the hand insertion section 3 using the two-sided adhesive tape 35. The two-sided adhesive tape 35 seals the gap between the window 26 and the hand insertion section 3.

Removing the screw 33 or the fixing member 31 as fixing means enables the window 26 or the UV irradiator 23 to be detached. In this manner, the hand dryer 1 may be configured such that the window 26 is detachable. The hand dryer 1 may be configured such that the window 26 is detachable and can be replaced with a new window 26. Accordingly, when the window 26 deteriorates and transmittance declines, the transmittance can be restored by replacing the window 26 with a new window 26. When the window 26 and the UV irradiator 23 are separate bodies, a configuration may be adopted in which the window 26 can be detached without detaching the UV irradiator 23. In addition, when the window 26 and the UV irradiator 23 are integrated, the window 26 may be placed with a new window 26 by replacing the entire UV irradiator 23 with a new UV irradiator 23.

A surface of the hand insertion section 3 facing the hand insertion space 5 may be white. Alternatively, the surface of the hand insertion section 3 facing the hand insertion space 5 may be black or yellow. Alternatively, the surface of the insertion bottom section 3a may have a different color from the surface of the insertion peripheral section. In addition, the surface of the insertion bottom section 3a may be black or yellow, and the surface of the insertion peripheral section may be a different color from the insertion bottom section 3a such as white. When the hand insertion section 3 is exposed to ultraviolet light over a long period of time due to ultraviolet irradiation by the UV irradiator 23, discoloration of the hand insertion section 3 may occur. In particular, when the hand insertion section 3 is white, the user is more likely to notice a change in color and get a poor impression. The insertion bottom section 3a is preferably irradiated with ultraviolet light in a concentrated manner. Therefore, in particular, the color of the insertion bottom section 3a is preferably a color of which a change due to ultraviolet irradiation is less likely to be noticed by the user or a color of which discoloration is less likely to occur.

The hand dryer 1 may further include output change means configured to reduce an output of the light source 24 or change the output of the light source 24 to zero in accordance with a detection by the hand detection section 21. Ultraviolet light may be harmful when striking a human body. By having the output change means reduce an output of the light source 24 or change the output of the light source 24 to zero in accordance with a detection of a hand by the hand detection section 21, ultraviolet light can be more reliably prevented from striking a human body. The output change means is capable of adjusting the output of the light source 24 by adjusting a current of the light source 24. For example, the output change means according to the present embodiment may be achieved by the control section 22 or by a control section (not illustrated) other than the control section 22.

In this case, the hand detection section 21 is capable of detecting the presence or absence of a hand inserted into the hand insertion space 5 of the hand insertion section 3 or a hand arranged on the hand insertion section 3. The control section 22 of the hand dryer 1 may be configured to cause the UV irradiator 23 to emit ultraviolet light when the hand detection section 21 detects that a hand is absent. Causing the UV irradiator 23 to emit ultraviolet light when a hand is absent in the hand insertion section 3 more reliably satisfies both safety and hygiene.

As shown in Fig. 10, the hand dryer 1 may further include a human detection section 39. The human detection section 39 is an example of human detection means for detecting a human body having approached the hand dryer 1. For example, the human detection section 39 may include a human sensor installed in the main body enclosure 2. Note that the hand dryer according to the present disclosure need not include human detection means. For example, the human detection section 39 detects a human body present at a position closer to the hand dryer 1 than a predetermined distance. For example, the human detection section 39 detects a movement of a human body present at a position closer to the hand dryer 1 than a predetermined distance. For example, the human detection section 39 detects a person standing in a predetermined region with respect to the hand dryer 1. For example, the human detection section 39 detects a posture of a human body. For example, the human detection section 39 detects a posture of a user immediately before inserting a hand into the hand insertion space 5 of the hand insertion section 3. For example, the human detection section 39 detects that one hand or one arm, or both hands or both arms, are positioned forward or upward as compared to a person standing upright in an ordinary posture. For example, when the user inserts a hand into the hand insertion space 5 of the hand insertion section 3, the human detection section 39 detects the presence of the user before the hand detection section 21 detects the hand. For example, the human detection section 39 may detect a hand.

The hand dryer 1 may further include output change means configured to reduce an output of the light source 24 or change the output of the light source 24 to zero in accordance with a detection by the human detection section 39. By having the output change means reduce an output of the light source 24 or change the output of the light source 24 to zero in accordance with a detection of a person by the human detection section 39, ultraviolet light can be more reliably prevented from striking a human body.

An ultraviolet irradiation amount is proportional to a product of illuminance and an irradiation time. Assuming that illuminance is constant, an irradiation time corresponding to an ultraviolet irradiation amount necessary for sufficiently sterilizing the inside of the hand insertion section 3 can be calculated.

When a total irradiation time of the UV irradiator 23 becomes longer, output and illuminance may decline due to a deterioration of the light source 24 approaching its lifetime or a decline in transmittance due to a deterioration of the window 26. In addition, constituent materials of the hand insertion section 3 may deteriorate due to being subjected to ultraviolet irradiation. In order to avoid such events as much as possible, when an irradiation time of one irradiation of the UV irradiator 23 reaches a time necessary for sufficiently sterilizing the inside of the hand insertion section 3, it is desirable to reduce an output of the light source 24 or change the output of the light source 24 to zero. Hereinafter, the irradiation time of one irradiation necessary for sufficiently sterilizing the inside of the hand insertion section 3 will be referred to as a "necessary sterilization time".

The output change means may be configured to reduce an output of the light source 24 or change the output of the light source 24 to zero when a duration of a state where the hand detection section 21 does not detect a hand or a duration of a state where the human detection section 39 does not detect a person exceeds a criterion. When the duration of a state where the hand detection section 21 does not detect a hand or the duration of a state where the human detection section 39 does not detect a person exceeds a criterion, an irradiation time of one irradiation of the UV irradiator 23 is considered to have reached the necessary sterilization time. At this point, by reducing the output of the light source 24 or changing the output of the light source 24 to zero, the irradiation time can be prevented from becoming longer than necessary. Accordingly, there are advantages in terms of extending a lifetime of the light source 24, preventing a decline in transmittance of the window 26, preventing a deterioration of constituent materials of the hand insertion section 3, and the like.

When the hand dryer 1 includes both the hand detection section 21 and the human detection section 39, the output change means may be configured to reduce an output of the light source 24 or change the output of the light source 24 to zero when a duration of a state where the hand detection section 21 does not detect a hand and the human detection section 39 does not detect a person exceeds a criterion. When the duration of a state where the hand detection section 21 does not detect a hand and the human detection section 39 does not detect a person exceeds a criterion, an irradiation time of one irradiation of the UV irradiator 23 is considered to have reached the necessary sterilization time. At this point, by reducing the output of the light source 24 or changing the output of the light source 24 to zero, the irradiation time can be prevented from becoming longer than necessary. Accordingly, there are advantages in terms of extending a lifetime of the light source 24, preventing a decline in transmittance of the window 26, preventing a deterioration of constituent materials of the hand insertion section 3, and the like.

The output change means or the control section 22 may be configured to reduce an output of the light source 24 or to change the output of the light source 24 to zero when a duration of emission of ultraviolet light by the UV irradiator 23 exceeds a criterion. Accordingly, the irradiation time per one irradiation can be prevented from becoming longer than necessary. Accordingly, there are advantages in terms of extending a lifetime of the light source 24, preventing a decline in transmittance of the window 26, preventing a deterioration of constituent materials of the hand insertion section 3, and the like.

The hand dryer 1 may further include irradiation notification means for notifying, when the UV irradiator 23 is emitting the ultraviolet light, emission of the ultraviolet light by the UV irradiator 23. For example, the display section 4 may be used as the irradiation notification means. For example, when the UV irradiator 23 is emitting ultraviolet light, the control section 22 may use the display section 4 to notify the user of the fact that ultraviolet irradiation is in progress. Since ultraviolet light is invisible, when the irradiation notification means is not provided, there is a possibility that the user is unable to perceive whether or not ultraviolet irradiation by the UV irradiator 23 is in progress. By comparison, according to the irradiation notification means, the user can more reliably perceive whether or not ultraviolet irradiation by the UV irradiator 23 is in progress.

The hand dryer 1 may further include hygienic condition notification means for notifying hygienic conditions of the hand insertion section 3. For example, the display section 4 may be used as the hygienic condition notification means. The control section 22 may evaluate the hygienic conditions of the hand insertion section 3 by classifying the hygienic conditions into a plurality of stages. For example, the control section 22 may make an evaluation that the hygienic conditions of the hand insertion section 3 is optimal when an irradiation time by the UV irradiator 23 after the end of previous hand-drying has reached the necessary sterilization time, the control section 22 may make an evaluation that the hygienic conditions of the hand insertion section 3 is good when an irradiation time by the UV irradiator 23 after the end of previous hand-drying has exceeded a first criterion time that is shorter than the necessary sterilization time but has not reached the necessary sterilization time, and the control section 22 may make an evaluation that the hygienic conditions of the hand insertion section 3 is poor when an irradiation time by the UV irradiator 23 after the end of previous hand-drying has not reached the first criterion time. For example, the control section 22 may use the display section 4 to notify the user of the hygienic conditions of the hand insertion section 3 evaluated as described above. When it is notified by the hygienic condition notification means that the hygienic conditions of the hand insertion section 3 is optimal or when it is notified by the hygienic condition notification means that the hygienic conditions of the hand insertion section 3 is good, the user can use the hand dryer 1 with a peace of mind. On the other hand, when it is notified by the hygienic condition notification means that the hygienic conditions of the hand insertion section 3 is poor, the user can avoid taking risks by adopting a measure such as refraining from using the hand dryer 1.

Fig. 28 is a sectional view of a reflection section 36. The hand dryer 1 may further include one or a plurality of the reflection sections 36. More specifically, the hand insertion section 3 may further include the reflection section 36 which reflects ultraviolet light emitted from the UV irradiator 23. The reflection section 36 is arranged so as to face the hand insertion space 5. The reflection section 36 is a member with high reflectance of ultraviolet light. In the present disclosure, "high reflectance of ultraviolet light" corresponds to reflectance with respect to a light beam with a specific ultraviolet wavelength, a wavelength emitted to the reflection section 36, or a dominant wavelength of the light source 24 being 50% or higher, preferably 80% or higher, and more preferably 90% or higher. For example, when a light beam of 10 mW is incident on the reflection section 36, the reflection section 36 reflects 5 mW or more, preferably 8 mW or more, and more preferably 9 mW or more.

Characteristics of reflection of the reflection section 36 may be any of specular reflection and diffuse reflection. Specular reflection refers to reflection like a mirror in which an angle of incidence and an angle of reflection are equal to each other with respect to a reflection surface. For example, with specular reflection, a face appears clearly as though looking at a mirror when looking at the reflection section 36 from the front, and specular reflection can be easily confirmed if an image appears clearly when viewing the reflection section 36. By comparison, diffuse reflection refers to scattered reflection of incident light by a reflection surface in various directions and is perceived as a gloss-less or luster-less surface, a rough surface, or the like. The reflection section 36 may be a member having characteristics of both specular reflection and diffuse reflection.

For example, when using the reflection section 36 that performs specular reflection, light can be reflected toward a specific region. For example, when only one UV irradiator 23 is arranged in the hand insertion section 3, unless reflection is used, there is a possibility that the hand insertion section 3 in the portion where the UV irradiator 23 is arranged may not be sufficiently irradiated with ultraviolet light. By comparison, by arranging the reflection section 36 in the hand insertion section 3 in the portion opposing the hand insertion section 3 in the portion where the UV irradiator 23 is arranged, the hand insertion section 3 in the portion where the UV irradiator 23 is arranged can be irradiated with ultraviolet light reflected by the reflection section 36. For example, when the UV irradiator 23 is arranged in the insertion front section 3b and the reflection section 36 is arranged in the insertion rear section 3c, due to the insertion front section 3b being irradiated with ultraviolet light reflected by the reflection section 36, illuminance of the insertion front section 3b can be increased. Diffuse reflection has a characteristic of performing scattered reflection in various directions. Therefore, when using the reflection section 36 that performs diffuse reflection, for example, illuminance of the entire hand insertion section 3 can be increased. As described above, providing the reflection section 36 enables germicidal power to be further increased. While an example of a method of use of specular reflection and diffuse reflection has been described, methods of use are not limited thereto.

For example, the reflection section 36 is desirably made of a metal or a fluorine resin as a material with high reflectance. Examples of metal include aluminum. Examples of a fluorine resin include polytetrafluoroethylene (PTFE). In addition, as processing for increasing reflectance, the reflection section 36 may be subjected to processing such as anodizing, coating surface treatment, painting, film application, and evaporation coating, for example, or may be subjected to a plurality of types of processing. Furthermore, when the reflection section 36 is to be subjected to processing for increasing reflectance as described above, a material or a base material of the reflection section 36 may be a material with low reflectance.

For example, the reflection section 36 may be a plate-like member. The reflection section 36 may include a reflection surface 36a, a rear surface 36b, and a reflection side wall section 36c. For example, the reflection surface 36a may have a substantially rectangular shape. For example, the reflection section 36 may have a substantially rectangular parallelepiped shape. In the present disclosure, "substantially rectangular" or "substantially rectangular parallelepiped" includes shapes with chamfered corners and shapes with rounded sides. In addition, in order to fix the reflection section 36 to the hand insertion section 3, the reflection section 36 may be provided with an opening, a slit, a notch, a dimple, or a recess. Note that the shape of the reflection section 36 may be a shape to be arranged over a plurality of faces of the hand insertion section 3.

A thickness of the reflection section 36 is, for example, around 0.2 mm to 5 mm and preferably around 0.5 mm to 3 mm. For example, among three mutually orthogonal axes in the X direction, the Y direction, and the Z direction, the thickness of the reflection section 36 corresponds to a dimension of the reflection section 36 in a direction with a shortest length.

The rear surface 36b of the reflection section 36 is a surface on an opposite side to the reflection surface 36a. The rear surface 36b is arranged at a position closer to the hand insertion section 3 than the reflection surface 36a. The rear surface 36b is in direct or indirect contact with the hand insertion section 3. Indirect contact means, for example, contact via an adhesive member such as a two-sided adhesive tape or an adhesive. A major portion of the rear surface 36b is a flat surface. The rear surface 36b is a surface that is substantially parallel to the reflection surface 36a.

The reflection side wall section 36c is a side wall of the reflection section 36. The reflection side wall section 36c is made up of a plurality of side walls. For example, in the case of a rectangular parallelepiped reflection section 36, four surfaces excluding the reflection surface 36a and the rear surface 36b constitute the reflection side wall section 36c. The reflection side wall section 36c is side walls with respect to the reflection surface 36a and the rear surface 36b. The reflection side wall section 36c may be formed perpendicular or inclined with respect to the reflection surface 36a and the rear surface 36b.

The reflection section 36 may be provided in a region that occupies an area that is half or more of surfaces that face the hand insertion space 5 among the insertion peripheral section of the hand insertion section 3. A shape of the reflection section 36 is not limited to a flat shape. For example, the reflection section 36 may have a shape that is curved along at least a part of the hand insertion section 3. For example, the reflection section 36 may be used pasted along a shape of at least a part of the hand insertion section 3. In this case, for example, the reflection section 36 is formed like a sheet or a film. With the reflection section 36 described above, the reflection section 36 with a complex shape that conforms to the shape of the hand insertion section 3 can be readily formed. Accordingly, the reflection section 36 can be made into any shape.

Examples of methods of fixing the reflection section 36 to the hand insertion section 3 include adhesion using an adhesive tape or an adhesive, fastening using a screw, inlaying, fixed fitting, fitting, and the like.

In addition, instead of mounting the reflection section 36 to the hand insertion section 3, the hand insertion section 3 itself may be made of a material with high reflectance. In other words, the reflection section 36 may be integrated with the hand insertion section 3. In other words, a rear surface-side which does not face the hand insertion space 5 of the hand insertion section 3 may also be formed of a material with high reflectance. For example, the hand insertion section 3 may be made of a metal or a fluorine resin which is a material with high reflectance. When the reflection section 36 is integrated with the hand insertion section 3, there is an advantage that the reflection section 36 does not separate or become detached from the hand insertion section 3 even when subjected to external force during use or affected by age-related degradation.

Compared to a position of a surface of the hand insertion section 3 in the periphery of the reflection section 36, the reflection surface 36a of the reflection section 36 may be arranged at a protruding position, at a position on a same plane, or at a recessed position. The reflection surface 36a of the reflection section 36 is preferably arranged at a position on a same plane as the surface of the hand insertion section 3 in the periphery of the reflection section 36 and more preferably arranged at a recessed position with respect to the surface of the hand insertion section 3 in the periphery of the reflection section 36. When the reflection section 36 or the reflection surface 36a is arranged at a position that is protruded from the surface of the hand insertion section 3 in the periphery thereof, a hand may come into contact with the reflection section 36 when the user inserts the hand into the hand insertion space 5. In this case, there is a possibility that the user may get injured, the reflection surface 36a may become stained or scratched, or the reflection section 36 may become susceptible to separation from the hand insertion section 3. Staining of the reflection surface 36a may potentially result in lower reflectance. By comparison, by arranging the reflection surface 36a of the reflection section 36 at a position on a same plane as the surface of the hand insertion section 3 in the periphery of the reflection section 36 or a recessed position with respect to the surface of the hand insertion section 3 in the periphery of the reflection section 36, since a hand is less likely to come into contact with the reflection section 36, safety, reflectance, and sterilization performance are improved. In addition, a fixed state of the reflection section 36 to the hand insertion section 3 can be more reliably maintained. For example, the reflection section 36 becomes more resistant to separation.

When the reflection section 36 is arranged in the insertion bottom section 3a, there is a possibility that a light beam reflected by the reflection section 36 advances upward, exits the hand insertion section 3, and irradiates the face of a person or the like. In order to prevent such a situation, desirably, the reflection section 36 is not arranged in the insertion bottom section 3a and the reflection section 36 is arranged in the insertion peripheral section. The reflection section 36 is desirably arranged on a surface of the hand insertion section 3 of a portion that opposes the hand insertion section 3 of a portion in which the UV irradiator 23 is arranged. Accordingly, light beams emitted from the UV irradiator 23 can be more efficiently reflected by the reflection section 36. While a height of the position of the reflection section 36 is not limited, the reflection section 36 is desirably arranged at a position lower than the UV irradiator 23. Accordingly, a portion on a lower side among the hand insertion section 3 can be irradiated with a larger amount of ultraviolet light. Due to water droplets dripping from above, many water droplets are present in the lower side among the hand insertion section 3. When the blowout port section faces obliquely downward, since air is also blown obliquely downward, a larger amount of water droplets are likely to adhere to the lower side among the hand insertion section 3. In a location with a large mass of water droplets, due to a high-speed airflow, the water droplets become an aerosol and are more readily scattered into air. In consideration of the above, by arranging the reflection section 36 at a position lower than the UV irradiator 23, a lower side among the hand insertion section 3 can be irradiated with a larger amount of ultraviolet light and hygiene is further improved.

When the reflection section 36 is arranged in the hand insertion section 3, the hand insertion section 3 may have an arrangement section 3n for arranging the reflection section 36. The arrangement section 3n may be formed in the hand insertion section 3 of a portion where the reflection section 36 is arranged. For example, the arrangement section 3n is a part of the hand insertion section 3 where the reflection section 36 is arranged. For example, the arrangement section 3n is integrally molded with the hand insertion section 3 where the reflection section 36 is arranged. For example, the arrangement section 3n is a same member as the hand insertion section 3 where the reflection section 36 is arranged.

For example, the arrangement section 3n includes an arrangement surface 3p and an arrangement side wall section 3q. For example, the arrangement surface 3p is a flat surface. For example, the arrangement surface 3p is in proximity to the rear surface 36b of the reflection section 36. For example, the arrangement surface 3p is in direct or indirect contact with the rear surface 36b. For example, the arrangement surface 3p is a surface parallel to the rear surface 36b. When the arrangement surface 3p is a flat surface, the reflection section 36 may be more easily fixed. The arrangement surface 3p is arranged at a recessed position in the hand insertion section 3 where the reflection section 36 is arranged.

The arrangement side wall section 3q constitutes a side wall of the arrangement section 3n. When the reflection section 36 having a substantially rectangular parallelepiped shape is arranged in the arrangement section 3n, for example, the arrangement side wall section 3q is arranged as four surfaces with the exception of the reflection surface 36a. The arrangement side wall section 3q has surfaces that oppose each other. The arrangement side wall section 3q may be formed perpendicular, inclined, or slightly inclined with respect to the arrangement surface 3p. A direction in which the arrangement side wall section 3q is inclined is a direction in which, the farther from the arrangement surface 3p, the greater a distance between arrangement side wall sections 3q that oppose each other. "Slightly inclined" means, for example, an angle of the arrangement side wall section 3q with respect to the arrangement surface 3p being around 90.2 degrees to 100 degrees and more preferably around 90.5 degrees to 95 degrees, for example. Such an angle is easily molded using a die, and even when the reflection side wall section 36c is a surface perpendicular to the reflection surface 36a and the rear surface 36b, a gap is less likely to be created between the arrangement side wall section 3q and the reflection side wall section 36c. Since a gap is less likely to be created, penetration by dust or water is less likely to occur and the arrangement section 3n can be kept clean, and a fixed state of the reflection section 36 with respect to the arrangement section 3n can be more reliably maintained. In addition, by making the arrangement side wall section 3q parallel to the reflection side wall section 36c, penetration by dust or water is less likely to occur and the arrangement section 3n can be kept clean, and a fixed state of the reflection section 36 with respect to the arrangement section 3n can be more reliably maintained.

The arrangement side wall section 3q is arranged at a position closer to the surface of the hand insertion section 3 in a periphery where the reflection section 36 is arranged than the arrangement surface 3p. When the reflection section 36 is arranged in the arrangement section 3n, the arrangement side wall section 3q comes close to or comes into contact with the reflection side wall section 36c. Each surface of the arrangement side wall section 3q may be arranged substantially parallel to each surface of the reflection side wall section 36c that is in proximity to or in contact with the surface of the arrangement side wall section 3q.

The thickness of the reflection section 36 in the reflection side wall section 36c such as the thickness in a direction perpendicular to the reflection surface 36a may be shorter than a length or, in other words, a depth of the arrangement side wall section 3q in the same direction. When an adhesive member is provided between the rear surface 36b of the reflection section 36 and the arrangement surface 3p, a sum of the thickness of the adhesive member and the thickness of the reflection section 36 may be shorter than the aforementioned depth of the arrangement side wall section 3q. In addition, an end section of the arrangement side wall section 3q at a position separated from the arrangement surface 3p may be arranged at a position closer to the hand insertion space 5 than the reflection surface 36a. In this manner, the reflection surface 36a may be arranged at a position that is recessed than the surface of the hand insertion section 3 in a vicinity of where the reflection section 36 is arranged. Accordingly, since the possibility that a hand comes into contact with the reflection section 36 decreases, safety, reflectance, and sterilization performance are improved. In addition, a fixed state of the reflection section 36 to the hand insertion section 3 can be more reliably maintained. For example, the reflection section 36 becomes more resistant to separation.

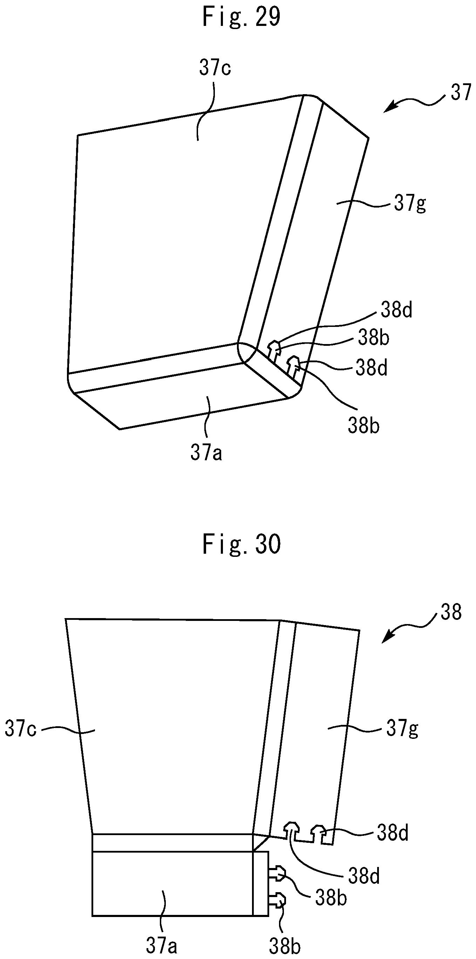

As another aspect of the reflection section 36 described above, the hand dryer 1 may include an assembly 37 formed by three-dimensionally assembling a sheet. Fig. 29 is a perspective view showing an example of the assembly 37 corresponding to another aspect of the reflection section 36. Fig. 30 is a development view showing a sheet prior to three-dimensionally assembling the assembly 37 shown in Fig. 29.

The assembly 37 can be installed in the hand insertion section 3. The assembly 37 is an example representing another aspect of the reflection section 36. In other words, the assembly 37 is a member with high reflectance and is mainly installed in order to increase reflectance. While the assembly 37 may be a resin member, the assembly 37 is desirably constituted of a member made of metal such as aluminum. In addition, the assembly 37 may be subjected to surface treatment such as that described above in order to increase reflectance. The assembly 37 can be mounted to a ready-made article. For example, the assembly 37 may be installable to the hand insertion section 3 of the hand dryer 1 already mounted in a toilet space. The assembly 37 may be mounted to the hand insertion section 3 using a fixing member such as a two-sided adhesive tape, an adhesive, or a screw or mounted to the hand insertion section 3 without using a fixing member.

The assembly 37 is suitable when providing the reflection section 36 on a plurality of faces among the surface of the hand insertion section 3 facing the hand insertion space 5. For example, the assembly 37 is suitable when providing the reflection section 36 on two or more faces among the front-side face, the rear-side face, the left-side face, the right-side face, and the bottom face of the hand insertion section 3.

For example, the assembly 37 is made of a single component. In other words, the assembly 37 has folds or bends in order to be arranged on a plurality of faces. In addition, the assembly 37 is subjected to bending work such as folding or bending.

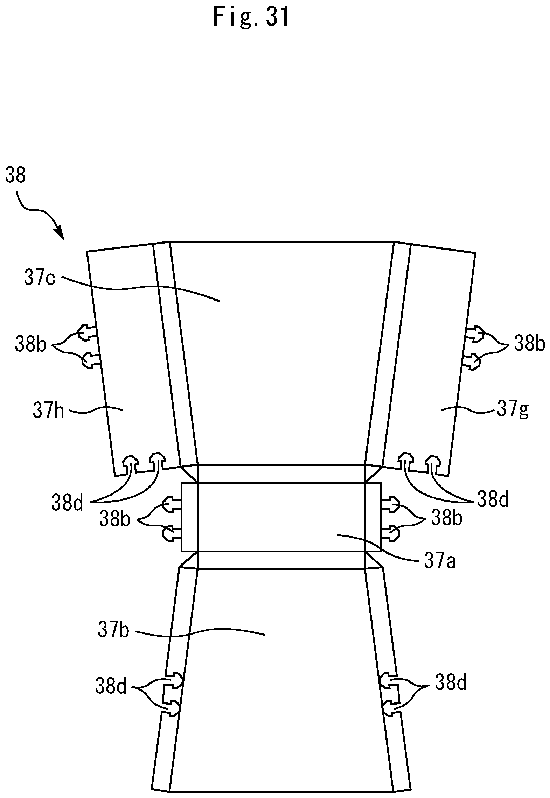

As shown in Fig. 30, the assembly 37 prior to being subjected to the bending work is a plate-like member. In the following description, a plate-like state of the assembly 37 prior to being subjected to bending work will be referred to as an assembly sheet 38. Fig. 31 is a development view showing another example of the assembly sheet 38. The assembly 37 and the assembly sheet 38 may include an assembly bottom section 37a and an assembly peripheral section. For example, when the assembly 37 is installed in the hand insertion section 3, the assembly bottom section 37a is positioned in the insertion bottom section 3a, the assembly bottom section 37a faces the insertion bottom section 3a, or the assembly bottom section 37a is in contact with the insertion bottom section 3a. The assembly peripheral section is positioned in the insertion peripheral section of the hand insertion section 3, the assembly peripheral section faces the insertion peripheral section, or the assembly peripheral section is in contact with the insertion peripheral section. In the following description, "positioned in", "faces", and "in contact with" will be collectively described as "positioned in".

The assembly peripheral section includes at least one of an assembly front section 37b being a portion positioned in the insertion front section 3b, an assembly rear section 37c being a portion positioned in the insertion rear section 3c, and an assembly side section being a portion positioned in the insertion side section 3d. The assembly front section 37b and the assembly rear section 37c are arranged at positions that oppose each other. The assembly side section includes at least one of an assembly left section 37g being a portion positioned in the insertion left section 3g and an assembly right section 37h being a portion positioned in the insertion right section 3h. The assembly left section 37g and the assembly right section 37h are arranged at positions that oppose each other.

In the assembly 37, the assembly front section 37b and the assembly left section 37g are adjacent to each other. In the assembly 37, the assembly front section 37b and the assembly left section 37g are arranged in orientations that are perpendicular or nearly perpendicular to each other. In the assembly 37, the assembly front section 37b and the assembly right section 37h are adjacent to each other. In the assembly 37, the assembly front section 37b and the assembly right section 37h are arranged in orientations that are perpendicular or nearly perpendicular to each other. In the assembly 37, the assembly rear section 37c and the assembly left section 37g are adjacent to each other. In the assembly 37, the assembly rear section 37c and the assembly left section 37g are arranged in orientations that are perpendicular or nearly perpendicular to each other. In the assembly 37, the assembly rear section 37c and the assembly right section 37h are adjacent to each other. In the assembly 37, the assembly rear section 37c and the assembly right section 37h are arranged in orientations that are perpendicular or nearly perpendicular to each other.

When the assembly 37 is installed in the hand insertion section 3, the assembly bottom section 37a is arranged in a horizontal or nearly horizontal orientation. When the assembly 37 is installed in the hand insertion section 3, the assembly peripheral section is arranged in an orientation that is parallel to a vertical line or nearly parallel to a vertical line.

A major part of each portion forming the assembly 37 is a flat plane. Each portion forming the assembly 37 has a bent section between the portion and an adjacent portion. The bent section may include a section that is folded perpendicularly. In addition, the bent section may be a portion shared by a plurality of adjacent portions among the assembly 37. For example, the bent section between the assembly front section 37b and the assembly right section 37h may be a part of the assembly front section 37b, a part of the assembly right section 37h, or made up of a part of the assembly front section 37b and a part of the assembly right section 37h.

The assembly 37 and the assembly sheet 38 include at least one of the plurality of portions of the assembly peripheral section. In other words, the assembly 37 and the assembly sheet 38 include at least one of the assembly front section 37b, the assembly rear section 37c, the assembly left section 37g, and the assembly right section 37h.

The assembly 37 is appropriately arranged so that primary functions of the hand insertion section 3 are not impaired. For example, the assembly 37 is arranged or molded so as not to cover the blowout port section, various sensors arranged in the hand insertion section 3, and the drain outlet 8. For example, in a state where the assembly 37 is installed in the hand insertion section 3, an upper part of the assembly 37 may be arranged at a position lower than the blowout port section and the various sensors. In addition, in a state where the assembly 37 is arranged in the hand insertion section 3, an upper part of the assembly 37 may be arranged higher than the blowout port section and the various sensors and the assembly 37 may include an opening or a notch so that the assembly 37 does not cover the blowout port section and the various sensors. In other words, in a state of being installed in the hand insertion section 3, the assembly 37 is arranged or molded so that the blowout port section and the various sensors are visually recognizable.

The assembly 37 shown in Fig. 29 and the assembly sheet 38 shown in Fig. 30 include the assembly bottom section 37a, the assembly rear section 37c, and the assembly left section 37g. As a different example, for example, the assembly 37 may at least include two portions that are adjacent to each other among the plurality of portions of the assembly peripheral section without including the assembly bottom section 37a.

The assembly sheet 38 shown in Fig. 31 includes the assembly bottom section 37a, the assembly front section 37b, the assembly rear section 37c, the assembly left section 37g, and the assembly right section 37h. In this manner, for example, the assembly 37 may at least include three portions and the assembly bottom section 37a among the plurality of portions of the assembly peripheral section.

For example, the assembly sheet 38 may be a sheet metal member. The assembly sheet 38 is made of a single component. A bent section is provided between one portion and a portion adjacent to the one portion of the assembly 37 and the assembly sheet 38. The assembly sheet 38 is constituted of a thin plate-like object so that the assembly sheet 38 can be bent.

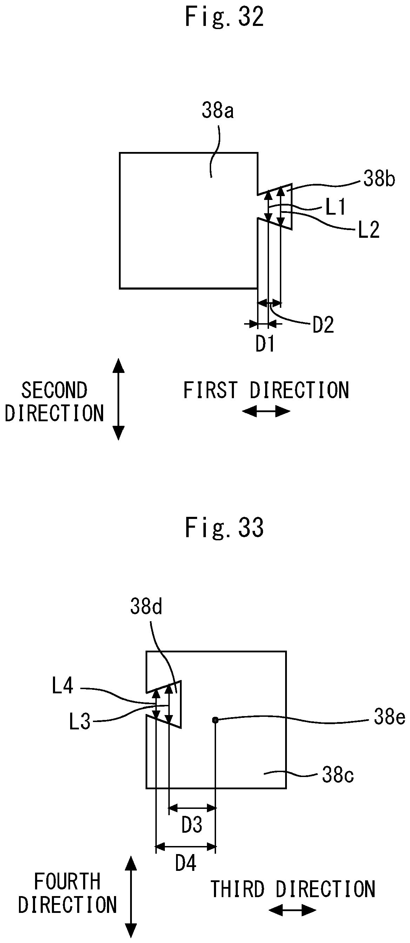

Figs. 32 and 33 are diagrams schematically showing an example of a structure for maintaining a state where the assembly sheet 38 has been assembled as the assembly 37. The assembly sheet 38 includes a protruding main body section 38a shown in Fig. 32 and a recessed main body section 38c shown in Fig. 33. For example, each of the protruding main body section 38a and the recessed main body section 38c may correspond to any of the assembly bottom section 37a, the assembly front section 37b, the assembly rear section 37c, the assembly left section 37g, and the assembly right section 37h.

As shown in Fig. 32, a convex shape section 38b is formed so as to protrude from a part of the protruding main body section 38a. There may be one or a plurality of the convex shape sections 38b.

As shown in Fig. 33, a concave shape section 38d is formed in the recessed main body section 38c. The concave shape section 38d corresponds to a notch where a part of the recessed main body section 38c is shaped so as to be recessed in a direction perpendicular to a thickness direction of the assembly sheet 38. Preferably, the number of the concave shape sections 38d is the same as the number of the convex shape sections 38b.

For example, the thickness of the assembly sheet 38 may be uniform over the entire assembly sheet 38. Thickness directions of the protruding main body section 38a, the convex shape section 38b, the recessed main body section 38c, and the concave shape section 38d are the same direction as the thickness direction of the assembly sheet 38.

The assembly sheet 38 becomes a three-dimensional shape by being bent at bent sections. At this point, the convex shape section 38b and the concave shape section 38d are positioned on the assembly sheet 38 so that the convex shape section 38b and the concave shape section 38d are arranged at positions which mesh together. A shape of the convex shape section 38b is a hook-type shape, a barbed shape, a retention-preventing shape, or the like. The concave shape section 38d is shaped so as to mesh together with the convex shape section 38b. The convex shape section 38b and the concave shape section 38d fit each other like a jigsaw puzzle.

In the following description, as shown in Fig. 32, a direction perpendicular to the thickness direction of the protruding main body section 38a and the convex shape section 38b will be referred to as a first direction and a direction perpendicular to both the thickness direction and the first direction will be referred to as a second direction. The convex shape section 38b protrudes from the protruding main body section 38a in the first direction. At a first position where a distance in the first direction from the protruding main body section 38a is a relatively short first distance D1, a length of the convex shape section 38b in the second direction is L1. At a second position where a distance in the first direction from the protruding main body section 38a is a second distance D2 that is longer than the first distance D 1, a length of the convex shape section 38b in the second direction is L2 that is longer than L1. In other words, the convex shape section 38b has a shape in which a thickness L2 at the second position close to a tip thereof is larger than a thickness L1 at the first position close to a base.

For example, the concave shape section 38d is recessed from a specific side of the recessed main body section 38c. In the following description, as shown in Fig. 33, a direction from a position 38e corresponding to a center or a center of gravity of the recessed main body section 38c toward a specific side on which the concave shape section 38d is formed will be referred to as a third direction and a direction perpendicular to both the thickness direction of the recessed main body section 38c and the concave shape section 38d and the third direction will be referred to as a fourth direction. At a third position where a distance in the third direction from the position 38e is a relatively short third distance D3, a length of the concave shape section 38d in the fourth direction is L3. At a fourth position where a distance in the third direction from the position 38e is a fourth distance D4 that is longer than the third distance D3, a length of the concave shape section 38d in the fourth direction is L4 that is shorter than L3. In other words, a width L4 of the concave shape section 38d in the fourth direction at the fourth position that is close to an opening of the concave shape section 38d is smaller than a width L3 of the concave shape section 38d in the fourth direction at the third position that is a deeper position of the concave shape section 38d than the fourth position.

The assembly sheet 38 shown in Figs. 30 and 31 is also provided with the convex shape section 38b and the concave shape section 38d. A function as a retention-preventing structure is achieved as the convex shape section 38b and the concave shape section 38d mesh together. As a result, as shown in Fig. 29, the three-dimensional shape of the assembly 37 can be maintained more stably.

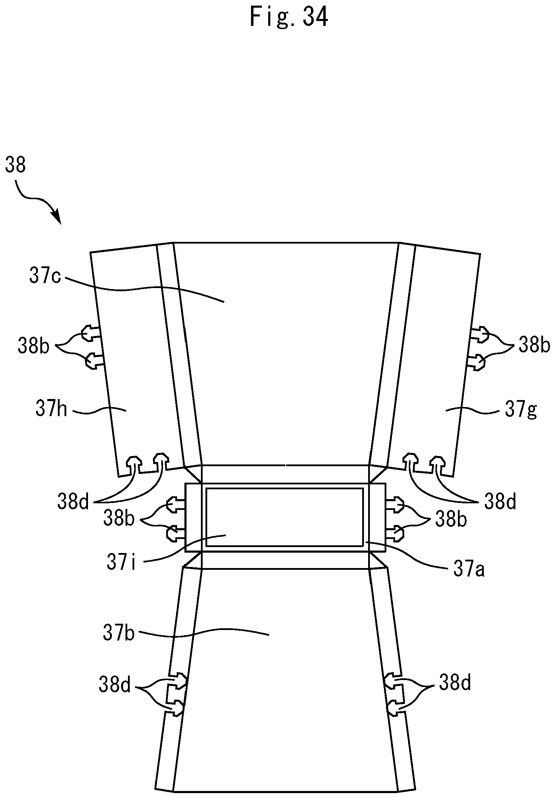

Fig. 34 is a development view showing a modification of the assembly sheet 38 shown in Fig. 31. In the modification shown in Fig. 34, a substantially rectangular opening 37i is provided in the assembly bottom section 37a. An area of the opening 37i is larger than an area of the assembly bottom section 37a of a portion excluding the opening 37i. Providing the opening 37i as described above causes most of the surface of the insertion bottom section 3a to be exposed to the hand insertion space 5 without being covered by a material with high ultraviolet reflectance. As a result, since upward reflection of ultraviolet light emitted toward the insertion bottom section 3a can be suppressed, ultraviolet light that leaks to the outside of the hand insertion space 5 can be more reliably reduced.

The assembly 37 desirably has outer dimensions matching inner dimensions of the hand insertion section 3 so that, when the assembly 37 is arranged in the hand insertion section 3, no gaps are created with respect to the hand insertion section 3. Note that the assembly 37 may be arranged in the hand insertion section 3 with a similar structure to that shown in Fig. 28. For example, when the assembly 37 is arranged in the hand insertion section 3, a rear surface of the face exposed to the hand insertion space 5 among the assembly 37 comes into contact with the arrangement surface 3p. For example, at least one rear surface of the assembly front section 37b, the assembly rear section 37c, the assembly left section 37g, and the assembly right section 37h comes into contact with the arrangement surface 3p. At this point, a reflection side wall section of a portion in contact with the arrangement surface 3p at least one of the rear surface of the assembly front section 37b, the assembly rear section 37c, the assembly left section 37g, and the assembly right section 37h may be in proximity to or may come into contact with the arrangement side wall section 3q. In other words, the arrangement side wall section 3q may be positioned directly above the assembly 37. In other words, the surface of the insertion peripheral section of the hand insertion section 3 that corresponds to a position where the assembly 37 is installed may be arranged at a position recessed by one step with respect to the surface of the hand insertion section 3 at a position adjacent to the assembly 37. Accordingly, when the assembly 37 is arranged, the assembly 37 is arranged at a position on a same plane as the surface of the hand insertion section 3 in the periphery of the assembly 37 or arranged at a recessed position with respect to the surface of the hand insertion section 3 in the periphery of the assembly 37. Accordingly, since the possibility that a hand comes into contact with the surface of the assembly 37 or the edge of the assembly 37 decreases, safety, reflectance, and sterilization performance are improved. In addition, even if adhesion between the assembly 37 and the hand insertion section 3 weakens, the assembly 37 does not move upward. In addition, the assembly 37 can also be directly arranged in the hand insertion section 3 without adhesion. Even in this case, the assembly 37 can be used without being detached from the hand insertion section 3. Furthermore, a restoring force due to elasticity to a state of an assembly sheet or, in other words, a state of a planar shape may act on the assembly 37. Even in this case, due to the presence of the arrangement side wall section 3q, a position with respect to the hand insertion section 3 does not shift.

The surface facing the hand insertion space 5 may include a first region having a reflectance of 70% or higher with respect to light of a dominant wavelength generated by the light source 24 and a second region having a reflectance of 30% or lower with respect to light of a dominant wavelength generated by the light source 24. Accordingly, by suppressing reflection of ultraviolet light by the second region while enabling emission of ultraviolet light over a wide range by irradiating other regions with ultraviolet light reflected by the first region, ultraviolet light that leaks to the outside of the hand insertion space 5 can be more reliably reduced. For example, the first region may be provided in at least one of the insertion front section 3b, the insertion rear section 3c, the insertion left section 3g, and the insertion right section 3h and the second region may be provided in the insertion bottom section 3a. The first region may be formed by providing the reflection section 36, and a region not covered by the reflection section 36 may be adopted as the second region.

The hand dryer 1 may further include a portion subjected to a photocatalyst treatment. A photocatalyst creates a catalytic action when irradiated with light. For example, when a catalytic action is created, bacteria or viruses are decomposed and made harmless. For example, a photocatalyst is made of titanium oxide. For example, a light beam emitted from the UV irradiator 23 irradiates at least a part of the hand dryer 1 either directly or after being reflected. Such a surface irradiated with a UV light beam will be referred to as an irradiated surface. For example, a photocatalyst treatment may be applied to at least a part of an irradiated surface. The photocatalyst treatment may be applied to a surface of the irradiated surface. Alternatively, kneading of a photocatalyst material and a resin material may be performed in a step preceding molding and an irradiated surface may be formed using a component created by molding the kneaded material. For example, when a material of the irradiated surface is a resin material, a photocatalyst material may be kneaded into the material. In the case of a material into which a photocatalyst material is kneaded, a photocatalyst effect lasts longer even when surface abrasion occurs due to scrubbing, wiping, or the like. When irradiated with a UV light beam, an irradiated surface subjected to a photocatalyst treatment decomposes bacteria or viruses due to a catalytic reaction in addition to a sterilization effect due to UV heretofore described. Accordingly, a rate at which bacteria or viruses are sterilized and made harmless is further accelerated. Therefore, a risk of infection further declines.

In addition, the UV irradiator 23 may sterilize air or sterilize the inside of the hand dryer 1. For example, the UV irradiator 23 may sterilize an air path inside the hand dryer 1 or perform sterilization by emitting UV towards a liquid inside the hand dryer 1. Even in such cases, hygiene can be improved.

### Second embodiment

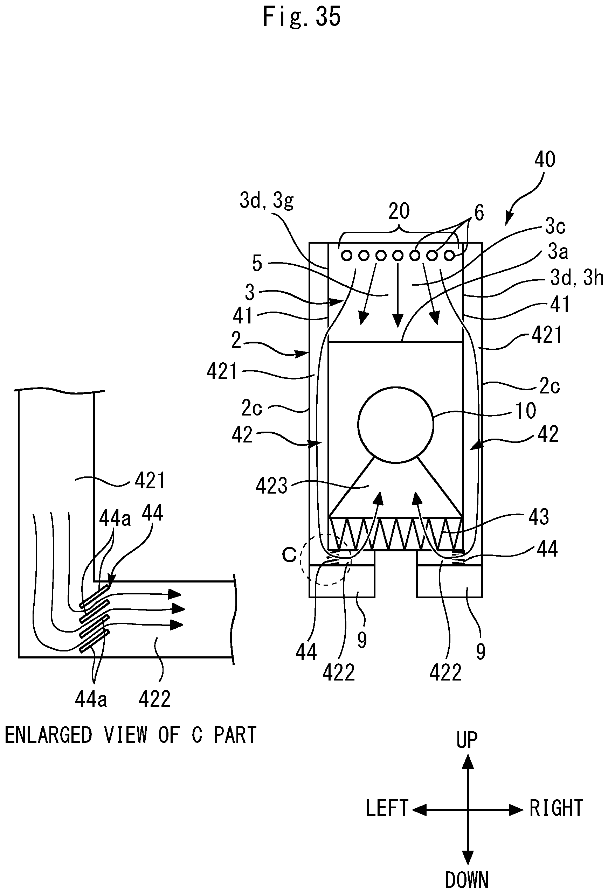

Next, while a second embodiment will be described with reference to Fig. 35, the description will focus on differences from the first embodiment described above and common descriptions will be simplified or omitted. In addition, elements in common with or corresponding to elements described earlier will be denoted by same reference signs.

Fig. 35 is a schematic sectional view of a hand dryer 40 according to the second embodiment. The sectional view corresponds to a sectional front view obtained by cutting the hand dryer 40 along a plane perpendicular to a front-rear direction of the hand dryer 40.

In the first embodiment, an example has been described in which an airflow is sucked by the blower 10 from the air inlet 16 arranged in a bottom section of the hand dryer 1, and a hand is dried by causing an airflow blown into the hand insertion space 5 from a blowout port section to strike the hand. In the first embodiment described above, there is a possibility that fine water droplets may scatter out from a side of or above the hand insertion section 3. With the hand dryer 40 according to the second embodiment, scatter of water droplets can be reduced as compared to the first embodiment.

Points of particular importance will be described below. The following points are similar to the first embodiment. The blowout port section including the air hole 6 is arranged in the hand insertion section 3. For example, the blowout port section is arranged in the insertion peripheral section. For example, the blowout port section is arranged in the insertion front section 3b or the insertion rear section 3c. The drain outlet 8 is formed in the insertion bottom section 3a. For example, an upper part of the insertion side section 3d may be arranged at a height equivalent to an upper part of the insertion front section 3b or arranged at a height equivalent to an upper part of the insertion rear section 3c. When the blower 10 is driven, air is blown out from the air hole 6 in the blowout port section.

Hereinafter, a description will be given with a focus on differences from the first embodiment. The hand dryer 40 has a suction port 41 that opens to the hand insertion space 5. For example, the suction port 41 is arranged in the hand insertion section 3. The suction port 41 may be arranged in a portion of the hand insertion section 3 adjacent to the blowout port section. For example, as shown in Fig. 35, when the blowout port section is arranged in the insertion front section 3b or the insertion rear section 3c, the suction port 41 may be arranged in the insertion side section 3d. Alternatively, the suction port 41 may be arranged in the insertion bottom section 3a. The suction port 41 is arranged at a position that is exposed to the hand insertion space 5. The suction port 41 in the illustrated example is arranged at a position lower than the blowout port section. However, the suction port 41 may be arranged at a position higher than the blowout port section. The hand dryer 40 may include only one suction port 41 or include a plurality of suction ports 41. For example, as in the illustrated example, the hand dryer 40 may include the suction port 41 arranged in the insertion left section 3g and the suction port 41 arranged in the insertion right section 3h. One suction port 41 may be formed by a collection of a plurality of small holes. Accordingly, since suction air speed can be increased while reducing the possibility that the user inserts a finger or the like into the suction port 41 by mistake, safety is improved.

The hand dryer 40 includes a suction air path 42. The suction air path 42 is in fluid communication with the suction port 41. In other words, air sucked in from the suction port 41 passes through the suction air path 42. The suction air path 42 may be provided in plurality. For example, the suction air path 42 in fluid communication with the suction port 41 arranged in the insertion left section 3g and the suction air path 42 in fluid communication with the suction port 41 arranged in the insertion right section 3h may be provided.

At least a part of the suction air path 42 may be arranged between a cover section of the main body enclosure 2 and the hand insertion section 3. At least a part of the suction air path 42 may be arranged in a region formed by the cover section and the hand insertion section 3. For example, the suction air path 42 includes a portion arranged in a region formed by the side cover 2c and the insertion side section 3d. For example, the suction air path 42 includes a portion arranged in a region formed by the left side cover 2c and the insertion left section 3g. For example, the suction air path 42 includes a portion arranged in a region formed by the right side cover 2c and the insertion right section 3h. In other words, the cover section and the hand insertion section 3 double as members that define the suction air path 42.

The suction air path 42 is arranged at a position of which a height is equivalent to the suction port 41 or at a position lower than the suction port 41. The suction air path 42 is directly or indirectly connected to an inlet side of the blower 10. In other words, when the blower 10 is actuated, an airflow is generated which causes air to be sucked into the suction port 41 from the hand insertion space 5 via the suction air path 42. The suction air path 42 may be in fluid communication with the inlet air path 18 as described in the first embodiment.

According to the present embodiment, when drying a hand, air in the hand insertion space 5 can be sucked into the suction port 41. Accordingly, water droplets can be prevented from scattering outside of the hand insertion space 5. Therefore, scatter of water droplets can be reduced as compared to the first embodiment.

For example, the suction air path 42 may be made up of a suction air path 421, a suction air path 422, and a suction air path 423. One end of the suction air path 421 is in fluid communication with the suction port 41. At least a part of the suction air path 421 is formed along the up-down direction. The suction air path 421 moves air having flowed in from the suction port 41 in a downward direction. The one end of the suction air path 421 is arranged above the blower 10. Another end of the suction air path 421 is arranged below the blower 10.

One end of the suction air path 422 is in fluid communication with the other end of the suction air path 421. At least a part of the suction air path 422 is formed along the left-right direction or the front-rear direction of the hand dryer 40. The suction air path 422 includes an air path which moves air having passed through the suction air path 421 toward a center side in a top view of the hand dryer 40. For example, when viewing the inside of the hand dryer 40 from above, the one end of the suction air path 422 is arranged at a position close to any of a left end, a right end, a front end, and a rear end of the hand dryer 40. In other words, when viewing the inside of the hand dryer 40 from above, the one end of the suction air path 422 is arranged at a position that does not overlap with the blower 10. When viewing the inside of the hand dryer 40 from above, at least a part of a side of another end of the suction air path 422 is arranged at a position to be covered by the blower 10. At least a part of the suction air path 422 may be formed along a horizontal direction or formed along a direction that is oblique with respect to a horizontal plane.

One end of the suction air path 423 is in fluid communication with the other end of the suction air path 422. For example, the suction air path 423 is arranged below the blower 10. At least a part of the suction air path 423 is formed along the up-down direction. The suction air path 423 includes an air path which moves air having passed through the suction air path 422 in an upward direction. The suction air path 423 is arranged at a position closer to the center side than the left end and the right end of the hand dryer 40. Another end of the suction air path 423 is in fluid communication with the inlet side of the blower 10.

The suction air path 422 and the suction air path 423 may have an integrated shape. For example, the suction air path 422 and the suction air path 423 may be air paths which move air having flowed in from the suction port 41 toward the center side of the hand dryer 40 when viewing the inside of the hand dryer 40 from above while moving air from below to above when viewing the inside of the hand dryer 40 from the front.

The drain tank 9 is arranged below the suction air path 421. The drain tank 9 may be arranged below the suction air path 422 and the suction air path 423.

When a hand is arranged in the hand insertion section 3, the blower 10 is actuated and an airflow having been sucked in from the suction port 41 passes through the suction air path 42 and the blower 10 and is blown into the hand insertion space 5 from the blowout port section. The airflow from the blowout port section strikes the hand and blasts away moisture adhering to the hand. Due to the actuation of the blower 10, a suction force is generated in the suction port 41 and the suction port 41 sucks at least a part of air blown out from the blowout port section. For example, at least a part of an airflow having struck the hand from one direction approaches the suction port 41 and is sucked into the suction port 41 by moving in a direction substantially perpendicular to the one direction.

In addition, the suction port 41 sucks water droplets having been blasted away from a hand or the hand insertion section 3 as well as water droplets floating in the hand insertion space 5. The water droplets sucked in from the suction port 41 are separated from air by water droplet removing means to be described later or the like and accumulate in the drain tank 9.

Air sucked in from the suction port 41 includes air blown out from the blowout port section. Air sucked in from the suction port 41 is once again blown out from the blowout port section by the blower 10.

Water droplets having dripped to the insertion bottom section 3a due to gravity and water droplets having flowed down to the insertion bottom section 3a along a wall surface of the insertion peripheral section gather in the drain outlet 8 by an inclined surface formed in the insertion bottom section 3a and are collected in the drain tank 9 from the drain outlet 8. In addition, the suction port 41 sucks water droplets and air floating in the hand insertion space 5. Accordingly, water droplets and air to be blown outside of the hand insertion space 5 are reduced and hygiene is improved.

Water droplets readily accumulate in the insertion bottom section 3a. Water droplets accumulated in the insertion bottom section 3a may scatter. A large amount of water droplets scatter from a side of a fingertip of a hand or, in other words, a lower side due to an airflow blown obliquely downward from the blowout port section. When the suction port 41 is positioned lower than the blowout port section, the scattered water droplets can be immediately sucked into the suction port 41. As a result, hygiene further improves.

On the other hand, when the suction port 41 is positioned higher than the blowout port section, water droplets having scattered from the hand insertion section 3 or water droplets having scattered from a hand are easily sucked into the suction port 41 when the water droplets move to the outside of the hand insertion space 5. As a result, even in this case, hygiene can be improved.

The hand dryer 40 may include at least one water droplet removing means. The water droplet removing means removes water droplets from an airflow that passes through the suction air path 42. For example, the hand dryer 40 includes first water droplet removing means 43. The first water droplet removing means 43 may be arranged in the suction air path 422, arranged in the suction air path 423, or arranged in a boundary section between the suction air path 422 and the suction air path 423.

The first water droplet removing means 43 mainly removes fine water droplets. For example, the first water droplet removing means 43 removes particles of 5 µm or less or aerosols. For example, the first water droplet removing means 43 includes an air filter. Preferably, the first water droplet removing means 43 includes a HEPA filter. The first water droplet removing means 43 removes and captures fine water droplets or fine particles contained in a sucked airflow from air of the sucked airflow. Clean air from which fine water droplets and fine particles have been removed by the first water droplet removing means 43 passes through the blower 10 and is blown out from the blowout port section.

The hand dryer 40 may further include hygiene improving means (not illustrated) for improving hygiene of the first water droplet removing means 43. For example, the hygiene improving means acts on the first water droplet removing means 43. For example, the hygiene improving means is arranged in a vicinity of the first water droplet removing means 43. For example, the hygiene improving means is heating means for heating the first water droplet removing means 43. For example, the heating means is a heater. When a water content of the first water droplet removing means 43 increases due to repetitive use of the hand dryer 40, there is a possibility that miscellaneous bacteria may propagate and hygienic conditions may decline. By comparison, when the first water droplet removing means 43 is heated by the heating means, temperature of the first water droplet removing means 43 rises. As a result, propagation of miscellaneous bacteria is suppressed, the number of miscellaneous bacteria decreases, and hygiene improves. In addition, by causing moisture adhering to the first water droplet removing means 43 to evaporate due to heat from the heating means, an environment that discourages propagation of miscellaneous bacteria is created and hygiene improves. Furthermore, the number of miscellaneous bacteria in moisture evaporated by the heating means can also be reduced by heating and hygiene is improved.

In addition, for example, the hygiene improving means may be UV irradiation means. For example, the UV irradiation means may be similar to the UV irradiator 23 described in the first embodiment. Due to the UV irradiation means irradiating the first water droplet removing means 43 with ultraviolet light, microorganisms adhering to the first water droplet removing means 43 can be inactivated and hygiene is improved. For example, the UV irradiation means may emit ultraviolet light in the UVA wavelength range. While UVA has lower germicidal power than UVB and UVC, UVA is less likely to cause deterioration of resins and has a smaller adverse effect on the human body. Therefore, in the case of UVA, long-term irradiation or constant irradiation can be performed.

When there are a plurality of suction air paths 42 such as when there are the suction air path 42 that connects to the suction port 41 in the insertion left section 3g and the suction air path 42 that connects to the suction port 41 in the insertion right section 3h, a confluence section where the plurality of suction air paths 42 join into one may be provided. The confluence section may be provided on an upstream side of the first water droplet removing means 43. In other words, a configuration may be adopted in which, after airflows from a plurality of suction air paths 42 join each other, water droplets are removed by the first water droplet removing means 43. When the confluence section is arranged on an upstream side of the first water droplet removing means 43, since only one first water droplet removing means 43 is required, a small number of components suffices. Alternatively, the confluence section may be provided on a downstream side of the first water droplet removing means 43. Airflows from the plurality of suction air paths 42 may join each other at the confluence section after water droplets have been removed by the first water droplet removing means 43.

For example, the hand dryer 40 includes second water droplet removing means 44. For example, the second water droplet removing means 44 may be arranged in the suction air path 421, arranged in the suction air path 422, arranged in the suction air path 423, arranged in a boundary section between the suction air path 421 and the suction air path 422, or arranged in a boundary section between the suction air path 422 and the suction air path 423.

The second water droplet removing means 44 is for removing water droplets with a relatively larger particle size as compared to the first water droplet removing means 43. The second water droplet removing means 44 is arranged on an upstream side of the first water droplet removing means 43. In other words, air which is sucked in from the suction port 41 and which moves along the suction air path 42 first passes through the second water droplet removing means 44 and then passes through the first water droplet removing means 43. Subsequently, the air passes through the blower 10 and is blown out from the blowout port section.

For example, the second water droplet removing means 44 is made of an inclined section. For example, the inclined section includes one or a plurality of plate-like members 44a. Each of the inclined sections or, in other words, each of the plate-like members 44a is constructed in a thin-plate shape. For example, the plurality of plate-like members 44a are arranged substantially parallel to each other. For example, the plurality of plate-like members 44a are arranged in substantially regular intervals. The inclined sections due to the plurality of plate-like members 44a have, for example, a louver shape.

The plate-like member 44a is arranged inclined with respect to an axial direction of the suction air path 42 at a position where the plate-like member 44a is arranged or with respect to a direction in which air in the air path flows. For example, when the plate-like member 44a is arranged in the suction air path 422, the plate-like member 44a is arranged inclined with respect to an axial direction of the suction air path 422 or with respect to a direction in which air in the suction air path 422 flows. For example, when the plate-like member 44a is arranged in the suction air path 423, the plate-like member 44a is arranged inclined with respect to an axial direction of the suction air path 423 or with respect to a direction in which air in the suction air path 423 flows.

The plate-like member 44a has a high end part and a low end part. The low end part is arranged at a lower position than the high end part. An inclined surface including the high end part and the low end part or an inclined surface defined by the high end part and the low end part is arranged inclined with respect to a vertical surface. For example, the inclined surface is arranged in an orientation that is closer to the vertical surface than a horizontal surface. For example, an angle formed between the horizontal surface and the inclined surface is around 45 degrees to 80 degrees. Preferably, the angle is around 55 degrees to 75 degrees.

When the second water droplet removing means 44 is arranged in the suction air path 422 or, in other words, when the second water droplet removing means 44 is arranged in the suction air path 42 along the horizontal direction as in the example shown in Fig. 35, the plurality of plate-like members 44a are arranged so as to be lined up in the up-down direction.

The hand dryer 40 has a center in a top view. For example, the center may be a position of a center of gravity. The center of the hand dryer 40 in a top view may be a position halfway between the left end of the hand dryer 40 and the right end of the hand dryer 40 and halfway between the front end of the hand dryer 40 and the rear end of the hand dryer 40. When the second water droplet removing means 44 is arranged in the suction air path 422, the high end part of the plate-like member 44a is arranged at a position closer to the center of the hand dryer 40 in a top view than the low end part of the plate-like member 44a. In this case, the suction air path 422 forms a suction air path toward a center side and in an upward direction.

A downward air path is formed in the suction air path 421, and an air path that at least moves in a horizontal direction is formed in the suction air path 422. In addition, the suction air path 422 is constructed to become an air path that moves upward due to an inclined surface of the inclined section or, in other words, the plate-like member 44a. In other words, a downward airflow of the suction air path 421 abruptly changes orientations to obliquely upward due to the inclined surface of the inclined section or, in other words, the plate-like member 44a. Therefore, when the airflow advances from the suction air path 421 to the suction air path 422, a centrifugal force acts on the airflow. Generally, a centrifugal force is proportional to mass. In other words, a centrifugal force is proportional to a product of volume and density. Since water droplets have a higher density than air, water droplets are more susceptible to the effect of a centrifugal force than air. Therefore, water droplets less readily pass through the suction air path 422 than air. In addition, the inclined surface described above is a surface that inclines obliquely upward facing the suction air path 423. Therefore, when water droplets adhering to the inclined surface gather and a volume thereof increases, the water droplets drip down along the inclined surface due to gravity. As a result, water droplets adhering to the inclined surface less readily move toward a side of the suction air path 423. Therefore, water is less likely to enter the blower 10 and the blower 10 is less likely to fail. In addition, water droplets containing microorganisms are less likely to be scattered from the blowout port section. As a result, hygiene further improves.

When the second water droplet removing means 44 is arranged in the suction air path 423 or, in other words, when the second water droplet removing means 44 is arranged in the suction air path 42 along the up-down direction, the plurality of plate-like members 44a of the inclined section are arranged so as to be lined up in the horizontal direction. Even in this case, water droplets can be more efficiently separated from air by the use of a centrifugal force and the effect described above is obtained.

Note that the hand dryer 40 may suck air from locations other than the suction port 41. For example, at least a part of the first water droplet removing means 43 may be exposed to outside air and the hand dryer 40 may suck air directly via the first water droplet removing means 43 from below the first water droplet removing means 43. Air blown out from the air hole 6 may retain heat. Circulating this air may possibly cause air temperature to rise excessively. By sucking together outside air that is not air blown out from the air hole 6 enables an excessive rise in temperature to be more reliably prevented. Note that the second water droplet removing means 44 may be provided at a plurality of locations. For example, the suction port 41 is provided in the insertion peripheral section. The second water droplet removing means 44 may be provided in a vicinity of the suction port 41. The second water droplet removing means 44 may be provided integrally with the suction port 41. In other words, a configuration may be adopted such that air sucked in from the suction port 41 passes through the second water droplet removing means 44 before moving downward through the suction air path 42. For example, the plate-like member 44a is arranged inclined with respect to a horizontal plane. For example, the plate-like member 44a is arranged so as to be oriented upward from the suction port 41 toward the suction air path 42. For example, the plate-like member 44a is arranged inclined such that air sucked in from the suction port 41 moves upward. As described above, by providing the second water droplet removing means 44 in a vicinity of the suction port 41 or integrally with the suction port 41, a part of water droplets having separated from a hand adheres to the second water droplet removing means 44. Water droplets having adhered to the plate-like member 44a drop to the insertion bottom section 3a to be discharged from the drain outlet 8. In this manner, by arranging the second water droplet removing means 44, water droplets that flow into the suction air path 42 can be suppressed. Accordingly, moisture that adheres to the first water droplet removing means 43 is reduced and hygiene is further improved.

### Third embodiment

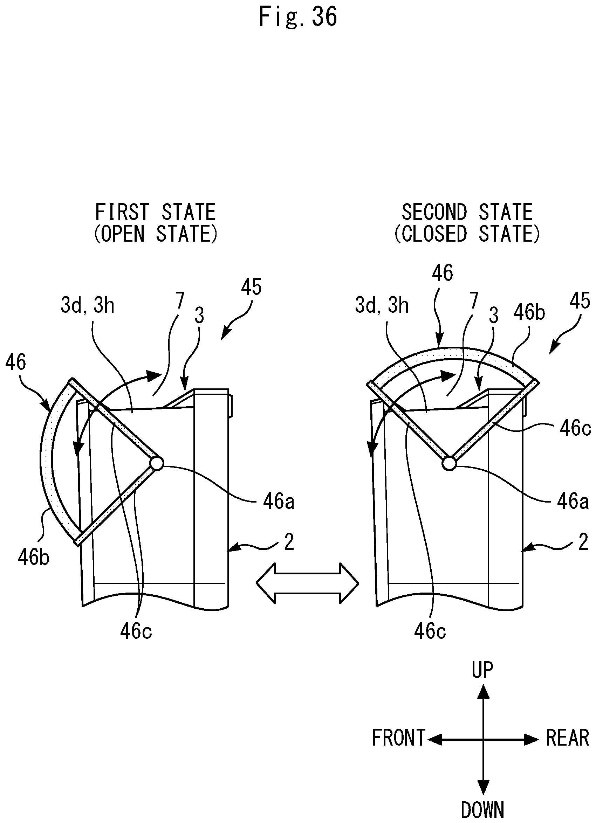

Next, while a third embodiment will be described with reference to Fig. 36, the description will focus on differences from the first embodiment described above and common descriptions will be simplified or omitted. In addition, elements in common with or corresponding to elements described earlier will be denoted by same reference signs.

Fig. 36 is a side view of an upper part of a hand dryer 45 according to the third embodiment. As shown in Fig. 36, the hand dryer 45 further includes a shielding body 46. The hand dryer 45 is configured to be capable of switching between a first state and a second state. The second state is a state where the shielding body 46 covers at least a part of the hand insertion opening 7 to be an inlet of a hand to the hand insertion space 5. The first state is a state where a proportion of the hand insertion opening 7 that is covered by the shielding body 46 is smaller than the second state or becomes zero. When ultraviolet light is emitted from the UV irradiator 23 in the second state, the shielding body 46 blocks ultraviolet light having passed through the hand insertion opening 7 and heading outside the hand insertion space 5. Accordingly, since ultraviolet light can be more reliably prevented from striking human bodies, a high level of safety is attained.

The first state is a state where a hand can be inserted from the hand insertion opening 7 into the hand insertion space 5. The first state is a state where the hand insertion opening 7 is opened. The first state is a state where the hand insertion opening 7 remains opened without the shielding body 46 blocking the hand insertion opening 7. When ultraviolet light is emitted from the UV irradiator 23, the first state is a state where at least a part of emitted light beams may be emitted above the hand insertion section 3.

For example, the second state is a state where inserting a hand into the hand insertion space 5 is not recommended. For example, the second state is a state where insertion of a hand into the hand insertion space 5 is physically difficult or impossible. For example, the second state is a state where the shielding body 46 is blocking the hand insertion opening 7. For example, the second state is a state where an open region among the hand insertion opening 7 is physically narrower than in the first state. Assuming that the light source 24 is turned on with a same output, an ultraviolet irradiation amount above the hand insertion section 3 in the second state is smaller than an ultraviolet irradiation amount above the hand insertion section 3 in the first state.

The hand dryer 1 according to the first embodiment does not include the shielding body 46. Therefore, there is a possibility that a part of light beams emitted from the UV irradiator 23 may be directly emitted above the hand insertion section 3 from the UV irradiator 23 or emitted above the hand insertion section 3 after being reflected by a surface of the hand insertion section 3 facing the hand insertion space 5 or by the reflection section 36. An amount, illuminance, or an irradiation amount of light beams emitted above the hand insertion section 3 as described above will be defined as a "leakage amount". The smaller the leakage amount, the smaller an amount of exposure to radiation for persons in the periphery and a level of safety is further improved.

The shielding body 46 prevents transmission of ultraviolet light and prevents ultraviolet light from being radiated HAoutside of the hand insertion space 5. The shielding body 46 reflects or absorbs a large portion of specific wavelengths of ultraviolet light and more preferably absorbs a large portion of wavelengths. More specifically, the shielding body 46 reflects or absorbs a large portion of wavelengths in an ultraviolet range emitted from the UV irradiator 23. For example, the shielding body 46 is made of a material having a transmittance of 20% or less and preferably a transmittance of 10% or less with respect to light beams of a dominant wavelength of the UV irradiator 23. For example, the shielding body 46 is made of a material which keeps transmittance and reflectance of light beams of a dominant wavelength of the UV irradiator 23 to 20% or less and preferably 10% or less. For example, the shielding body 46 is made of a material which keeps transmittance and absorptance of light beams of a dominant wavelength of the UV irradiator 23 to 20% or less and preferably 10% or less. A large portion of the shielding body 46 is made of a resin material or a metal material.

The shielding body 46 may be arranged in an upper part among the hand insertion section 3 or arranged above the hand insertion section 3. The shielding body 46 is arranged above the UV irradiator 23.

In the present embodiment, due to the shielding body 46 rotating and moving around a rotary shaft 46a, a switch is made between the first state or an open state and the second state or a closed state. The rotary shaft 46a is parallel to the left-right direction of the hand dryer 45. The shielding body 46 includes a wall section 46b and a support section 46c which connects the wall section 46b to the rotary shaft 46a. In the illustrated example, the wall section 46b has a curved surface shape that conforms to a cylindrical surface centered on the rotary shaft 46a. In the second state, the wall section 46b covers at least a part of the hand insertion opening 7 from above. In the first state, the wall section 46b moves to a front side of the main body enclosure 2 and the hand insertion opening 7 is opened.

The user can switch between the first state and the second state by manually moving the shielding body 46.

The hand dryer 45 may include an actuator (not illustrated) for moving the shielding body 46. The control section 22 may automatically switch between the first state and the second state by driving the actuator and moving the shielding body 46 in accordance with a detection by the hand detection section 21 or a detection by the human detection section 39. For example, the control section 22 may make a switch from the second state to the first state in accordance with a detection of a hand by the hand detection section 21 or a detection of a person by the human detection section 39. In addition, the control section 22 may make a switch from the first state to the second state in accordance with a non-detection of a hand by the hand detection section 21 or a non-detection of a person by the human detection section 39.

In the present embodiment, the hand detection section 21 may be arranged above the shielding body 46. Alternatively, in addition to the hand detection section 21 according to the first embodiment, the hand dryer 45 may include hand detection means arranged above the shielding body 46 and may switch between the first state and the second state in accordance with a detection of a hand by the hand detection means.

The hand dryer 45 may further include output change means configured to reduce an output of the light source 24 of the UV irradiator 23 or change the output of the light source 24 to zero when a switch is made from the second state to the first state. In other words, control may be performed by the output change means so that ultraviolet irradiation by the UV irradiator 23 is performed in the second state but ultraviolet irradiation by the UV irradiator 23 is suppressed in the first state. Accordingly, ultraviolet light from the UV irradiator 23 can be more reliably prevented from striking human bodies. For example, the output change means may be achieved by the control section 22.

An example of a relationship among a detection state of the hand detection section 21 or the human detection section 39, an output of the light source 24 of the UV irradiator 23, and a motion of the shielding body 46 will be described below. Immediately after a change is made from a state where the human detection section 39 is detecting an absence of a person to a state where the human detection section 39 is detecting a presence of a person, the output change means reduces an output of the light source 24 of the UV irradiator 23 or changes the output of the light source 24 to zero. Immediately afterwards, the hand dryer 45 is switched from the second state to the first state. In addition, immediately after a time point at which a change is made from a state where the hand detection section 21 is detecting a presence of a hand or the human detection section 39 is detecting a presence of a person to a state where the hand detection section 21 is detecting an absence of a hand or the human detection section 39 is detecting an absence of a person or after a predetermined period of time has elapsed from the time point, the hand dryer 45 is switched from the first state to the second state. Immediately afterwards or after a predetermined period of time has elapsed, the output change means increases the output of the light source 24 of the UV irradiator 23 or changes the light source 24 to a lighted state.

In the hand dryer 45, the shielding body 46 may be provided as a detachable attachment. Providing the shielding body 46 as a detachable attachment enables, for example, the shielding body 46 to be retro-fitted to an existing hand dryer that does not include the shielding body 46. For example, the shielding body 46 may be attachable to and detachable from the hand insertion section 3 or attachable to and detachable from the main body enclosure 2.

### Fourth embodiment

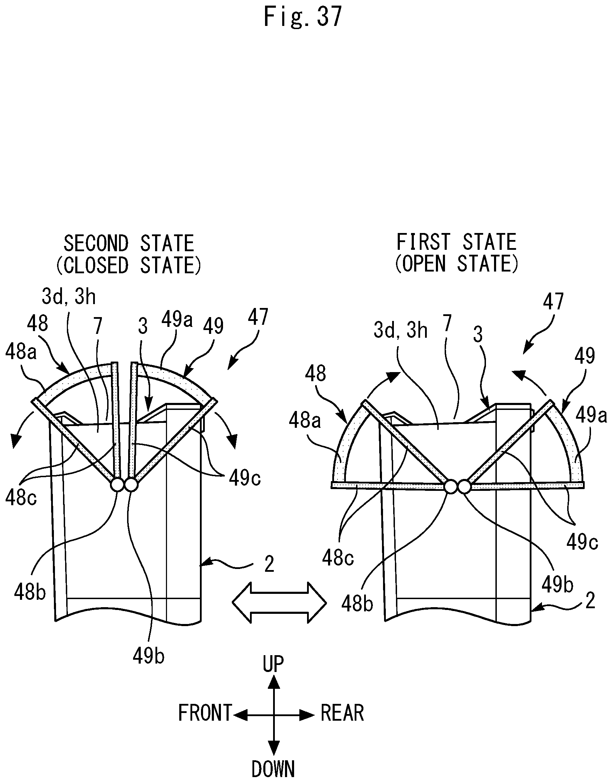

Next, while a fourth embodiment will be described with reference to Fig. 37, the description will focus on differences from the third embodiment described above and common descriptions will be simplified or omitted. In addition, elements in common with or corresponding to elements described earlier will be denoted by same reference signs. The fourth embodiment differs from the third embodiment in a configuration of a shielding body.

Fig. 37 is a side view of an upper part of a hand dryer 47 according to the fourth embodiment. As shown in Fig. 37, the hand dryer 47 according to the fourth embodiment includes a first shielding body 48 and a second shielding body 49 in place of the shielding body 46 according to the third embodiment. As in this example, one hand dryer 47 according to the present disclosure may include a plurality of shielding bodies. The first shielding body 48 includes a wall section 48a, a gear section 48b, and a support section 48c which connects the wall section 48a to the gear section 48b. The first shielding body 48 rotates and moves around the gear section 48b. In the illustrated example, the wall section 48a has a curved surface shape that conforms to a cylindrical surface centered on the gear section 48b.

The second shielding body 49 includes a wall section 49a, a gear section 49b, and a support section 49c which connects the wall section 49a to the gear section 49b. The second shielding body 49 rotates and moves around the gear section 49b. In the illustrated example, the wall section 49a has a curved surface shape that conforms to a cylindrical surface centered on the gear section 49b.

Respective rotary shafts of the gear section 48b and the gear section 49b are parallel to the left-right direction of the hand dryer 47. The gear section 48b meshes together with the gear section 49b. Therefore, the gear section 48b rotates in an opposite direction to the gear section 49b. Accordingly, the first shielding body 48 and the second shielding body 49 rotate in mutually opposite directions.

In the second state or the closed state, the wall section 48a and the wall section 49a cover at least a part of the hand insertion opening 7 from above. When the wall section 48a and the wall section 49a rotate from the second state so as to separate from each other, a switch is made to the first state or an open state. In the first state, as the wall section 48a moves to a front side of the main body enclosure 2 and the wall section 49a moves to a rear side of the main body enclosure 2, the hand insertion opening 7 is opened. When the wall section 48a and the wall section 49a rotate from the first state so as to approach each other, a switch is made to the second state.

The hand dryer 47 may include an actuator (not illustrated) for moving the shielding body 46. For example, the actuator need only rotate and drive only one of the gear section 48b and the gear section 49b. By having the actuator rotate and drive only one of the gear section 48b and the gear section 49b, a torque thereof is transmitted to the other of the gear section 48b and the gear section 49b and both the first shielding body 48 and the second shielding body 49 can be rotated and moved.

### Fifth embodiment

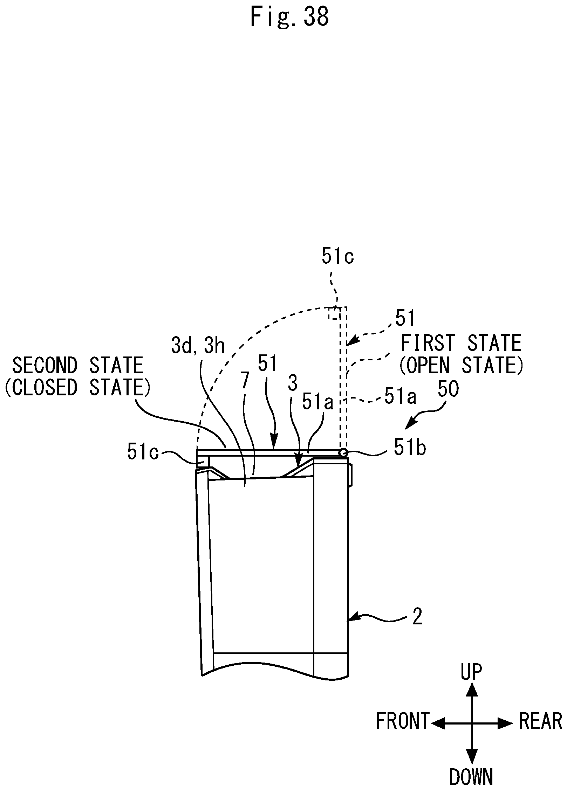

Next, while a fifth embodiment will be described with reference to Fig. 38, the description will focus on differences from the third embodiment described above and common descriptions will be simplified or omitted. In addition, elements in common with or corresponding to elements described earlier will be denoted by same reference signs. The fifth embodiment differs from the third embodiment in a configuration of a shielding body.

Fig. 38 is a side view of an upper part of a hand dryer 50 according to the fifth embodiment. As shown in Fig. 38, the hand dryer 50 according to the fifth embodiment includes a shielding body 51 in place of the shielding body 46 according to the third embodiment. In Fig. 38, the shielding body 51 at a position of the second state is depicted by a solid line while the shielding body 51 at a position of the first state is depicted by a dashed line.

The shielding body 51 includes a wall section 51a, a hinge section 51b connecting a proximal end section of the wall section 5 1a to a rear part of an upper part of the main body enclosure 2, and a projecting section 51c that protrudes from a distal end section of the wall section 51a. The shielding body 51 rotates and moves around the hinge section 51b. In the illustrated example, the wall section 51a has a flat-plate shape. A rotary shaft of the hinge section 51b is parallel to the left-right direction of the hand dryer 50.

In the second state or the closed state, the wall section 51a covers at least a part of the hand insertion opening 7 from above. In the second state, the wall section 51a becomes horizontal or approaches horizontal. In the second state, the projecting section 51c projects downward from the distal end section of the wall section 51a. In the second state, the projecting section 51c comes into contact with a front part of the upper part of the main body enclosure 2. When the wall section 51a rotates from the second state such that a position of a front end part of the wall section 51a rises, a switch is made to the first state or an open state.

### Sixth embodiment

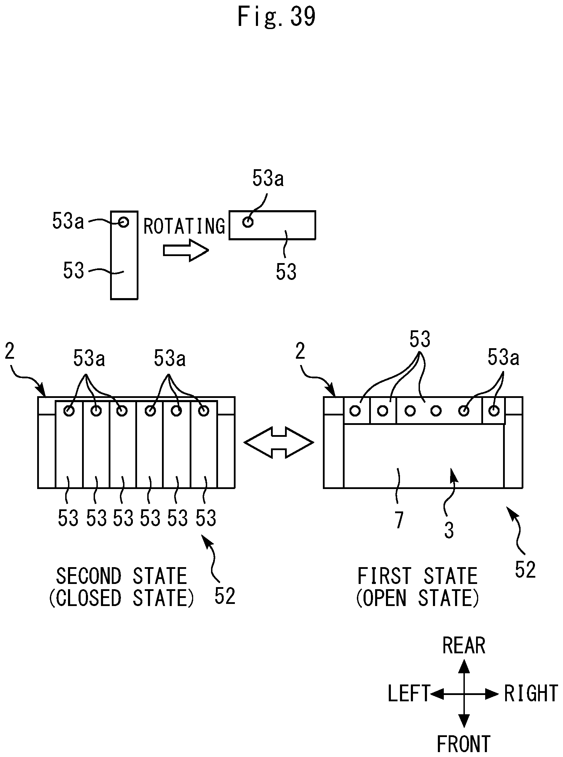

Next, while a sixth embodiment will be described with reference to Fig. 39, the description will focus on differences from the third embodiment described above and common descriptions will be simplified or omitted. In addition, elements in common with or corresponding to elements described earlier will be denoted by same reference signs. The sixth embodiment differs from the third embodiment in a configuration of a shielding body.

Fig. 39 is a schematic top view of a hand dryer 52 according to the sixth embodiment. As shown in Fig. 39, the hand dryer 52 according to the sixth embodiment includes a plurality of shielding bodies 53 in place of the shielding body 46 according to the third embodiment. Each of the plurality of shielding bodies 53 has a substantially rectangular plate shape. Each of the plurality of shielding bodies 53 is capable of rotating and moving around a rotary shaft 53a. Each rotary shaft 53a may be parallel to a vertical line. The shielding body 53 is connected to a rear part of an upper part of the main body enclosure 2 via the rotary shaft 53a. A wall surface of the shielding body 53 may be horizontal. The rotary shaft 53a is at a position close to one end in a longitudinal direction of the shielding body 53.

In the second state or a closed state, the longitudinal direction of each shielding body 53 becomes parallel to the front-rear direction of the hand dryer 52. In the second state, the plurality of shielding bodies 53 line up in a direction perpendicular to the longitudinal direction thereof. In the second state, the plurality of shielding bodies 53 line up in a left-right direction of the hand dryer 52. In the second state, the plurality of shielding bodies 53 cover at least a part of the hand insertion opening 7 from above.

When each shielding body 53 rotates by, for example, 90 degrees from the second state, a switch is made to the first state or the open state and the hand insertion opening 7 is opened. In the first state, the longitudinal direction of each shielding body 53 becomes parallel to the left-right direction of the hand dryer 52. In the first state, the plurality of shielding bodies 53 line up in a same direction as the longitudinal direction thereof. In the first state, each shielding body 53 is arranged so as to partially overlap with other shielding bodies 53 in a top view.

### Seventh embodiment

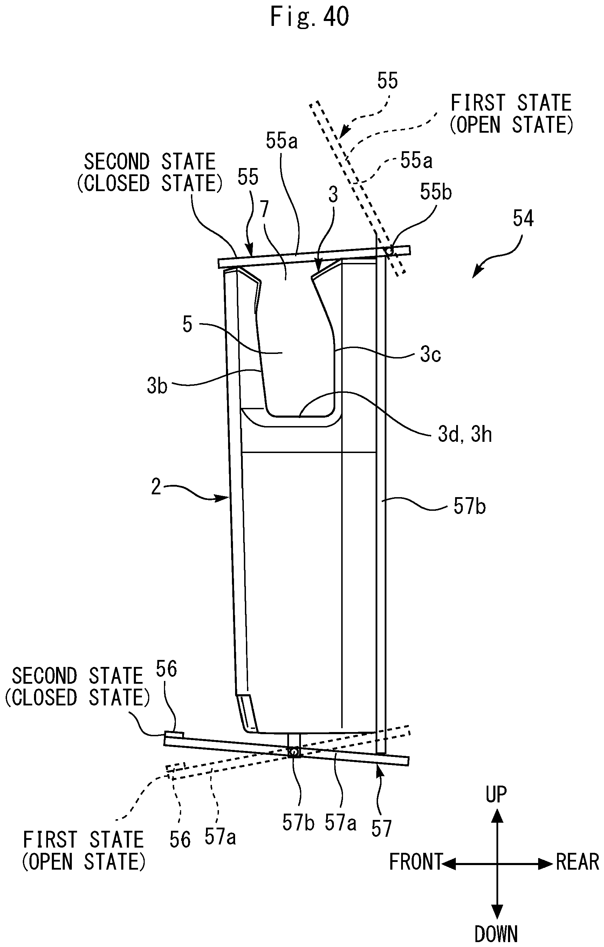

Next, while a seventh embodiment will be described with reference to Fig. 40, the description will focus on differences from the third embodiment described above and common descriptions will be simplified or omitted. In addition, elements in common with or corresponding to elements described earlier will be denoted by same reference signs. The seventh embodiment differs from the third embodiment in a configuration of a shielding body.

Fig. 40 is a side view of a hand dryer 54 according to the seventh embodiment. As shown in Fig. 40, the hand dryer 54 according to the seventh embodiment includes a shielding body 55 in place of the shielding body 46 according to the third embodiment. The shielding body 55 includes a wall section 55a and a hinge section 55b connecting a proximal end section of the wall section 55a to a rear part of an upper part of the main body enclosure 2. The shielding body 55 rotates and moves around the hinge section 55b. In the illustrated example, the wall section 55a has a flat-plate shape. A rotary shaft of the hinge section 55b is parallel to the left-right direction of the hand dryer 54.

In the second state or the closed state, the wall section 55a covers at least a part of the hand insertion opening 7 from above. In the second state, the wall section 55a becomes horizontal or approaches horizontal. When the wall section 55a rotates from the second state such that a position of a front end part of the wall section 55a rises, a switch is made to the first state or an open state.

The hand dryer 54 according to the present embodiment further includes a pedal 56 and a mover 57. The mover 57 moves the shielding body 55 such that a switch is made from the second state to the first state when the user steps on the pedal 56 and a switch is made from the first state to the second state when the user releases the pedal 56. According to the present embodiment, a switch can be made between the first state and the second state without the user touching the shielding body 55 with a hand. Therefore, superior hygiene is achieved.

For the sake of convenience, in Fig. 40, both the shielding body 55, the pedal 56, and the mover 57 at positions corresponding to the first state and the shielding body 55, the pedal 56, and the mover 57 at positions corresponding to the second state are depicted in solid lines.

The pedal 56 is arranged at a position corresponding to a foot of the user standing in front of the hand dryer 54. The mover 57 includes a lever 57a and a push rod 57b. The lever 57a is capable of rotating and moving around a fulcrum 57c. A rotary shaft of the fulcrum 57c is parallel to the left-right direction of the hand dryer 54. The pedal 56 is installed in a front end part of the lever 57a. When the lever 57a rotates and moves, the pedal 56 moves in the up-down direction. A lower end part of the push rod 57b is in contact with a rear end part of the lever 57a. An upper end part of the push rod 57b is in contact with the wall section 55a of the shielding body 55 in front of the hinge section 55b.

When the user steps on the pedal 56 from the second state where the shielding body 55 is closed, the front end part of the lever 57a descends while the rear end part of the lever 57a ascends. The push rod 57b is pushed by the rear end part of the lever 57a and ascends. As the upper end part of the ascended push rod 57b pushes the wall section 55a upward, the shielding body 55 changes to the open first state. When the user releases the pedal 56, a switch is made from the first state to the second state as the shielding body 55 returns to an original position due to its own weight.

According to the present embodiment, since the shielding body 55 can be moved using a weight of the user's foot, power consumption can be reduced.

### Eighth embodiment

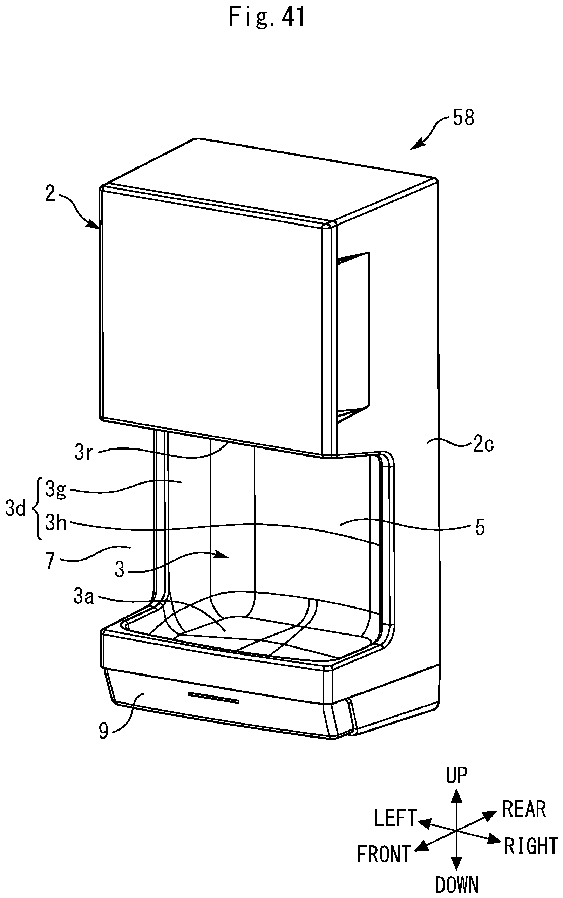

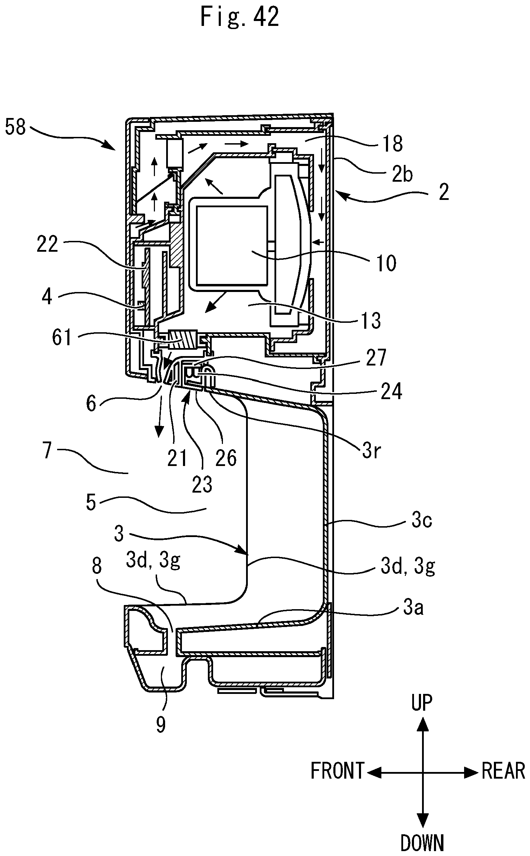

Next, while an eighth embodiment will be described with reference to Figs. 41 and 42, the description will focus on differences from the first embodiment described above and common descriptions will be simplified or omitted. In addition, elements in common with or corresponding to elements described earlier will be denoted by same reference signs.

Fig. 41 is a perspective view of a hand dryer 58 according to the eighth embodiment. Fig. 42 is a sectional side view of the hand dryer 58 according to the eighth embodiment. The hand insertion section 3 of the hand dryer 58 shown in these diagrams is configured such that the user can insert a hand into the hand insertion space 5 from the front of the hand dryer 58. The hand insertion opening 7 is opened so as to face the front of the hand dryer 58. The hand insertion section 3 includes an insertion upper section 3r. The insertion upper section 3r defines an upper part of the hand insertion space 5. The insertion upper section 3r opposes the insertion bottom section 3a. A surface of the insertion upper section 3r facing the hand insertion space 5 faces downward.

As shown in Fig. 42, inside the main body enclosure 2, the blower 10 is arranged at a position above the hand insertion section 3. The air hole 6 that constitutes a blowout port section, the hand detection section 21, and the UV irradiator 23 are arranged in the insertion upper section 3r. The blowout port section blows an airflow into the hand insertion space 5 downward from the air hole 6. At least a part of ultraviolet light emitted from the UV irradiator 23 is radiated downward. The hand dryer 58 may include a heater 61 that heats air passing through the blowout air path 13.

According to the present embodiment, in the outer appearance of the hand dryer 58, due to the light source 24 being covered by the main body enclosure 2, the light source 24 is not visually recognizable in any of a front view, a rear view, a side view, a top view, and a bottom view. Accordingly, the possibility that the user directly views the light source 24 can be reduced, a risk of a light beam entering an eye can be reduced, and a high level of safety is provided.

### Ninth embodiment

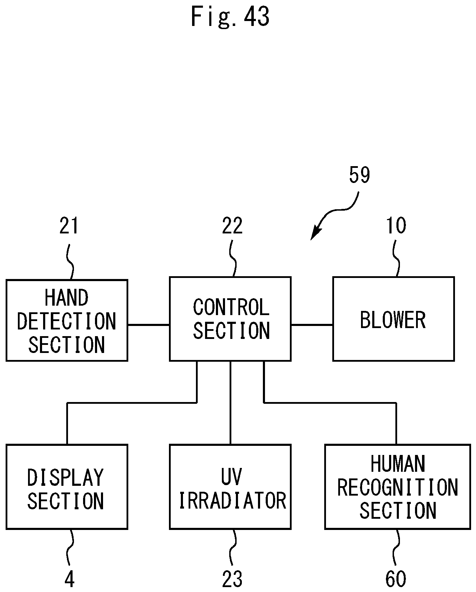

Next, while a ninth embodiment will be described with reference to Fig. 43, the description will focus on differences from the first embodiment described above and common descriptions will be simplified or omitted. In addition, elements in common with or corresponding to elements described earlier will be denoted by same reference signs.

Fig. 43 is a functional block diagram of a hand dryer 59 according to the ninth embodiment. As shown in Fig. 43, the hand dryer 59 according to the ninth embodiment includes a human recognition section 60. The human recognition section 60 is configured to identify a plurality of users. For example, the human recognition section 60 may identify individuals according to a face recognition technique or a face identification technique using a camera for photographing a face of a user or identify individuals according to a fingerprint authentication technique or a fingerprint identification technique using a fingerprint reader. The human recognition section 60 may identify individuals by, for example, reading identification information from an employee ID card, a membership card, an IC card, an RFID tag, or the like carried by the user and collating the read identification information with identification information stored in advance.

With respect to each user identified by the human recognition section 60, the control section 22 counts the number of times the individual had used the hand dryer 59 on the day (hereinafter, referred to as "use frequency"). When a specific user identified by the human recognition section 60 is to use the hand dryer 59 and the user's use frequency on the day is equal to or lower than a reference frequency, the control section 22 causes a hand of the user to be irradiated with ultraviolet light from the UV irradiator 23. Accordingly, microorganisms potentially adhering to the hand of the user can be sterilized by the ultraviolet light and hygiene is improved.

For example, the reference frequency may be a value determined in accordance with an average frequency of toilet use per day by a healthy adult.

Generally, given that ultraviolet light is potentially harmful to the human body, an allowable daily ultraviolet dosage is specified. Illuminance due to the UV irradiator 23 and an irradiation time per irradiation are preferably set using a relationship represented by the following expression so that, even when daily use frequency of a specific user reaches the reference frequency, a total amount of ultraviolet light irradiated to a hand of the user on the day equals or falls below a daily allowable dosage. Note that, as a daily allowable dosage or an 8-hour allowable dosage, a value based on TLV (Threshold Limit Values) specified by Japanese Industrial Standards JIS Z8812 may be used or a lower value than a value based on TLV may be used.

Daily allowable dosage
[mW/cm²] = illuminance of irradiated surface (hand)
[mJ/cm²] × upper limit of total daily irradiation time
[seconds] = illuminance of irradiated surface (hand)
[mJ/cm²] × irradiation time per irradiation
[seconds] × reference frequency

The control section 22 is configured to, when a specific user identified by the human recognition section 60 is to use the hand dryer 59, reduce the output of the light source 24 of the UV irradiator 23 or change the output of the light source 24 to zero when the user's use frequency on the day exceeds the reference frequency as compared to when the user's use frequency on the day is equal to or lower than the reference frequency. Accordingly, a total amount of ultraviolet light irradiated to a hand of the user can be more reliably prevented from exceeding a daily allowable dosage.

When the use frequency of a specific user identified by the human recognition section 60 on the day exceeds the reference frequency, the control section 22 may cause the output of the light source 24 to approach zero as the use frequency increases.

The hand dryer according to the present disclosure may further include a reception section which receives a signal for controlling on/off states of emission by the UV irradiator 23. The control section 22 may control on/off states of emission by the UV irradiator 23 when the reception section receives a detection signal of a sensor provided in the hand dryer. In addition, the control section 22 may control on/off states of emission by the UV irradiator 23 when the reception section receives a detection signal of a sensor provided outside the hand dryer. The reception section may be capable of receiving a signal from a remote location. For example, the control section 22 may control on/off states of emission by the UV irradiator 23 when the reception section receives a signal from a terminal 103 used by a manager responsible for management or maintenance of the hand dryer. The terminal 103 will be described later.

### Tenth embodiment

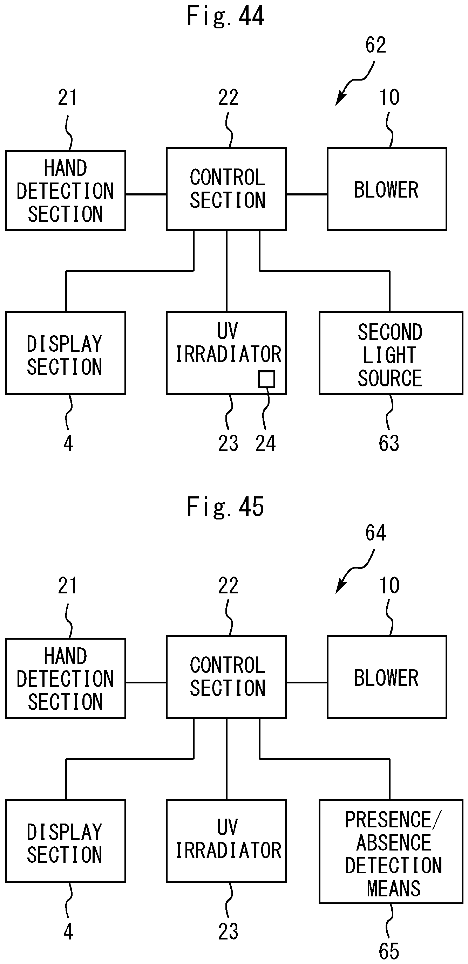

Next, while a tenth embodiment will be described with reference to Fig. 44, the description will focus on differences from the embodiments described above and common descriptions will be simplified or omitted. In addition, elements in common with or corresponding to elements described earlier will be denoted by same reference signs.

Fig. 44 is a functional block diagram of a hand dryer 62 according to the tenth embodiment. As shown in Fig. 44, the hand dryer 62 according to the tenth embodiment includes a second light source 63. The light source 24 of the UV irradiator 23 corresponds to a first light source. A dominant wavelength of the second light source 63 is longer than the dominant wavelength of the first light source 24. The control section 22 is configured to turn on the second light source 63 without turning on the first light source 24 when the hand detection section 21 detects a hand.

Since a wavelength of light emitted by the second light source 63 is longer than the wavelength of light emitted by the light source 24, a higher level of safety is provided. According to the present embodiment, when a hand is inserted into or arranged on the hand insertion section 3, the hand can be sterilized safely by irradiating the hand and the hand insertion section 3 with light from the second light source 63. In addition, since the hand dryer 62 is mounted with the UV irradiator 23 and the user more readily recognizes that the level of hygiene is high, the user can be provided with a sense of security.

When the dominant wavelength of the light source 24 is UVC, the dominant wavelength of the second light source 63 may be any of UVB, UVA, and visible light. When the dominant wavelength of the light source 24 is UVB, the dominant wavelength of the second light source 63 may be UVA or visible light. When the dominant wavelength of the light source 24 is UVA, the dominant wavelength of the second light source 63 may be visible light. Note that the second light source 63 may be integrated with the UV irradiator 23 or the second light source 63 may be provided at a different position from the UV irradiator 23.

### Eleventh embodiment

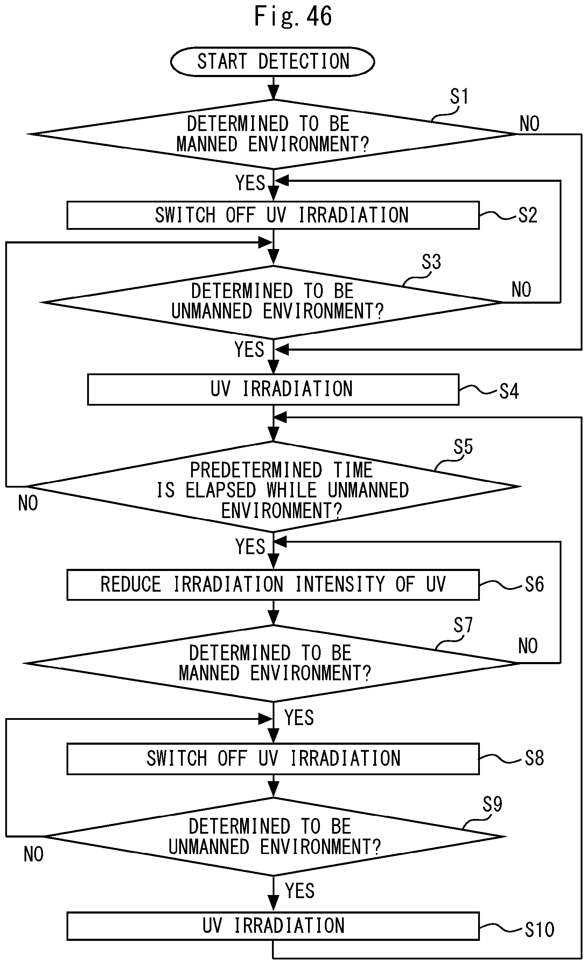

Next, while an eleventh embodiment will be described with reference to Figs. 45 and 46, the description will focus on differences from the embodiments described above and common descriptions will be simplified or omitted. In addition, elements in common with or corresponding to elements described earlier will be denoted by same reference signs.

In the present disclosure, another device that cooperates with a hand dryer and the hand dryer may be collectively referred to as a hand-drying system. Note that the "other device" may be arranged at a position separated from the hand dryer, arranged adjacent to the hand dryer, or integrally arranged with the hand dryer. The hand dryer included in the hand-drying system according to the present disclosure may be the same as or similar to the hand dryer according to any one of the first to tenth embodiments described above.

Fig. 45 is a functional block diagram of a hand-drying system 64 according to the eleventh embodiment. The hand dryer according to the present disclosure is arranged in a user region being a specific region. The user region is a region which a user of the hand dryer may possibly enter. As shown in Fig. 45, the hand-drying system 64 according to the tenth embodiment includes presence/absence detection means 65 for detecting whether or not a person is present in the user region.

In the present disclosure, a space including at least one of a toilet bowl and a urinal, a hand dryer, and a hand-wash station is referred to as a "toilet space". A toilet space may correspond to a user region. A partition such as a door may be provided at an entrance to the toilet space or the partition may not be provided. In commercial facilities and the like, an L-shaped passageway may be provided before the entrance of a toilet space, in which case the L-shaped passageway doubles as a partition. In such a case, the L-shaped space may be considered a part of the toilet space or a space excluding the L-shaped space may be considered the toilet space. In addition, a multipurpose toilet may be considered a toilet space. The user region according to the present disclosure is not limited to a toilet space. For example, the user region according to the present disclosure may be a space in which a toilet bowl is not arranged but which includes a hand dryer and a hand-wash station.

The presence/absence detection means 65 may detect whether or not a person is present in the user region according to any principle. Hereinafter, an example of the presence/absence detection means 65 will be described. The presence/absence detection means 65 may detect the presence or absence of a person using a human sensor such as an infrared sensor arranged in the user region. The presence/absence detection means 65 may detect the presence or absence of a person by processing an image of a camera that photographs the user region.

The presence/absence detection means 65 may detect the presence or absence of a person by detecting at least one of a brightness of the user region and an on/off state of an illuminator which illuminates the user region. The user may manually turn the illuminator on/off or the illuminator may be automatically turned on/off according to a signal of a human sensor such as an infrared sensor. When the illuminator is turned on, the user region becomes bright and a determination that a person is present in the user region can be made. Conversely, when the illuminator is turned off, the user region becomes dark and a determination that a person is not present in the user region can be made.

The presence/absence detection means 65 may detect an on/off state of the illuminator by detecting a current value or a voltage of energization to the illuminator by electric detection means that is electrically connected to the illuminator.

The presence/absence detection means 65 may determine that the illuminator is turned on and a person is present in the user region when brightness of the user region is higher than a threshold and determine that the illuminator is turned off and a person is not present in the user region when brightness of the user region is lower than the threshold. The presence/absence detection means 65 may detect illuminance of the user region as the brightness of the user region. In addition, the hand-drying system 64 may further include threshold adjustment means capable of adjusting the threshold. The brightness of a user region when the illuminator is turned off may possibly differ depending on conditions of a periphery of the user region. For example, when the illuminator is turned off, the user region may become brighter to a certain degree due to sunlight that comes in from a window. In this case, by configuring the threshold adjustment means to be capable of adjusting the threshold for detecting whether the illuminator is turned on or off, a detection can be made with higher accuracy. For example, the threshold adjustment means may change the threshold in accordance with information received from the terminal 103 used by a manager responsible for management or maintenance of the hand dryer.

The presence/absence detection means 65 may detect brightness of the user region by detecting a specific wavelength that is emitted from the illuminator and detect the presence or absence of a person in accordance with the brightness of the user region due to the specific wavelength. Accordingly, whether the illuminator is turned on or off can be detected with higher accuracy without being influenced by a change in the brightness of the user region due to sunlight. Therefore, the presence/absence detection means 65 can detect whether or not a person is present in the user region with higher accuracy.

The presence/absence detection means 65 may detect the presence or absence of a person by door lock detection means for detecting whether or not a door of an entrance of a user region or a door of a cubicle provided in the user region is locked. For example, the door lock detection means may detect whether or not the door of a cubicle in which a toilet bowl is installed is locked. For example, the door lock detection means may detect whether or not the door of an entrance of a multipurpose toilet is locked. When the door is locked, the presence/absence detection means 65 can determine that there is a person inside. When the door is not locked, the presence/absence detection means 65 can determine that there is no one inside.

The presence/absence detection means 65 may detect the presence or absence of a person by detecting a sound in the user region. For example, the presence/absence detection means 65 may detect a sound of a door being opened or closed, a sound of water being flushed down a toilet, a sound of water flowing in a hand-wash station, a sound of a person walking, voices talking, or the like, and determine that a person is present when a sound is detected but determine that a person is absent when a sound is not detected. The presence/absence detection means 65 may detect a specific sound by storing reference waveforms of sound waves of specific sounds such as those described above in advance and comparing a waveform of a sound wave of a detected sound with the reference waveforms.

The control section 22 of the hand-drying system 64 may be configured to cause the UV irradiator 23 to emit ultraviolet light when the presence/absence detection means 65 determines that there is no one in the user region. Ultraviolet light may be harmful to the human body. Causing the UV irradiator 23 to emit ultraviolet light to perform sterilization when there is no one in the user region more reliably satisfies both safety and hygiene.

The control section 22 of the hand-drying system 64 may be configured to reduce the output of the light source 24 of the UV irradiator 23 or change the output of the light source 24 to zero when the presence/absence detection means 65 determines that there is a person in the user region. Since ultraviolet light can be more reliably prevented from striking someone by reducing the output of the light source 24 of the UV irradiator 23 or changing the output of the light source 24 to zero when there is a person in the user region, the level of safety is further improved.

The control section 22 of the hand-drying system 64 may be configured to reduce an output of the light source 24 or to change the output of the light source 24 to zero in the event that a duration of emission of ultraviolet light by the UV irradiator 23 exceeds a criterion when the presence/absence detection means 65 has determined that there is no one in the user region. Accordingly, the irradiation time per one irradiation can be prevented from becoming longer than necessary. Therefore, there are advantages in terms of extending a lifetime of the light source 24, preventing a decline in transmittance of the window 26, preventing a deterioration of constituent materials of the hand insertion section 3, and the like.

In the present disclosure, a state where the UV irradiator 23 is not emitting ultraviolet light will also be referred to as an "off state". The control section 22 of the hand-drying system 64 may switch the UV irradiator 23 to the off state until a predetermined amount of time elapses from a time point where the presence/absence detection means 65 had last detected that a person is present in the user region. Accordingly, even when the presence/absence detection means 65 instantaneously detects, by mistake, that there is no one when there is actually a person present in the user region, the off state of the UV irradiator 23 is continued. Therefore, since ultraviolet light can be more reliably prevented from striking human bodies, the level of safety is further improved.

The control section 22 of the hand-drying system 64 may both, in combination, switch the UV irradiator 23 to the off state while the presence of a person in the user region is being detected by the presence/absence detection means 65 and switch the UV irradiator 23 to the off state until a predetermined amount of time elapses from a time point where the presence/absence detection means 65 had last detected that a person is present in the user region. Accordingly, the level of safety is further improved.

Fig. 46 is a flowchart showing an example of processing to be executed by the hand-drying system 64 according to the eleventh embodiment. Hereinafter, a description will be given based on the flowchart shown in Fig. 46. In the present disclosure, a state where a person is present in the user region may be referred to as a "manned environment" while a state where a person is present in the user region may be referred to as an "unmanned environment".

When the presence/absence detection means 65 detects that a manned environment exists in step S 1 in Fig. 46, the control section 22 switches the UV irradiator 23 to the off state as step S2. In the event that the presence/absence detection means 65 detects that an unmanned environment exists as step S3 when the UV irradiator 23 is in the off state, the control section 22 causes ultraviolet light to be emitted from the UV irradiator 23 as step S4. In addition, when the presence/absence detection means 65 has not detected that a manned environment exists or the presence/absence detection means 65 has detected that an unmanned environment exists in step S 1, the control section 22 causes ultraviolet light to be emitted from the UV irradiator 23 as step S4. Furthermore, when the presence/absence detection means 65 has not detected that an unmanned environment exists or the presence/absence detection means 65 has detected that a manned environment exists in step S3, the control section 22 switches the UV irradiator 23 to the off state as step S2.

After step S4, the control section 22 determines whether or not a predetermined amount of time has elapsed while an unmanned environment is being detected by the presence/absence detection means 65 as step S5. When the predetermined amount of time has not yet elapsed while an unmanned environment is being detected by the presence/absence detection means 65, a return is made to step S3. When the predetermined amount of time has already elapsed while an unmanned environment is being detected by the presence/absence detection means 65, the control section 22 causes irradiation intensity of the UV irradiator 23 to decline by reducing the output of the light source 24 as step S6.

After step S6, when the presence/absence detection means 65 detects that a manned environment exists in step S7, the control section 22 switches the UV irradiator 23 to the off state as step S8. By comparison, when the presence/absence detection means 65 has not detected that a manned environment exists or the presence/absence detection means 65 has detected that an unmanned environment exists in step S7, a return is made to step S6.

After step S8, when the presence/absence detection means 65 detects that an unmanned environment exists in step S9, the control section 22 causes ultraviolet light to be emitted from the UV irradiator 23 as step S10. By comparison, when the presence/absence detection means 65 has not detected that an unmanned environment exists or the presence/absence detection means 65 has detected that a manned environment exists in step S9, the control section 22 switches the UV irradiator 23 to the off state as step S8.

The control section 22 of the hand dryer according to the present disclosure may be provided with a non-insertion time drive mode in which the blower 10 is driven when the hand detection section 21 is not detecting a hand as a control mode. The non-insertion time drive mode is an operation that produces an air volume or an air speed that is equivalent to a minimum air volume or a minimum air speed when the blower 10 is driven in order to dry a hand or an operation that produces an air volume or an air speed that is lower than the minimum air volume or the minimum air speed. For example, in the non-insertion time drive mode, the control section 22 drives the blower 10 so that a rotational speed of the blower 10 is equal to the rotational speed of the blower 10 when drying a hand or lower than the rotational speed of the blower 10 when drying a hand.

In the non-insertion time drive mode, the control section 22 may cause ultraviolet light to be emitted from the UV irradiator 23. Accordingly, since an airflow generated by the blower 10 is irradiated with the ultraviolet light, the air in the user region can be sterilized.

The hand dryer according to the present disclosure may include an air filter for filtering an airflow generated by the blower 10. The air filter may be a HEPA filter. In the non-insertion time drive mode, the air in the user region can be cleaned by having the airflow generated by the blower 10 filtered by the air filter.

The control section 22 may execute the non-insertion time drive mode after the hand dryer is used by a large number of users. Accordingly, the air in the user region that has possibly been polluted by use by a large number of users can be sterilized or cleaned. For example, the control section 22 can determine whether or not a large number of users have used the hand dryer based on the number of times the hand detection section 21 had detected a hand.

In the present disclosure, the control section 22 may have a timer function for managing the time of day. In addition, the control section 22 may have a calendar function for managing dates and days of the week.

The control section 22 may execute the non-insertion time drive mode during a predetermined time slot of a day. For example, the control section 22 may execute the non-insertion time drive mode during late night hours or early-morning hours. Accordingly, the non-insertion time drive mode can be executed to sterilize or clean the air in the user region during time slots in which the frequency of use of the hand dryer is low.

In the hand-drying system 64, the control section 22 may execute the non-insertion time drive mode when the presence/absence detection means 65 is detecting that there is no one in the user region. Accordingly, the air in the user region can be sterilized or cleaned when there is no one in the user region.

### Twelfth embodiment

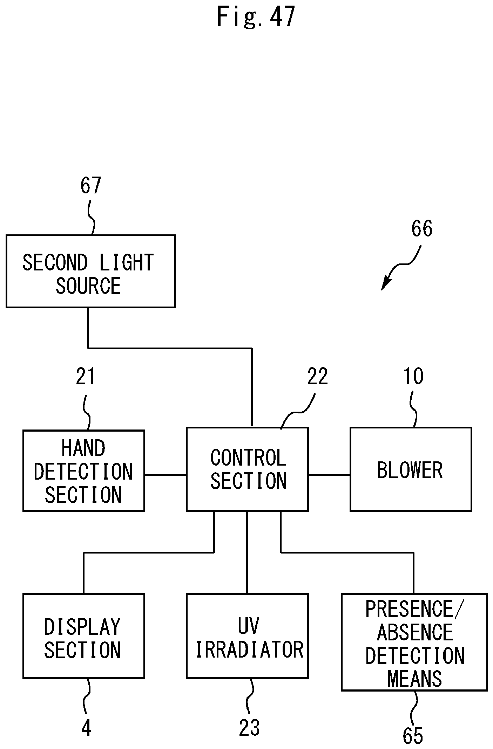

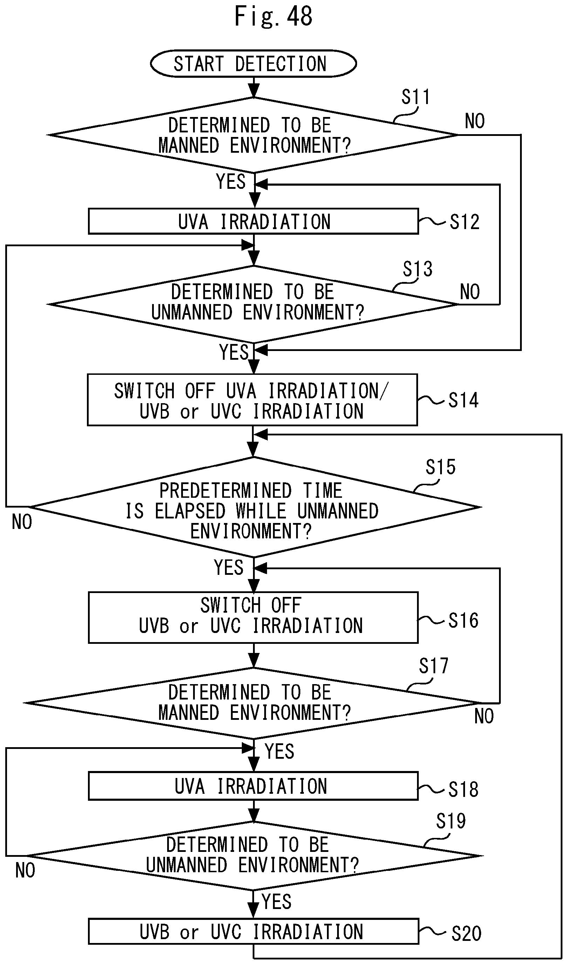

Next, while a twelfth embodiment will be described with reference to Figs. 47 and 48, the description will focus on differences from the eleventh embodiment described above and common descriptions will be simplified or omitted. In addition, elements in common with or corresponding to elements described earlier will be denoted by same reference signs.

Fig. 47 is a functional block diagram of a hand-drying system 66 according to the twelfth embodiment. As shown in Fig. 47, the hand-drying system 66 according to the twelfth embodiment includes a second light source 67 and the presence/absence detection means 65 described in the eleventh embodiment. The light source 24 of the UV irradiator 23 corresponds to a first light source. A dominant wavelength of the second light source 67 is longer than the dominant wavelength of the first light source 24. When the dominant wavelength of the light source 24 is UVC, the dominant wavelength of the second light source 67 may be any of UVB, UVA, and visible light. When the dominant wavelength of the light source 24 is UVB, the dominant wavelength of the second light source 67 may be UVA or visible light. When the dominant wavelength of the light source 24 is UVA, the dominant wavelength of the second light source 67 may be visible light.

The second light source 67 may be arranged so as to emit light toward the hand insertion section 3 or the hand insertion space 5. The second light source 67 may be integrated with the UV irradiator 23. For example, the optical axis of the light source 24 and an optical axis of the second light source 67 may be parallel to each other. The second light source 67 may be adjacent to the light source 24. Alternatively, the second light source 67 may be provided at a different position from the UV irradiator 23.

Alternatively, the second light source 67 may be arranged so as to emit light toward the outside of the hand dryer. For example, the second light source 67 may be provided as a part of the display section 4 on an upper face of the hand dryer.

In the hand-drying system 66, the control section 22 is configured to turn off the light source 24 of the UV irradiator 23 and turn on the second light source 67 in the event that a duration of lighting of the light source 24 exceeds a criterion when the presence/absence detection means 65 has determined that there is no one in the user region. Since a wavelength of light emitted by the second light source 67 is longer than the wavelength of light emitted by the light source 24, an effect of emitted light on materials struck by the light is small. Since it is conceivable that microorganisms have been reduced by the time the duration of lighting of the light source 24 of the UV irradiator 23 exceeds a criterion, there is not much need to further emit ultraviolet light from the UV irradiator 23. Therefore, by turning off the light source 24 of the UV irradiator 23 and turning on the second light source 67 in the event that a duration of lighting of the light source 24 exceeds a criterion, sterilization by ultraviolet light from the second light source 67 can be continued while avoiding deterioration of materials at positions that may be struck by the emitted light such as the hand insertion section 3 or walls of the user region. In addition, when the second light source 67 emits visible light, albeit a sterilization effect is low, showing the user the visible light from the second light source 67 enables the user to be notified that sterilization has been completed. Therefore, the user can be provided with a sense of security.

Fig. 48 is a flowchart showing an example of processing to be executed by the hand-drying system 66 according to the twelfth embodiment. Hereinafter, a description will be given based on the flowchart shown in Fig. 48. When the presence/absence detection means 65 detects that a manned environment exists in step S11 in Fig. 48, the control section 22 causes UVA to be emitted from the second light source 67 and switches the UV irradiator 23 to the off state as step S12. After step S12, when the presence/absence detection means 65 detects that an unmanned environment exists in step S13, the control section 22 stops the emission of UVA from the second light source 67 and causes UVB or UVC to be emitted from the UV irradiator 23 as step S14. By comparison, when the presence/absence detection means 65 has not detected that an unmanned environment exists or the presence/absence detection means 65 has detected that a manned environment exists in step S13, a return is made to step S12. In addition, when the presence/absence detection means 65 has not detected that a manned environment exists or the presence/absence detection means 65 has detected that an unmanned environment exists in step S11, the control section 22 stops the emission of UVA from the second light source 67 and causes UVB or UVC to be emitted from the UV irradiator 23 as step S14.

After step S14, the control section 22 determines whether or not a predetermined amount of time has elapsed while an unmanned environment is being detected by the presence/absence detection means 65 as step S15. When the predetermined amount of time has not yet elapsed while an unmanned environment is being detected by the presence/absence detection means 65, a return is made to step S13. When the predetermined amount of time has already elapsed while an unmanned environment is being detected by the presence/absence detection means 65, the control section 22 switches the UV irradiator 23 to the off state and stops emission of UVB or UVC as step S16.

After step S16, when the presence/absence detection means 65 detects that a manned environment exists in step S 17, the control section 22 causes UVA to be emitted from the second light source 67 as step S18. By comparison, when the presence/absence detection means 65 has not detected that a manned environment exists or the presence/absence detection means 65 has detected that an unmanned environment exists in step S 17, a return is made to step S16.

After step S18, when the presence/absence detection means 65 detects that an unmanned environment exists as step S 19, the control section 22 causes UVB or UVC to be emitted from the UV irradiator 23 as step S20. At this point, the control section 22 may stop emission of UVA from the second light source 67. On the other hand, when the presence/absence detection means 65 has not detected that an unmanned environment exists or the presence/absence detection means 65 has detected that a manned environment exists in step S19, a return is made to step S18.

### Thirteenth embodiment

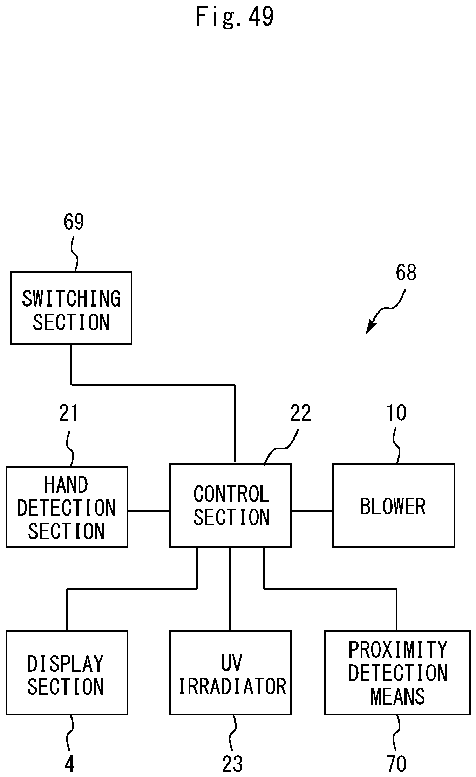

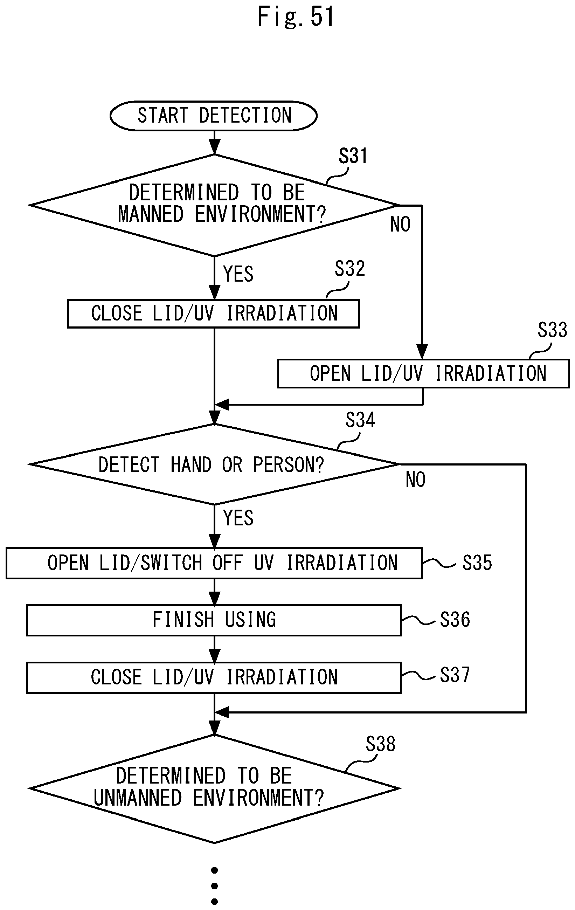

Next, while a thirteenth embodiment will be described with reference to Figs. 49 and 51, the description will focus on differences from the embodiments described above and common descriptions will be simplified or omitted. In addition, elements in common with or corresponding to elements described earlier will be denoted by same reference signs.

Fig. 49 is a functional block diagram of a hand-drying system 68 according to the thirteenth embodiment. The hand-drying system 68 according to the thirteenth embodiment includes a cover section (not illustrated) capable of covering at least a surface of the hand insertion space 5 of the hand dryer. For example, the cover section may be configured in the same or similar manner to the shielding body described in any of the third to seventh embodiments.

As shown in Fig. 49, the hand-drying system 68 includes a switching section 69 and proximity detection means 70. The switching section 69 includes an actuator that moves the cover section. The switching section 69 moves the cover section so that the cover section can be switched between a closed state where the cover section covers at least one face of the hand insertion space 5 and an open state where a hand can be inserted into the hand insertion space 5.

The proximity detection means 70 is capable of detecting that a hand or a person is in proximity to the hand insertion section 3 when the cover section is in the closed state. The proximity detection means 70 may be configured in the same or similar manner to the human detection section 39 described earlier. Alternatively, the proximity detection means 70 may be configured in the same or similar manner to the presence/absence detection means 65 described earlier. In other words, the proximity detection means 70 may be capable of detecting whether a user region is a manned environment or an unmanned environment.

The proximity detection means 70 in the present embodiment is provided separately from the hand detection section 21. When the hand detection section 21 detects a hand, the control section 22 dries the hand by actuating the blower 10. In other words, detection of a hand by the hand detection section 21 triggers actuation of the blower 10. By comparison, the proximity detection means 70 detects that a person or a hand has approached the hand dryer. When a person or a hand approaches the hand dryer, the proximity detection means 70 first detects the person or the hand before the hand detection section 21 does. Subsequently, as the hand further approaches the hand insertion section 3, the hand detection section 21 detects the hand.

The hand detection section 21 is arranged at a position where the hand detection section 21 does not detect a hand when the cover section is in the closed state. For example, the hand detection section 21 is arranged in an inner space formed by the hand insertion section 3 and the cover section.

The cover section may cover an upper face of the hand insertion section 3. Positions covered by the cover section differs depending on an aspect of the hand dryer. For example, in the case of the hand insertion section 3 with a side face opened, the cover section may cover the side face of the hand insertion section 3. In addition, in the case of the hand insertion section 3 with a front face opened, the cover section may cover the front face of the hand insertion section 3. In addition, these features may be combined. In other words, the cover section may cover a plurality of faces of the hand insertion section 3. Furthermore, a plurality of cover sections may be provided. Each of the plurality of cover sections may be configured to cover a plurality of faces of the hand insertion section 3.

In the hand-drying system 68, the control section 22 drives the switching section 69 so that the cover section is changed to the open state when the proximity detection means 70 detects that a hand or a person is in proximity to the hand insertion section 3. Accordingly, since the cover section automatically changes to the open state when a hand or a person is in proximity to the hand insertion section 3, the user can smoothly insert a hand into the hand insertion space 5.

In the hand-drying system 68, the control section 22 may drive the switching section 69 so that the cover section is changed to the closed state when a duration in which the proximity detection means 70 is detecting that a hand or a person is not in proximity to the hand insertion section 3 reaches a predetermined amount of time. Furthermore, when the cover section is in the closed state, the control section 22 may cause ultraviolet light to be emitted from the UV irradiator 23. In the event that ultraviolet light is emitted from the UV irradiator 23 when the cover section is in the closed state, since the ultraviolet light is blocked by the cover section, the ultraviolet light can be prevented from being radiated outside of the hand insertion space 5. Therefore, both more reliably preventing ultraviolet light from striking a person and more reliably sterilizing the hand insertion space 5 can be achieved.

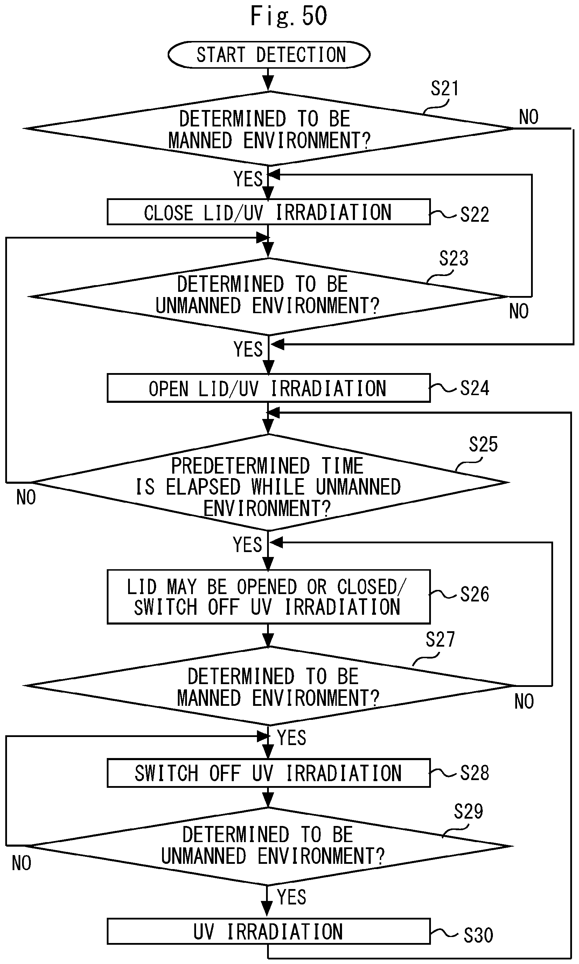

Fig. 50 is a flowchart showing an example of processing to be executed by the hand-drying system 68 according to the thirteenth embodiment. Hereinafter, a description will be given based on the flowchart shown in Fig. 50. In the present disclosure, the "cover section" or the "shielding body" described above may be referred to as a "lid".

When the proximity detection means 70 detects that a manned environment exists in step S21 in Fig. 50, the control section 22 changes the lid to the closed state using the switching section 69 and causes ultraviolet light to be emitted from the UV irradiator 23 as step S22.

After step S22, when the proximity detection means 70 detects that an unmanned environment exists in step S23, the control section 22 changes the lid to the open state using the switching section 69 and causes ultraviolet light to be emitted from the UV irradiator 23 as step S24. In addition, when the proximity detection means 70 has not detected that a manned environment exists or the proximity detection means 70 has detected that an unmanned environment exists in step S21, the control section 22 changes the lid to the open state using the switching section 69 and causes ultraviolet light to be emitted from the UV irradiator 23 as step S24. In this manner, according to the present embodiment, by changing the lid to the open state and causing ultraviolet light to be emitted from the UV irradiator 23 in an unmanned environment, the air in the user region can be sterilized while reliably preventing ultraviolet light from striking a person.

When the proximity detection means 70 has not detected that an unmanned environment exists or the proximity detection means 70 has detected that a manned environment exists in step S23, a return is made to step S22.

After step S24, the control section 22 determines whether or not a predetermined amount of time has elapsed while an unmanned environment is being detected by the proximity detection means 70 as step S25. When the predetermined amount of time has not yet elapsed while an unmanned environment is being detected by the proximity detection means 70, a return is made to step S23. When the predetermined amount of time has already elapsed while an unmanned environment is being detected by the proximity detection means 70, the control section 22 switches the UV irradiator 23 to the off state as step S26. At this point, the control section 22 may change the lid to the open state or to the closed state.

After step S26, when the proximity detection means 70 detects that a manned environment exists in step S27, the control section 22 switches the UV irradiator 23 to the off state as step S28. By comparison, when the proximity detection means 70 has not detected that a manned environment exists or the proximity detection means 70 has detected that an unmanned environment exists in step S27, a return is made to step S26.

After step S28, when the proximity detection means 70 detects that an unmanned environment exists in step S29, the control section 22 causes ultraviolet light to be emitted from the UV irradiator 23 as step S30. By comparison, when the proximity detection means 70 has not detected that an unmanned environment exists or the proximity detection means 70 has detected that a manned environment exists in step S29, a return is made to step S28.

Fig. 51 is a flowchart showing another example of processing to be executed by the hand-drying system 68 according to the thirteenth embodiment. Hereinafter, a description will be given based on the flowchart shown in Fig. 51. When the proximity detection means 70 detects that a manned environment exists in step S31, the control section 22 changes the lid to the closed state using the switching section 69 and causes ultraviolet light to be emitted from the UV irradiator 23 as step S32. By comparison, when the proximity detection means 70 has not detected that a manned environment exists or the proximity detection means 70 has detected that an unmanned environment exists in step S31, the control section 22 changes the lid to the open state using the switching section 69 and causes ultraviolet light to be emitted from the UV irradiator 23. In this manner, according to the present embodiment, by changing the lid to the open state and causing ultraviolet light to be emitted from the UV irradiator 23 in an unmanned environment, the air in the user region can be sterilized while reliably preventing ultraviolet light from striking a person.

After step S32 or step S33, when the proximity detection means 70 detects a hand or a person or when the hand detection section 21 detects a hand in step S34, the control section 22 changes the lid to the open state using the switching section 69 and switches the UV irradiator 23 to the off state.

After step S35, when the user finishes using the hand dryer in step S36, the control section 22 changes the lid to the closed state using the switching section 69 and causes ultraviolet light to be emitted from the UV irradiator 23 as step S37.

After step S37, the control section 22 determines whether or not the proximity detection means 70 is detecting that an unmanned environment exists in step S38. Subsequently, when the predetermined amount of time elapses while an unmanned environment is being detected by the proximity detection means 70, the control section 22 may switch the UV irradiator 23 to the off state. In addition, when the proximity detection means 70 has not detected a hand or a person and the hand detection section 21 has not detected a hand in step S34, the control section 22 proceeds to step S38.

### Fourteenth embodiment

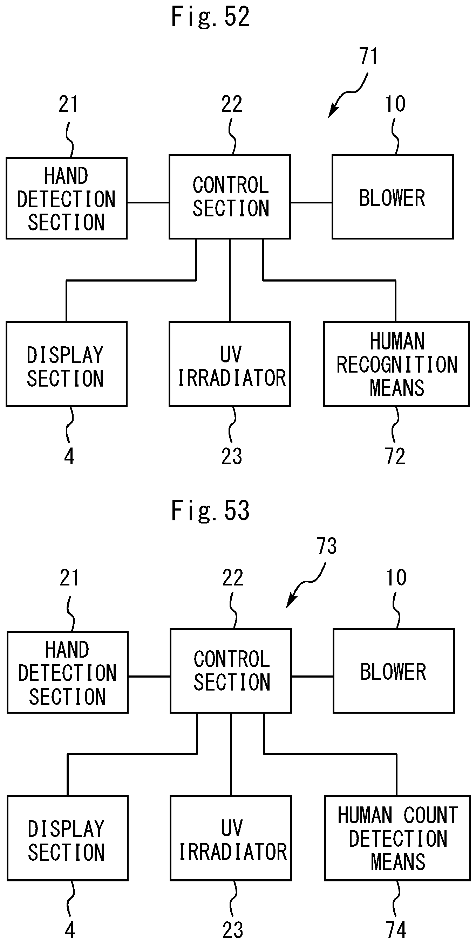

Next, while a fourteenth embodiment will be described with reference to Fig. 52, the description will focus on differences from the embodiments described above and common descriptions will be simplified or omitted. In addition, elements in common with or corresponding to elements described earlier will be denoted by same reference signs.

Fig. 52 is a functional block diagram of a hand-drying system 71 according to the fourteenth embodiment. As shown in Fig. 52, the hand-drying system 71 according to the fourteenth embodiment includes: human recognition means 72 for recognizing a same user; human storage means for counting a use frequency by a same user; and output variable means which changes an output of ultraviolet light emission by the UV irradiator 23 in accordance with a daily use frequency by a same user. In the present embodiment, the control section 22 corresponds to the human storage means and the output variable means.

In the hand-drying system 71, the UV irradiator 23 irradiates a hand of the user with ultraviolet light. Accordingly, microorganisms potentially adhering to the hand of the user can be sterilized by the ultraviolet light and hygiene is improved. In addition, with the hand-drying system 71, since a daily ultraviolet dosage from the UV irradiator 23 by a same user can be limited, safety is improved.

The human recognition means 72 may identify a same user by, for example, reading identification information from an employee ID card, a membership card, an IC card, an RFID tag, or the like carried by the user and collating the read identification information with identification information stored in advance. For example, the human recognition means 72 may identify the same user according to a face recognition technique or a face identification technique using a camera for photographing a face of a user or identify the same user according to a fingerprint authentication technique or a fingerprint identification technique using a fingerprint reader. The human recognition means 72 may be configured in the same or similar manner to the human recognition section 60 described earlier. When the human recognition means 72 recognizes a same person, the human storage means counts use frequency.

As a first example, the output change means may lower intensity or illuminance of ultraviolet light emitted from the UV irradiator 23 every time the daily use frequency by the same user increases.

As a second example, the output change means may lower intensity or illuminance of ultraviolet light emitted from the UV irradiator 23 when the daily use frequency by the same user exceeds a predetermined number of times.

As a third example, the output change means may change the output of the light source 24 of the UV irradiator 23 to zero when the daily use frequency by the same user exceeds a predetermined number of times.

The hand-drying system 71 may execute, in combination, any two controls or all three controls among the first to third examples described above. It is needless to say that these controls are not executed when the hand dryer is used by another user.

In the hand-drying system 71, the predetermined number of times described above and intensity or illuminance of ultraviolet light emitted from the UV irradiator 23 may be determined so that an amount of ultraviolet light emitted from the UV irradiator 23 with respect to a same user is equal to or smaller than the daily allowable dosage or the 8-hour allowable dosage described earlier.

### Fifteenth embodiment

Next, while a fifteenth embodiment will be described with reference to Fig. 53, the description will focus on differences from the embodiments described above and common descriptions will be simplified or omitted. In addition, elements in common with or corresponding to elements described earlier will be denoted by same reference signs.

Fig. 53 is a functional block diagram of a hand-drying system 73 according to the fifteenth embodiment. As shown in Fig. 53, the hand-drying system 73 according to the fifteenth embodiment includes human count detection means 74 for detecting the number of people in the user region. The control section 22 controls the UV irradiator 23 so as to emit ultraviolet light when the number of people in the user region is zero. In addition, the control section 22 stops emission of ultraviolet light by the UV irradiator 23 when the number of people in the user region is one or more. Accordingly, since ultraviolet light can be more reliably prevented from irradiating people from the UV irradiator 23, the level of safety is further improved.

The human count detection means 74 may detect the number of people present in the user region using any principle. For example, by arranging a camera for taking moving images or thermography at an entrance of the user region as the human count detection means 74 and subjecting a moving image taken by the camera or thermography to image processing, people entering the user region and people exiting the user region can be detected by detecting the presence or absence of people passing through the entrance of the user region and detecting a direction of movement of the people. When a person passes through the entrance and enters the user region, the human count detection means 74 increments the counted number of people by one. When a person passes through the entrance and exits the user region, the human count detection means 74 decrements the counted number of people by one.

When a single user region has a plurality of entrances, the human count detection means 74 detects the number of people present in the user region by detecting people entering the user region and people exiting the user region with respect to each entrance and adding up the number of people.

An installation site of the human count detection means 74 is not limited to an entrance of the user region. For example, the number of people present in the user region may be detected by arranging a camera or thermography on a ceiling or the like of the user region as the human count detection means 74 and processing images taken by the camera or thermography.

### Sixteenth embodiment

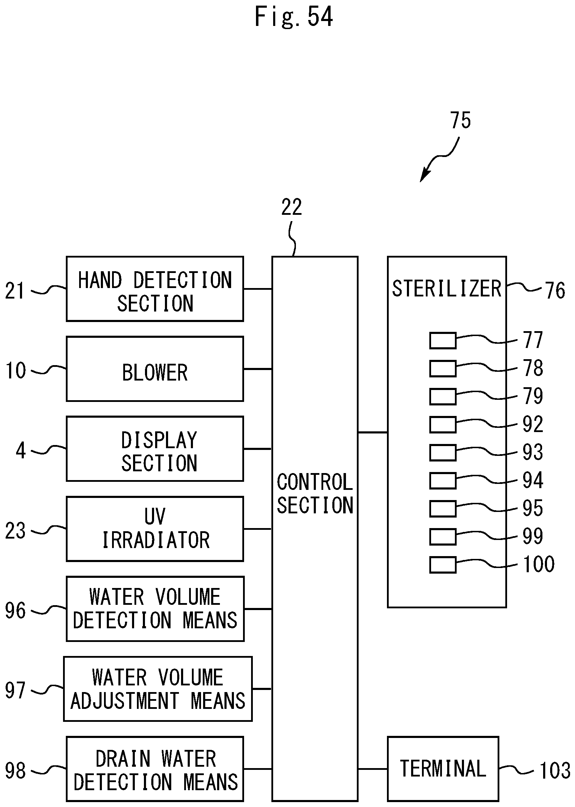

Next, while a sixteenth embodiment will be described with reference to Figs. 54 to 58, the description will focus on differences from the embodiments described above and common descriptions will be simplified or omitted. In addition, elements in common with or corresponding to elements described earlier will be denoted by same reference signs. Moreover, with respect to matters not described in the present embodiment, known art can be applied when deemed appropriate.

Fig. 54 is a functional block diagram of a hand-drying system 75 according to the sixteenth embodiment. As shown in Fig. 54, the hand-drying system 75 according to the sixteenth embodiment further includes a sterilizer 76. The sterilizer 76 may be arranged in a vicinity of the hand dryer. In other words, the sterilizer 76 may be arranged at a position that is slightly separated from the hand dryer. The sterilizer 76 may be integrally arranged with the hand dryer. For example, the sterilizer 76 may be arranged in the hand insertion section 3.

The sterilizer 76 is an apparatus which performs sterilization using a sterilizing liquid being a liquid that has a sterilization effect. In the present disclosure, the term "sterilizing liquid" can be paraphrased as "disinfectant" or "cleaning solution". As the sterilizing liquid, for example, alcohol, ions, hypochlorous acid, an hypochlorous acid aqueous solution, ozone, or electrolyzed water may be used. For example, the sterilizing liquid may be created by electrolyzing salt water, a salt solution, or a sodium chloride solution. The sterilizer 76 may use a plurality of types of sterilizing liquids.

The sterilizer 76 includes discharge means 77. The discharge means 77 discharges the sterilizing liquid. The discharge means 77 may discharge the sterilizing liquid as microscopic particles. The discharge means 77 may discharge the sterilizing liquid as atomized particles. The discharge means 77 may discharge the sterilizing liquid as a mist. The sterilizer 76 using a plurality of types of sterilizing liquids may be configured to be capable of switching among sterilizing liquids to be discharged from the discharge means 77.

The discharge means 77 may be arranged in a vicinity of the hand dryer, integrally arranged with the hand dryer, or arranged in the hand insertion section 3. The discharge means 77 may discharge the sterilizing liquid toward the hand insertion section 3. The discharge means 77 may discharge the sterilizing liquid toward the hand insertion space 5 formed by the hand insertion section 3. The discharge means 77 may discharge the sterilizing liquid toward a space of the user region in which the hand dryer is installed. The discharge means 77 may discharge the sterilizing liquid toward a hand of the user. The discharge means 77 may discharge the sterilizing liquid toward two or more of these targets.

The sterilizer 76 may include only one discharge means 77 or include a plurality of discharge means 77. The discharge means 77 discharges moisture. Specifically, the discharge means 77 discharges moisture by at least one means among dispersion, scattering, atomization, jetting, dripping, dispensing, emission, diffusion, conveyance, and blowing. Each of the discharge means 77 provided in plurality may discharge moisture or a sterilizing liquid by a mutually different method.

For example, the discharge means 77 may drip the sterilizing liquid so that the sterilizing liquid trickles down a face of the hand insertion section 3 where the discharge means 77 is provided. For example, the discharge means 77 may be provided in the insertion front section 3b and a liquid dripped from the discharge means 77 may move toward below the insertion front section 3b. In addition, the discharge means 77 may dispense the sterilizing liquid toward any of the faces of the hand insertion section 3 or toward the plurality of faces of the hand insertion section 3. Furthermore, the discharge means 77 may dispense the sterilizing liquid toward a hand arranged in the hand insertion section 3 or the hand insertion space 5. In addition, the discharge means 77 may atomize the sterilizing liquid toward a hand arranged in the hand insertion section 3 or the hand insertion space 5. Furthermore, the discharge means 77 may emit an atomized sterilizing liquid in a space where the hand insertion section 3 is arranged.

For example, a plurality of discharge means 77 may be arranged in the hand insertion section 3. The plurality of discharge means 77 may be arranged in the insertion front section 3b. One of the discharge means 77 may be arranged at a position on a left side of a center of the insertion front section 3b and another discharge means 77 may be arranged at a position on a right side of the center of the insertion front section 3b. One of the discharge means 77 may be arranged at a position at a center of a portion of a left half of the insertion front section 3b and another discharge means 77 may be arranged at a position at a center of a portion of a right half of the insertion front section 3b. When the user places both hands in the hand insertion space 5, one of the discharge means 77 may be arranged at a position opposing a center of a palm of the left hand in a front view and another discharge means 77 may be arranged at a position opposing a center of a palm of the right hand in a front view. Arranging the discharge means 77 as described above causes the sterilizing liquid discharged from the discharge means 77 to more readily adhere to the hand of the user when the user inserts the hand into the hand insertion space 5 of the hand insertion section 3. The discharge means 77 arranged at a position on the left side of the center of the insertion front section 3b and the discharge means 77 arranged at a position on the right side of the center of the insertion front section 3b are desirably bilaterally symmetrical with respect to the center of the insertion front section 3b.

In the discharge means 77, a plurality of micro-ports may be formed as a discharge port. Accordingly, the discharge means 77 can atomize fine particles in a similar manner to an atomizer. For example, the discharge means 77 may perform atomization over a wide range of a face that opposes a face of the hand insertion section 3 where the discharge means 77 is provided. For example, the discharge means 77 may be provided in the insertion front section 3b and fine moisture atomized from the discharge means 77 may adhere to the insertion rear section 3c.

A direction of atomization by the discharge means 77 is not limited to a direction from a face on which the discharge means 77 is provided toward a face opposing the face. For example, the direction of atomization by the discharge means 77 may be a direction from a face of the hand insertion section 3 on which the discharge means 77 is provided toward a face positioned to the side of the face. The direction of atomization by the discharge means 77 may be a direction from a face of the hand insertion section 3 on which the discharge means 77 is provided toward a face positioned below the face.

One of the discharge means 77 may be provided on one of two faces that oppose each other and another discharge means 77 may be provided on the other face. For example, one of the discharge means 77 may be arranged in the insertion front section 3b and another discharge means 77 may be arranged in the insertion rear section 3c. Arranging the discharge means 77 as described above enables a configuration to be adopted in which the sterilizing liquid discharged from the discharge means 77 arranged in the insertion front section 3b adheres to the insertion rear section 3c and the sterilizing liquid discharged from the discharge means 77 arranged in the insertion rear section 3c adheres to the insertion front section 3b. In other words, the sterilizing liquid can be caused to adhere to the hand insertion section 3 over a wide range.

The discharge means 77 may discharge a mist that is finer than that produced by an atomizer. The discharge means 77 may discharge ultrafine particles that drift in air such as a mist that is released from a humidifier or a nebulizer. For example, the discharge means 77 may discharge a mist or particles of the sterilizing liquid toward the hand insertion space 5. The discharge means 77 may discharge a mist or particles of the sterilizing liquid toward a space in which the hand dryer is arranged.

The hand-drying system 75 may use the control section 22 to perform control so that the atomized sterilizing liquid discharged from the discharge means 77 is conveyed together with an airflow generated by a fan or the like. For example, when the discharge means 77 atomizes and discharges the sterilizing liquid, the control section 22 may actuate the blower 10 so that the atomized sterilizing liquid is conveyed together with the generated airflow. Accordingly, the sterilizing liquid can be caused to reach a wider range.

The hand dryer may include a mist conveyance air path for blowing and conveying, using an airflow generated by the blower 10, the atomized sterilizing liquid discharged from the discharge means 77. The mist conveyance air path may include a mist conveyance switching section for switching between blowing air into the mist conveyance air path and not blowing air into the mist conveyance air path when the blower 10 is driven. As the mist conveyance switching section, for example, a solenoid valve may be used. The mist conveyance switching section switches between an open state where air flows through the mist conveyance air path and a closed state where air does not flow through the mist conveyance air path when the blower 10 is actuated. For example, when drying a hand, the control section 22 changes the mist conveyance switching section to the closed state. When conveying the atomized sterilizing liquid discharged from the discharge means 77, the control section 22 changes the mist conveyance switching section to the open state. Accordingly, when drying a hand, a drop in air speed from the air hole 6 can be avoided.

Next, a timing of sterilization by the sterilizer 76 will be described. The hand-drying system 75 may perform sterilization by the sterilizer 76 in coordination with a detection by the hand detection section 21. In other words, the control section 22 may perform control so that the sterilizing liquid is discharged from the discharge means 77 when a hand is inserted into the hand insertion space 5 and the hand detection section 21 detects the hand. A step of discharging the sterilizing liquid from the discharge means 77 so that the sterilizing liquid discharged from the discharge means 77 adheres to the hand will be referred to as a sterilizing step. The hand is sterilized as the discharged sterilizing liquid adheres to the hand according to the sterilizing step. Note that, in the present disclosure, when a hand is not sterilized, sterilizing a periphery of the hand insertion section 3 or a periphery of the hand dryer may be referred to as a sterilizing step. In other words, discharging the sterilizing liquid by the discharge means 77 may be referred to as a sterilizing step.

The control section 22 operates the blower 10 when a hand is inserted into the hand insertion space 5 and the hand detection section 21 detects the hand. In other words, operation of the blower 10 is switched on when the hand detection section 21 detects a hand. This step may be referred to as a drying step. According to the drying step, the hand inserted into the hand insertion space 5 is dried.

As a first example, the hand-drying system 75 may perform the sterilizing step and the drying step in this order. In other words, when the hand detection section 21 detects a hand, the hand-drying system 75 may cause the discharge means 77 to discharge the sterilizing liquid before operation of the blower 10 is switched on. In addition, the hand-drying system 75 may start the drying step after the sterilizing step starts and before the sterilizing step ends. The hand-drying system 75 may start the drying step after the sterilizing step ends. The hand-drying system 75 may perform the drying step once a predetermined amount of time elapses after the end of the sterilizing step.

The hand-drying system 75 may switch on operation of the blower 10 immediately after the discharge means 77 discharges the sterilizing liquid. The hand-drying system 75 more desirably provides a time lag of a predetermined amount of time between a time at which the discharge means 77 discharges the sterilizing liquid and a time at which operation of the blower 10 is switched on. For example, the hand-drying system 75 desirably switches on operation of the blower 10 once several seconds elapse after the sterilizing liquid is discharged from the discharge means 77. Accordingly, the following advantageous effects are produced. When the sterilizing liquid is discharged from the discharge means 77 as the user inserts a hand into the hand insertion space 5, the sterilizing liquid adheres to the user's hand. The user rubs both hands together so that the hands become coated by the sterilizing liquid. In this manner, the entire hands can be more reliably sterilized. When the blower 10 is switched on and blows dry air in a state where the hands have been sterilized, the number of microorganisms contained in water droplets scattered from the hands decrease. Therefore, even when water droplets are scattered to the hand insertion section 3 or water droplets are scattered to a space where the hand dryer is installed, a risk of infection can be reduced. As a result, hygiene improves. The sterilization effect by the sterilizing liquid may decline in a state where moisture adheres to a hand. Therefore, a sterilizing liquid with its concentration enhanced in advance may be used.

As a second example, the hand-drying system 75 may perform the drying step and the sterilizing step in this order. For example, the hand-drying system 75 may start the sterilizing step after the drying step starts and before the drying step ends. The hand-drying system 75 may start the sterilizing step after the drying step ends. After hand drying is performed by an operation of the blower 10, the hand-drying system 75 may spray the sterilizing liquid discharged from the discharge means 77. For example, the hand-drying system 75 may discharge the sterilizing liquid from the discharge means 77 immediately after the end of the drying step, immediately before the drying step ends, or during the drying step. The sterilization effect by the sterilizing liquid may decline in a state where moisture adheres to a hand. Therefore, by discharging the sterilizing liquid after the drying air is applied to a hand and the hand is dried, the sterilization effect can be more reliably prevented from declining.

As a third example, the hand-drying system 75 may perform a first sterilizing step, the drying step, and a second sterilizing step in this order. As described above, in the event that hand washing is insufficient when drying a hand that has become wet due to the hand washing, there is a possibility that microorganisms adhering to the hand may be scattered in the hand dryer or scattered in a space where the hand dryer is installed together with water droplets. Therefore, the hand is desirably sterilized before drying. On the other hand, discharging a sterilizing liquid to a wet hand may result in diluting the sterilizing liquid and causing a decline in a sterilization effect. In consideration thereof, after sterilizing a hand with a sterilizing liquid in a first sterilizing step, the hand may be dried using the blower 10 in the drying step, and the hand may be further sterilized with the sterilizing liquid in a second sterilizing step. In this case, an amount of microorganisms which is scattered in the hand dryer together with water droplets or an amount of microorganisms which is scattered in the space where the hand dryer is installed together with water droplets can be more reliably reduced and hygiene is improved. In addition, by having the sterilizing liquid discharged to the hand once again in the second sterilizing step after drying the hand in the drying step, the hand can be more reliably sterilized. As a result, hygiene further improves. Furthermore, while the hand-drying system 75 may switch on the operation of the blower 10 immediately after the sterilizing liquid is discharged in the first sterilizing step, the hand-drying system 75 more desirably provides a time lag of a predetermined amount of time between a time at which the sterilizing liquid is discharged in the first sterilizing step and a time at which operation of the blower 10 is switched on in the drying step. For example, the hand-drying system 75 desirably switches on operation of the blower 10 once several seconds elapse after the sterilizing liquid is discharged from the discharge means 77 in the first sterilizing step.

As a fourth example, the hand-drying system 75 may perform a first sterilizing step, a first drying step, a second sterilizing step, and a second drying step in this order. By performing the second drying step following the second sterilizing step, a hand of the user can be dried. In other words, since the hand no longer remains wet due to the sterilizing liquid, a comfort level of the user increases. The hand-drying system 75 may start the second drying step after the second sterilizing step starts and before the second sterilizing step ends. The hand-drying system 75 may start the second drying step after the second sterilizing step ends. The hand-drying system 75 may perform the second drying step once a predetermined amount of time elapses after the end of the second sterilizing step. By performing the steps as described above, due to the hand being dried by the first drying step in a state where the hand has been sterilized in the first sterilizing step and the hand being further sterilized in the second sterilizing step, microorganisms adhering to the hand further decrease and the hand is further dried by the second drying step. As a result, hygiene of the hand dryer and hygiene of the space in which the hand dryer is installed are further improved, and the hand is placed in a hygienic and dried state.

The hand-drying system 75 may include a sterilization notification section which notifies that a sterilizing step is in progress. For example, the display section 4 may correspond to the sterilization notification section. By having the sterilization notification section notify that the sterilizing step is in progress, the user becomes cognizant of the fact that sterilization by a sterilizing liquid is being performed and gains a sense of security.

In addition, the hand-drying system 75 may include a drying notification section which notifies that a drying step is in progress. For example, the display section 4 may correspond to the drying notification section.

In addition, the hand-drying system 75 may include a step notification section. For example, the display section 4 may correspond to the step notification section. In addition to notifying that the sterilizing step is in progress and notifying that the drying step is in progress, the step notification section notifies about all of the steps and notifies which of all of the steps the present step corresponds to. For example, when sterilization and drying is performed by the three steps of a first sterilizing step, a subsequent drying step, and a second sterilizing step, the step notification section may notify about the three steps by displaying a text or an illustration or notify which of the three steps the present step corresponds to by a light such as a lamp. The hand-drying system 75 may include a monitor or a display arranged in the hand dryer or a monitor or a display arranged in a space in which the hand dryer is arranged, and display the text or the illustration described above on the monitor or the display. Providing the step notification section enables the user to readily understand sterilization and drying steps and enables the user to use the hand dryer with a greater sense of security.

When the hand detection section 21 detects that a hand is not arranged in the hand insertion section 3 in any of the steps described above, the hand-drying system 75 may forcibly terminate the steps described above. In the case of forcible termination, the hand-drying system 75 ends a step regardless of whether the step is a drying step or a sterilizing step and also does not perform next and subsequent steps. Accordingly, wasteful operations can be avoided.

The hand-drying system 75 may include human detection means. The human detection means of the hand-drying system 75 may be configured in the same or similar manner to the human detection section 39, the presence/absence detection means 65, or the proximity detection means 70 described earlier.

The hand-drying system 75 may cause the discharge means 77 to discharge the sterilizing liquid when the hand detection section 21 is not detecting a hand. The hand-drying system 75 may cause the discharge means 77 to discharge the sterilizing liquid when the human detection means is not detecting a person. For example, the hand-drying system 75 may cause the discharge means 77 to discharge the sterilizing liquid after the user uses the hand dryer. Accordingly, the hand insertion section 3 after use can be sterilized and the space in which the hand dryer is arranged can be sterilized. In addition, when a person inhales a sterilizing liquid with a particle size small enough to drift in air while breathing, the sterilizing liquid may have an adverse effect on the human body. In this regard, by discharging a sterilizing liquid by the discharge means 77 when the hand detection section 21 is not detecting a hand or discharging a sterilizing liquid by the discharge means 77 when the human detection means is not detecting a person, safety with respect to the human body can be further enhanced.

In anticipation of the manager cleaning the hand dryer or the user region, the hand-drying system 75 may be configured to be capable of manually operating on/off states of discharge by the discharge means 77 of the sterilizer 76. For example, the hand-drying system 75 may include one or both detection switching means of detection switching means for switching between on/off states of the sensing function of the hand detection section 21 and detection switching means for switching between on/off states of the sensing function of the human detection means. When the sensing function of the hand detection section 21 is switched off by the detection switching means, the hand detection section 21 does not react even when a hand is inserted into the hand insertion space 5 of the hand insertion section 3. When the sensing function of the human detection means is switched off by the detection switching means, the human detection means does not react even when a person approaches the hand dryer. Accordingly, since an actuation of a drying step or a sterilizing step is to be prevented from starting unexpectedly when the manager is cleaning the hand dryer or the user region, wasteful operations can be avoided.

In addition, the hand-drying system 75 may include a continuous operation switching section of the discharge means 77 of the sterilizer 76. The hand-drying system 75 may be configured such that, when the continuous operation switching section is switched on, the discharge means 77 is continuously operated, and when the continuous operation switching section is switched off, a sterilizing liquid is not discharged from the discharge means 77. When continuous operation of the discharge means 77 is executed, the sterilizing liquid is continuously discharged from the discharge means 77. The continuous operation switching section is desirably switched off with the exception of during cleaning of the hand dryer or the user region by the manager. When the manager cleans the hand dryer or the user region, by switching on the continuous operation switching section, the sterilizing liquid discharged from the discharge means 77 can be used for cleaning when necessary. In other words, when desiring to use the sterilizing liquid during cleaning, the continuous operation switching section may be temporarily switched on. Accordingly, the person responsible for cleaning is freed from the burden of separately preparing a sterilizing liquid and, at the same time, cleaning can be readily performed using the sterilizing liquid discharged from the discharge means 77. Note that the hand-drying system 75 including the continuous operation switching section need not necessarily include the detection switching means described above. Even in this case, by keeping the continuous operation switching section switched on, since the sterilizing liquid is continuously discharged from the discharge means 77 regardless of a detection result of the hand detection section 21 or the human detection means, the sterilizing liquid can be used for cleaning.

The sterilizer 76 may include a storage for storing an aqueous chloride solution. The sterilizer 76 may include: electrolyzed water generation means 92 for electrolyzing the aqueous solution stored in the storage and generating electrolyzed water; and a first discharge section for discharging the electrolyzed water generated by the electrolyzed water generation means 92 to the outside of the sterilizer 76. The first discharge section is an example of the discharge means 77. Note that electrolyzed water corresponds to a sterilizing liquid. In the present disclosure, the description "sterilizing liquid" may be used when including liquids other than electrolyzed water that produce a sterilization effect.

For example, the electrolyzed water generation means 92 may generate electrolyzed water having liquid sodium hypochlorite dissolved therein by electrolyzing an aqueous chloride solution. Liquid sodium hypochlorite is also called "Hypochlorous Acid". A composition formula of liquid sodium hypochlorite is, for example, "HClO". The electrolyzed water generation means 92 includes electrodes including a positive electrode and a negative electrode. The electrodes are provided in the storage.

The electrodes of the electrolyzed water generation means 92 are desirably made of a material that is hard to dissolve in water. Examples of such a material include titanium and platinum. In order to promote electrolysis, a metal of the platinum group such as iridium, platinum, or ruthenium or an oxide thereof may be supported on the electrodes. A chemical species such as hydrogen peroxide, active oxygen, or OH radical may be generated in electrolyzed water.

The electrolyzed water generation means 92 may be a one-chamber type without a partition between the positive electrode and the negative electrode or a two-chamber type, a three-chamber type, or a multi-chamber type with a partition between the positive electrode and the negative electrode. The one-chamber type electrolyzed water generation means 92 neutralizes acidic ion water generated on a side of the positive electrode and alkali ion water generated on a side of the negative electrode to generate electrolyzed water containing liquid sodium hypochlorite with an appropriate concentration. The multi-chamber type electrolyzed water generation means 92 generates acidic ion water in a chamber housing the positive electrode and generates alkali ion water in a chamber housing the negative electrode.

In the multi-chamber type electrolyzed water generation means 92, there is a possibility that an amount of use of the acidic ion water and an amount of use of the alkali ion water may become imbalanced. In such a case, it may be necessary to dispose the ion water of whichever remains. By comparison, with the one-chamber type electrolyzed water generation means 92, since there is no need to dispose the ion water of whichever remains, convenience is improved.

The storage is a container capable of housing water and chloride. The water housed in the storage may be clean water or tap water. The chloride housed in the storage is, for example, at least one of sodium chloride (NaCl), potassium chloride, magnesium chloride, and calcium chloride. The chloride may be supplied to the storage as a solid or in a solid state such as a powder, granules, pills, tablets, and the like. The chloride may be in liquid form or gel form. In order to enhance convenience of supplying water to the storage and convenience of supplying chloride to the storage, the storage may be configured to be detachable from the sterilizer 76. The storage may include an openable/closeable cover. By opening the cover or detaching the cover of the storage, water or chloride can be readily added to the inside of the storage. When the storage does not have a cover, water or chloride can be added to the inside of the storage from an opening of the storage. An opening may be provided in the cover of the storage and water or chloride may be added from the opening. A storage with a cover reduces spillage of water when carried around. The storage may be provided with at least one detection means among liquid volume detection means 93 for detecting an amount of liquid in the storage, concentration detection means 94 for detecting a concentration of chloride dissolved in an aqueous solution in the storage, and pH detection means 95 for detecting a pH of an aqueous solution in the storage.

The liquid volume detection means 93 may be a contact type or a non-contact type. Examples of a contact-type liquid volume detection means 93 include a float type and a capacitive type. A float-type liquid volume detection means 93 detects a liquid level based on a position in a vertical direction of a float provided inside the storage. A capacitive liquid volume detection means 93 detects a liquid level by detecting a capacitance between a pair of electrodes. Examples of a non-contact liquid volume detection means 93 include those that detect a liquid level using radio waves, ultrasonic waves, or light waves.

Electrodes of the electrolyzed water generation means 92 may double as the liquid volume detection means 93. With electrodes that extend in the vertical direction, a ratio between a portion submerged in liquid and a portion exposed to gas inside the storage changes in accordance with a change in the liquid level inside the storage or, in other words, the liquid level of electrolyzed water. When the ratio changes, a value of a current that flows between the positive electrode and the negative electrode of the electrode changes. Therefore, by detecting a change in the value of a current that flows between the positive electrode and the negative electrode of the electrode, an amount of liquid stored in the storage can be detected. Alternatively, the liquid volume detection means 93 may detect an amount of liquid stored in the storage based on, for example, a weight of the storage. For example, the liquid volume detection means 93 may detect the amount of liquid stored in the storage based on an increased mass relative to a weight of an empty storage.

For example, the concentration detection means 94 may be a refractometer which measures a concentration of chloride in an aqueous solution using a refractive phenomenon of light or an electrical conductivity meter which measures a concentration of chloride in an aqueous solution based on a relationship between a concentration of chloride in an aqueous solution and an electrical conductivity of the aqueous solution. Alternatively, the concentration detection means 94 may measure chlorine concentration using, for example, absorption photometry. In addition, the concentration detection means 94 may estimate chlorine concentration based on other information such as turbidity.

The pH detection means 95 may use, for example, a glass electrode. The pH detection means 95 may use, for example, a glass electrode method. According to the pH detection means 95, pH of an aqueous solution in the storage can be detected within a range of 0 to 14 or a narrower range.

The electrolyzed water generation means 92 may be provided in the storage. The electrolyzed water generation means 92 may be provided outside of the storage. For example, the electrolyzed water generation means 92 may be provided in a liquid passage that connects to the first discharge section. The electrolyzed water generation means 92 may generate electrolyzed water before the aqueous solution stored in the storage is discharged to the outside of the sterilizer 76. In the present disclosure, as a general rule, a "passage" corresponds to a pathway which a solid or a liquid passes through.

When a liquid used by the sterilizer 76 is a liquid that does not require electrolysis, the sterilizer 76 need not include the electrolyzed water generation means 92. An example of a liquid that does not require electrolysis is alcohol. In addition, the manager may directly add a sterilizing liquid to the storage.

A configuration may be adopted such that water is supplied to the sterilizer 76 from a water supply. In other words, the hand dryer may be coupled to a water supply. For example, the storage may be connected to a water supply via an automatic on-off valve. Accordingly, the storage is automatically refilled with water from the water supply. The storage may be automatically supplied with water from the water supply when the liquid volume detection means 93 detects a drop in the liquid level inside the storage so as to restore a predetermined liquid level. The storage may be automatically supplied with water from the water supply when the concentration detection means 94 detects a rise in concentration of the aqueous solution inside the storage so that the concentration drops to a predetermined concentration.

The discharge means 77 may include at least one discharge section among a first discharge section, a second discharge section, and a third discharge section. Each of the first discharge section, the second discharge section, and the third discharge section may include a mechanism section for discharging a sterilizing liquid. The first discharge section discharges the sterilizing liquid from the storage to a surface of the hand insertion section 3 or a wall surface that defines the hand insertion space 5. The second discharge section discharges the sterilizing liquid from the storage to a hand of a user. The third discharge section discharges the sterilizing liquid from the storage to the space in which the hand dryer is installed.

The sterilizing liquid discharged from the discharge means 77 may be exposed to the outer appearance of the hand dryer. The sterilizing liquid discharged from the discharge means 77 may come into contact with outside air. The sterilizing liquid discharged from the discharge means 77 may be exposed at a position that can come into contact with a person.

The first discharge section may include a first discharge port that discharges a sterilizing liquid to the hand insertion space 5, a first on-off valve, and a first liquid passage that guides the sterilizing liquid to the first discharge port. When the first on-off valve opens, the sterilizing liquid is discharged to the first discharge port. When the first on-off valve closes, discharge of the sterilizing liquid to the first discharge port stops.

The first discharge section may include the first discharge port in plurality. For example, the plurality of first discharge ports may be lined up in a row in the left-right direction or, in other words, the width direction of the hand insertion section 3. According to the plurality of first discharge ports lined up in this manner, a wide range of the hand insertion section 3 can be moistened by the sterilizing liquid. Alternatively, the first discharge port may be an elongated opening that extends in the left-right direction or, in other words, the width direction of the hand insertion section 3. In addition, the first discharge port may be configured to appear circular as a whole due to a plurality of micro-ports being arranged in proximity to each other.

Typically, the first on-off valve is made of a solenoid valve. By opening the first on-off valve, the first discharge section may discharge the sterilizing liquid using a difference in height between a liquid level of the sterilizing liquid in the storage and the first discharge port or, in other words, a water head difference. The first discharge section may include a pump that pumps the sterilizing liquid in the storage instead of the first on-off valve. The first discharge port is preferably configured such that a group of particles of the liquid discharged from the first discharge port contains microparticles of which a particle size is equal to or smaller than 300 µm in diameter. In addition, the first discharge section may simply be a flow path that allows the sterilizing liquid in the storage to flow out. For example, the first discharge section may be a simple tubule or an orifice. In such a case, an inner diameter of the tubule or a diameter of the orifice of the first discharge port may be set such that an amount of discharge of the sterilizing liquid per unit time equals a required value.

The second discharge section serves a function of discharging a sterilizing liquid to a hand of a user. The second discharge section may include a second discharge port that discharges the sterilizing liquid to a hand of the user, a second on-off valve that performs discharge and blocking of discharge of the sterilizing liquid to the second discharge port, and a second liquid passage that guides the sterilizing liquid to the second discharge port.

For example, the second discharge port may be a nozzle capable of atomizing the sterilizing liquid. The second discharge section may include the second discharge port in plurality. For example, the plurality of second discharge ports may be arranged in the insertion front section 3b. One of the second discharge ports may be arranged at a position on a left side of a center of the insertion front section 3b and another second discharge port may be arranged at a position on a right side of the center of the insertion front section 3b. One of the second discharge ports may be arranged at a position at a center of a portion of a left half of the insertion front section 3b and another second discharge port may be arranged at a position at a center of a portion of a right half of the insertion front section 3b. When the user places both hands in the hand insertion space 5, one of the second discharge ports may be arranged at a position opposing a center of a palm of the left hand in a front view and another second discharge port may be arranged at a position opposing a center of a palm of the right hand in a front view. Arranging the second discharge ports as described above causes the sterilizing liquid discharged from the second discharge ports to more readily adhere to the hand of the user when the user inserts the hand into the hand insertion space 5 of the hand insertion section 3.

Typically, the second on-off valve is made of a solenoid valve. By opening the second on-off valve, the second discharge section may discharge the sterilizing liquid using a difference in height between a liquid level of the sterilizing liquid in the storage and the second discharge port or, in other words, a water head difference. The second discharge section may include a pump that pumps the sterilizing liquid in the storage instead of the second on-off valve. In addition, the second discharge section may simply be a flow path that allows the sterilizing liquid in the storage to flow out. For example, the second discharge section may be a simple tubule or an orifice. In such a case, an inner diameter of the tubule or a diameter of the orifice of the second discharge port may be set such that an amount of discharge of the sterilizing liquid per unit time equals a required value.

Instead of including the second discharge section, the discharge means 77 may include a first discharge section that also has the function of the second discharge section. In other words, the first discharge section may be capable of discharging the sterilizing liquid to the hand insertion section 3 and the first discharge section may also be capable of discharging the sterilizing liquid to a hand of the user. For example, the first discharge section may be structured such that a plurality of fine first discharge ports are provided in proximity to each other. The first discharge section provided on a face of the hand insertion section 3 may be capable of discharging the sterilizing liquid toward a face opposing the face of the hand insertion section 3. The first discharge section may be configured such that the sterilizing liquid is radially discharged from the first discharge section.

The plurality of first discharge section may be arranged in the insertion front section 3b. One of the first discharge sections may be arranged at a position on a left side of a center of the insertion front section 3b and another first discharge section may be arranged at a position on a right side of the center of the insertion front section 3b. One of the first discharge sections may be arranged at a position at a center of a portion of a left half of the insertion front section 3b and another first discharge section may be arranged at a position at a center of a portion of a right half of the insertion front section 3b. The first discharge section arranged at a position on the left side of the center of the insertion front section 3b and the first discharge section arranged at a position on the right side of the center of the insertion front section 3b are desirably bilaterally symmetrical with respect to the center of the insertion front section 3b.

A hand of the user is arranged at a position closer to a face of the insertion rear section 3c that opposes the insertion front section 3b as viewed from a face of the insertion front section 3b. Therefore, particles immediate after being radiated from the first discharge section strikes the hand before being fully diffused. Therefore, a large amount of the radiated sterilizing liquid can be caused to strike the hand. By comparison, when the sterilizing liquid radiated from the first discharge section strikes the face of the insertion rear section 3c, the sterilizing liquid is already diffused since the face of the insertion rear section 3c is farther than the position of the hand. Therefore, the sterilizing liquid adheres to a wide range of the insertion rear section 3c. According to such a first discharge section, the sterilizing liquid can be discharged to both the hand of the user and the hand insertion section 3. Therefore, hygiene can be made more preferable with a simple configuration.

The third discharge section serves a function of scattering a sterilizing liquid to at least one space of the hand insertion space 5 and the space in which the hand dryer is installed. The third discharge section may include an atomization device that atomizes the sterilizing liquid and discharges the atomized sterilizing liquid to the space and a third liquid passage that guides the sterilizing liquid to the atomization device.

The atomization device diffuses or scatters the atomized sterilizing liquid to the space. The atomization device may be a heating type which atomizes the sterilizing liquid by heating the sterilizing liquid, an ultrasound type which atomizes the sterilizing liquid by ultrasonically vibrating the sterilizing liquid, a spray which uses a Venturi effect, a system which atomizes the sterilizing liquid using an atomizer, an electrostatic atomization system which atomizes the sterilizing liquid using a corona discharge, or a fragmentation type which fragments droplets by diffusing the sterilizing liquid with a propeller or the like rotating at high speed. Regardless of the atomization method used, the atomization device preferably atomizes the sterilizing liquid so as to generate a group of particles including microparticles with a diameter of 100 µm or smaller and more preferably atomizes the sterilizing liquid so as to generate a group of particles including microparticles with a diameter of 10 µm or smaller.

When the discharge means 77 discharges a sterilizing liquid to a hand, a sterilizing liquid with a concentration which is less harmful even when touched by a person and which can be used safely is desirably discharged. When the discharge means 77 sprays an atomized sterilizing liquid to a space, a sterilizing liquid with a concentration which is less harmful even when inhaled by a person and which can be used safely is desirably sprayed.

There is a small possibility of adverse effects when a person inhales fine particles discharged from the second discharge section or the third discharge section. In such a case, the hand insertion section 3 may be provided with the shielding body, the cover section, or the lid described earlier in the hand-drying system 75 and a sterilizing liquid may be discharged from the second discharge section or the third discharge section when the shielding body, the cover section, or the lid is in the closed state (the second state). Accordingly, sterilization can be performed, albeit limited to the inside of the hand insertion section 3. Subsequently, after a predetermined amount of time elapses from the discharge of the sterilizing liquid, the hand-drying system 75 may cause the shielding body, the cover section, or the lid to be automatically restored to the open state (the first state).

Each of the first liquid passage, the second liquid passage, and the third liquid passage is made of, for example, piping. The first liquid passage, the second liquid passage, and the third liquid passage may be branched from another liquid passage or each may be independently connected to the storage.

The storage is capable of accepting water and chloride. The storage stores an aqueous chloride solution. The sterilizer 76 may include at least one acceptance port of a first acceptance port for accepting water or another liquid and a second acceptance port for accepting chloride. The first acceptance port and the second acceptance port may be the same. In other words, an acceptance port may double as the first acceptance port and the second acceptance port. The first acceptance port and the second acceptance port may not be openings.

The hand-drying system 75 may include a liquid passage for sending a liquid accepted from the first acceptance port to the storage. The liquid passage is connected to the storage. An opening of the liquid passage may be the first acceptance port. The hand-drying system 75 may not include the liquid passage. In such a case, the first acceptance port is to be provided in the storage.

In a case where the sterilizer 76 is configured to include a storage with an open upper part and a liquid is to be added to the storage from the upper part, the upper part of the storage may be interpreted as the first acceptance port or addition of a liquid may be interpreted as being performed from above due to an absence of the first acceptance port. In this manner, the first acceptance port is not necessarily strictly defined. The sterilizer 76 need not necessarily include the first acceptance port.

The first acceptance port may be an opening for accepting water such as tap water for generating electrolyzed water. The sterilizer 76 may accept liquids other than water from the first acceptance port. The sterilizer 76 may accept a liquid with a low sterilization effect or a liquid without a sterilization effect from the first acceptance port. The sterilizer 76 may accept a liquid with a low sterilization effect or a liquid without a sterilization effect from the first acceptance port and generate a liquid with a sterilization effect using the electrolyzed water generation means 92. The sterilizer 76 may accept a liquid that is salt water such as a salt solution or a sodium chloride solution from the first acceptance port. When accepting salt water from the first acceptance port, the sterilizer 76 may generate a hypochlorous acid aqueous solution by electrolyzing the salt water. In this case, there is no need for the sterilizer 76 to accept anything from the second acceptance port for chloride. The sterilizer 76 may accept salt water from the second acceptance port for chloride instead of the first acceptance port. In this case, there is no need for the sterilizer 76 to accept anything from the first acceptance port.

The first acceptance port is preferably arranged at a position of a same height as a top face of the storage or arranged at a position that is higher than the top face of the storage. Accordingly, a liquid accepted from the first acceptance port flows to the storage due to gravity. In addition, the first acceptance port may be arranged on the top face of the storage.

The second acceptance port for accepting chloride to the storage may be provided in the storage. The second acceptance port is preferably arranged at a position of a same height as the top face of the storage or arranged at a position that is higher than the top face of the storage. The second acceptance port may double as an opening for accepting water such as tap water for generating electrolyzed water besides chloride. The manager may add water to the storage from the first acceptance port and add chloride to the storage from the second acceptance port. The manager may add an aqueous solution in which chloride is dissolved in water to the storage from the first acceptance port or the second acceptance port.

When the storage is a multi-chamber type, the first acceptance port and the second acceptance port are preferably each connected to a different chamber.

The hand-drying system 75 may include a tank for temporarily storing water. The hand-drying system 75 may store water accepted from the first acceptance port in the tank. The tank may be provided with the first acceptance port. For example, the first acceptance port may be provided on a top face of the tank. The tank is desirably arranged at a position that is higher than the storage.

The hand-drying system 75 may include a water intake passage that connects the first acceptance port to the tank. For example, the water intake passage is formed of piping. Water that flows in from the first acceptance port passes through the water intake passage and accumulates in the tank. When the hand-drying system 75 includes a tank, a liquid passage connects the tank to the storage. Water that flows in from the first acceptance port passes through the water intake passage, the tank, and the liquid passage in this order and flows into the storage. The liquid passage may be provided on a lower side of the tank. The liquid passage may be provided on a bottom face of the tank.

The hand-drying system 75 may further include water volume detection means 96 for detecting a water volume in the tank and water volume adjustment means 97 for adjusting the water volume in the tank. For example, the water volume detection means 96 may be provided in the tank. The water volume detection means 96 may detect a water volume according to any of the detection methods described with respect to the liquid volume detection means 93 described earlier. The water volume adjustment means 97 may be means for switching between discharging water from the tank to the storage and stopping discharge of water from the tank to the storage. For example, the water volume adjustment means 97 may be provided in one of or both of the tank and the liquid passage. For example, the water volume adjustment means 97 may use a solenoid valve or a pump. As the solenoid valve or the pump, a solenoid valve or a pump using known art or the mechanism described above may be applied.

By using at least two of the concentration detection means 94, the water volume detection means 96, and the water volume adjustment means 97 in combination, the hand-drying system 75 move a desired amount of water to the storage. For example, the hand-drying system 75 can control a water volume to be moved with high accuracy by moving water to the storage using the water volume adjustment means 97 while detecting a water volume using the water volume detection means 96. In addition, the hand-drying system 75 can control a concentration of an aqueous solution in the storage with high accuracy by moving water to the storage using the water volume adjustment means 97 while detecting a concentration of the aqueous solution in the storage using the concentration detection means 94. The hand-drying system 75 may move water in the tank to the storage using all of the concentration detection means 94, the water volume detection means 96, and the water volume adjustment means 97. Accordingly, water can be moved with even higher accuracy. In this manner, by moving the water in the tank to the storage with high accuracy, the concentration or pH of the aqueous solution in the storage can be more appropriately managed.

The sterilizer 76 may include a chloride supplying section 78. The chloride supplying section 78 is for supplying chloride to the storage. The chloride supplying section 78 may be provided as a part of the storage. The chloride supplying section 78 may include a second acceptance port, a first supply path, a second supply path, and a chloride storage section 79. The chloride supplying section 78 may include only the second acceptance port. The first supply path is a pathway for moving chloride from the second acceptance port to the chloride storage section 79. The second supply path is a pathway for moving chloride from the chloride storage section 79 to the storage. The chloride storage section 79 is for temporarily storing chloride accepted from the second acceptance port. The chloride storage section 79 is connected to the storage. For example, the chloride placed in the second acceptance port passes through the first supply path, the chloride storage section 79, and the second supply path in this order and is supplied to the storage.

Note that the first supply path need not be included when the second acceptance port is provided in the chloride storage section 79. In a case where the chloride supplying section 78 is configured to include the chloride storage section 79 with an open upper part and chloride is to be added to the chloride storage section 79 from the upper part, the upper part of the chloride storage section 79 may be interpreted as the second acceptance port or addition of chloride to the chloride storage section 79 may be interpreted as being performed from above due to an absence of the second acceptance port. In this manner, the second acceptance port is not necessarily strictly defined. The sterilizer 76 need not necessarily include the second acceptance port.

In a state where the second acceptance port is installed in the hand-drying system 75, the second acceptance port may be covered by any portion of the hand-drying system 75. Accordingly, since foreign objects less readily penetrate into the second acceptance port, penetration into the storage by foreign objects can be advantageously prevented.

The chloride supplying section 78 may not include the chloride storage section 79. In such a case, the chloride placed in the second acceptance port passes through the second supply path and is supplied to the storage. In addition, the second supply path need not be included when the second acceptance port is provided in the storage.

The hand-drying system 75 may have a structure that enables the second acceptance port to be extracted or retrieved with respect to the sterilizer 76. Alternatively, the hand-drying system 75 may have a structure that enables at least a part of the chloride supplying section 78 including the second acceptance port to be extracted or retrieved with respect to the sterilizer 76. According to such a structure, by extracting or retrieving the second acceptance port or at least a part of the chloride supplying section 78 including the second acceptance port, work can be performed more readily when placing chloride in the second acceptance port.

The chloride storage section 79 is capable of storing a certain number of or a certain amount of chloride. The chloride storage section 79 may have a mechanism for automatically discharging chloride. The sterilizer 76 may include chloride adjustment means 99 for adjusting an amount of chloride in the chloride storage section 79. The chloride adjustment means 99 may convey a certain amount of chloride stored in the chloride storage section 79 to the storage. For example, the chloride adjustment means 99 may convey a certain amount of chloride from the chloride storage section 79 to the storage when an amount of a sterilizing liquid in the storage is small. For example, the chloride adjustment means 99 may convey a certain amount of chloride from the chloride storage section 79 to the storage when a concentration of the sterilizing liquid in the storage is lower than a predetermined value. For example, the chloride adjustment means 99 may convey a certain amount of chloride from the chloride storage section 79 to the storage when pH of the sterilizing liquid in the storage is higher than a predetermined value.

The chloride storage section 79 may store granulated chloride. Hereinafter, an example of the chloride adjustment means 99 in a case where a grain of chloride is small will be described. A case where a grain of chloride is small refers to a case where, for example, a length in a direction in which a diameter of a grain is maximized is less than 5 mm, more preferably 3 mm or less, and even more preferably 1 mm or less. In the case of such a small grain, the chloride adjustment means 99 collectively supplies a plurality of grains of chloride to the storage. For example, the chloride adjustment means 99 may include an on-off valve and chloride volume detection means 100. Typically, the on-off valve is made of a solenoid valve. When the on-off valve is opened, chloride is conveyed from the chloride storage section 79 to the storage due to a difference in height.

The chloride volume detection means 100 may detect an amount of chloride in the chloride storage section 79. The chloride volume detection means 100 may detect an amount of chloride conveyed from the chloride storage section 79 to the storage. The chloride volume detection means 100 may detect an amount of chloride conveyed from the chloride storage section 79 to the storage based on a change in weight of the chloride storage section 79 including chloride. The hand-drying system 75 controls opening/closing operations of the on-off valve based on a detection result of the chloride volume detection means 100. Note that the hand-drying system 75 may adjust the on-off valve in accordance with a detection result of the concentration detection means 94 provided in the storage in place of the chloride volume detection means 100.

Hereinafter, an example of the chloride adjustment means 99 in a case where a grain of chloride is large will be described. A case where a grain of chloride is large refers to a case where, for example, a length in a direction in which a diameter of a grain is maximized is 5 mm or more. In the case of such a large grain, the chloride adjustment means 99 preferably supplies grains of chloride to the storage one grain at a time. Note that, in the present disclosure, the chloride adjustment means 99 may collectively supply a plurality of grains of chloride to the storage even when the length in a direction in which a diameter of a grain is maximized is 5 mm or more.

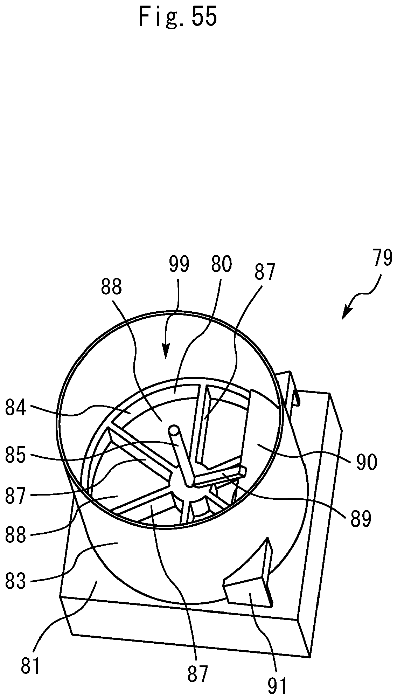

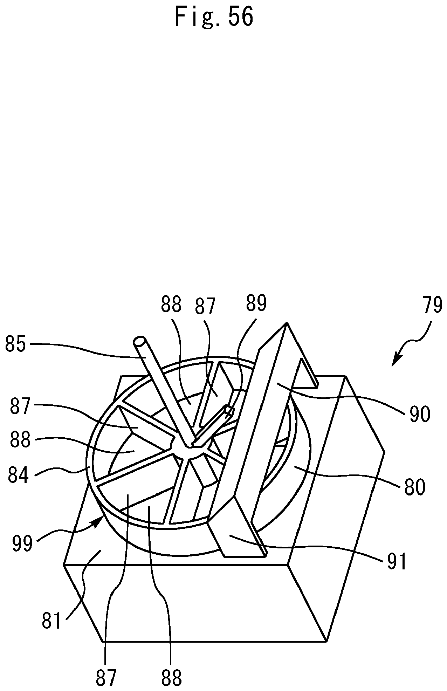

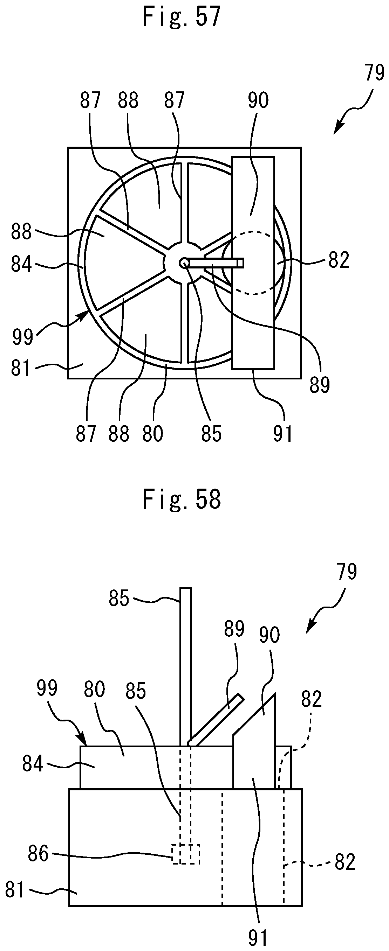

Figs. 55 and 56 are perspective views of an example of the chloride storage section 79 included in the hand-drying system 75 according to the sixteenth embodiment. Fig. 57 is a top view of the chloride storage section 79 shown in Fig. 56. Fig. 58 is a side view of the chloride storage section 79 shown in Fig. 56.

Hereinafter, the chloride storage section 79 shown in Figs. 55 to 58 will be described. The illustrated chloride storage section 79 includes the chloride adjustment means 99 suited for conveying chloride with a large grain size to the storage one grain at a time. For example, the chloride adjustment means 99 of the chloride storage section 79 includes a rotating section 80, an electric motor, a closed section 81, a discharge port 82, and a wall 83. Figs. 56 to 58 show a state where the wall 83 has been removed.

The electric motor is for rotating the rotating section 80. Illustration of the electric motor is omitted in Figs. 55 to 58. The electric motor rotates the rotating section 80 by a predetermined rotation amount. The electric motor may include a gear. For example, the electric motor may be a stepping motor. For example, the electric motor may be installed inside the closed section 81 or below the closed section 81.

The closed section 81 corresponds to a portion at a low position among the chloride storage section 79. The discharge port 82 is a hole provided in a lower part of the chloride storage section 79. An inner diameter of the discharge port 82 is larger than an outer diameter of a grain of chloride. The discharge port 82 is connected to the second supply path or the storage. Chloride conveyed from the discharge port 82 flows into the storage. The discharge port 82 corresponds to an opening provided in a lower part of the chloride storage section 79 or an opening provided in the closed section 81. The wall 83 is for preventing chloride from leaking to the outside of the chloride adjustment means 99.

The rotating section 80 includes an outer frame 84, a shaft section 85, a gear 86, a partition section 87, and a gap 88. The shaft section 85 corresponds to a shaft at a center of rotation of the rotating section 80. A central axis of the gear 86 of the rotating section 80 coincides with a central axis of the shaft section 85. The gear 86 of the rotating section 80 meshes together with the gear of the electric motor. A rotation of the gear of the electric motor is transmitted to the rotating section 80 by the gear 86 and the rotating section 80 rotates.

The outer frame 84 is at a position separated from the shaft section 85 in a radial direction of the rotating section 80. The partition section 87 provides a connection between the outer frame 84 and the shaft section 85. A shape of the outer frame 84 is, for example, a circular shape when viewed from a direction parallel to the shaft section 85. In the illustrated example, a plurality of partition sections 87 are provided in a radial pattern that is centered on the shaft section 85. A plurality of gaps 88 are formed by the outer frame 84, the shaft section 85, and the partition sections 87. The plurality of partition sections 87 are preferably arranged at regular intervals in terms of positions in a circumferential direction of the rotating section 80. Accordingly, a plurality of gaps 88 with mutually equivalent shapes can be defined.

For example, the number of the partition sections 87 and the number of the gaps 88 may range from around two to eight and more preferably from around four to six. One gap 88 has a space that allows entry of one gain of chloride. The number of the partition sections 87 may be appropriately set in accordance with a size of the grains of chloride. A variance in external shapes of the grains of chloride is desirably small to some extent.

A lower part of the outer frame 84 opposes a top face of the closed section 81. The discharge port 82 is provided at a position corresponding to an inner side of the outer frame 84, a lower side of the outer frame 84, and an outer side of the shaft section 85. In the illustrated example, a shape of the discharge port 82 is a circular shape when viewed from a direction parallel to the shaft section 85. When viewed from a direction parallel to the shaft section 85, an area of the discharge port 82 is desirably smaller than an area of the gap 88. The discharge port 82 may have a size that enables the discharge port 82 to be completely enclosed by the gap 88 when the gap 88 overlaps with the discharge port 82 when viewed from a direction parallel to the shaft section 85.

When granulated chloride is supplied from the second acceptance port to the chloride storage section 79, the granulated chloride falls freely and a grain of chloride is arranged in each of the gaps 88. When the number of grains of chloride is larger than the number of gaps 88, the grains of chloride may become stacked on the partition sections 87 or stacked on the grains of chloride arranged in the gaps 88.

When the rotating section 80 rotates due to drive of the electric motor, any of the gaps 88 is overlaid on the discharge port 82. An inner diameter of the gap 88 and an inner diameter of the discharge port 82 are larger than an outer diameter of a grain of chloride. Therefore, a grain of chloride arranged in the gap 88 overlaid on the discharge port 82 passes through the discharge port 82 and is conveyed to the storage. In this manner, by adjusting an amount of rotation of the rotating section 80 due to the electric motor, an amount of chloride conveyed to the storage can be adjusted.

When a specific gap 88 is positioned above the discharge port 82, after chloride in the specific gap 88 is discharged from the discharge port 82, until the rotating section 80 makes one rotation and the specific gap 88 once again becomes positioned above the discharge port 82, chloride arranged in another gap 88 is discharged from the discharge port 82. Therefore, chloride need only be placed in the specific gap 88 again while the rotating section 80 makes one rotation. Therefore, the possibility that chloride is not present in the gaps 88 is extremely low.

The hand-drying system 75 may automatically adjust an amount of chloride supplied to the storage by adjusting an amount of rotation due to the electric motor in accordance with a detection result of the chloride volume detection means 100. Alternatively, the hand-drying system 75 may automatically adjust an amount of chloride supplied to the storage by adjusting an amount of rotation due to the electric motor in accordance with a detection result of the concentration detection means 94 provided in the storage instead of the chloride volume detection means 100.

As shown in Fig. 55, the wall 83 is provided on an outer side of the outer frame 84. A part of the wall 83 is adjacent to an outer circumferential surface of the rotating section 80. An upper end of the wall 83 is at a position higher than an upper end of the outer frame 84. Since such a wall 83 is provided, even when chloride is moved by rotation of the rotating section 80, the chloride is not scattered to the outside of the outer frame 84.

The rotating section 80 may further include an organizing section 89. The organizing section 89 is arranged above the outer frame 84, above the partition sections 87, or above the gaps 88. The organizing section 89 rotates integrally with the rotating section 80. For example, the organizing section 89 protrudes from the shaft section 85 in an outer circumference direction. For example, the organizing section 89 has a rod-like or plate-like shape. The rotating section 80 may be provided with a plurality of organizing sections 89. The number of organizing sections 89 may be smaller than the number of partition sections 87. Providing the organizing sections 89 enables chloride to more reliably enter the gaps 88 one grain at a time. When the chloride storage section 79 is supplied with a large amount of chloride, there is a possibility that the chloride does not enter the gaps 88 and becomes clogged above the gaps 88. When the organizing section 89 rotates together with the rotating section 80, the chloride in the chloride storage section 79 is mixed by the organizing section 89. As a result, the chloride is smoothly placed in the gaps 88. Specifications of the organizing section 89 such as a shape, dimensions, and number may be appropriately adjusted so that chloride enters the gaps 88 in a suitable manner. Accordingly, reliability of chloride entering the gaps 88 is further improved.

The chloride adjustment means 99 may further include a penetration prevention section 90 and a penetration prevention support section 91. The penetration prevention section 90 may be provided in plurality. The penetration prevention section 90 is arranged above the discharge port 82. The penetration prevention section 90 is provided so as to cover at least a part of the discharge port 82 in a top view. The penetration prevention section 90 is for preventing a plurality of grains of chloride from penetrating into the discharge port 82 at one time. The penetration prevention section 90 is for preventing chloride from penetrating into the discharge port 82 from above the penetration prevention sections 90.

The penetration prevention section 90 is positioned above the partition section 87. The penetration prevention section 90 is positioned above the gap 88. The penetration prevention section 90 is provided as a separate member from the rotating section 80. In other words, the penetration prevention section 90 does not rotate even when the rotating section 80 rotates.

The penetration prevention section 90 is provided below the organizing section 89. When the rotating section 80 rotates, the penetration prevention section 90 desirably does not come into contact with the outer frame 84, the shaft section 85, the gap 88, the partition section 87, and the organizing section 89. The penetration prevention section 90 may be a rod-like member, a plate-like member, or an elastic body such as a spring.

The penetration prevention section 90 may prevent chloride positioned above the gap 88 or, in other words, chloride positioned so as to overlap with chloride arranged in the gap 88 from rotating and moving to a position above the discharge port 82. The penetration prevention section 90 may be shaped so as to move, to above the penetration prevention section 90, chloride positioned above the gap 88 or, in other words, chloride positioned so as to overlap with chloride arranged in the gap 88.

Providing the penetration prevention section 90 limits the number of grains of chloride positioned above the discharge port 82 and below the penetration prevention section 90 to one at the most. Accordingly, two or more grains of chloride can be more reliably prevented from being discharged from the discharge port 82 at one time.

If dimensions or the like are appropriately adjusted so that chloride is reliably positioned in the gap 88 in a state where an appropriate amount of chloride is stored in the chloride storage section 79, an amount of discharged chloride is proportional to an amount of rotation of the gear of the electric motor. Therefore, even if the sterilizer 76 does not include the chloride volume detection means 100 and the liquid volume detection means 93, the sterilizer 76 can adjust an amount of chloride discharged from the chloride storage section 79 with high accuracy.

A shape of the penetration prevention section 90 is preferably a shape that does not allow chloride to mount the penetration prevention section 90 in a stable manner. Enabling chloride to mount the penetration prevention section 90 in a stable manner gives rise to the possibility that chloride does not enter the gap 88 even when there is enough space for the chloride to enter the gap 88. Therefore, a frequency of supplying the chloride storage section 79 with chloride may increase. From such a perspective, the shape of the penetration prevention section 90 is preferably a cylindrical shape or a shape in which a top face of the penetration prevention section 90 is inclined. Accordingly, chloride is no longer able to mount the penetration prevention section 90 in a stable manner. When the penetration prevention section 90 is an elastic body such as a spring, flexure, oscillation, or the like of the penetration prevention section 90 occurs when chloride attempts to mount the penetration prevention section 90. This makes it difficult for chloride to mount the penetration prevention section 90 in a stable manner and is, therefore, preferable. In addition, when the penetration prevention section 90 is an elastic body such as a coil spring, since the penetration prevention section 90 has a spiral shape, it is more difficult for chloride to mount the penetration prevention section 90 and is, therefore, preferable.

When the penetration prevention section 90 is an elastic body, for example, when chloride comes into contact with the penetration prevention section 90 from above, the penetration prevention section 90 desirably has stiffness of such an extent that the penetration prevention section 90 deforms to some extent but does not deform to an extent to allow chloride to enter the discharge port 82.

Alternatively, when the penetration prevention section 90 is an elastic body, stiffness and a positional relationship of the penetration prevention section 90 may be set such that, for example, when chloride comes into contact with the penetration prevention section 90 from above, the penetration prevention section 90 deforms but the penetration prevention section 90 is supported by the partition sections 87 arranged below and the deformation of the penetration prevention section 90 is suppressed to prevent chloride from entering the discharge port 82.

In addition, when the penetration prevention section 90 is an elastic body, in the event that chloride arranged in the gap 88 is stacked, the elastic body biases the chloride downward when a center of the chloride is lower than the elastic body but the elastic body biases the chloride upward when the center of the chloride is above the elastic body. In other words, a structure may be adopted in which chloride correctly arranged in the gap 88 is discharged to the discharge port 82 and chloride stacked on chloride correctly arranged in the gap 88 is not discharged to the discharge port 82 until correctly arranged in the gap 88.

The penetration prevention support section 91 is for supporting the penetration prevention section 90. As shown in Fig. 55, for example, the penetration prevention section 90 is provided on an outer side of the wall 83. In this case, the penetration prevention section 90 is installed, the penetration prevention section 90 is placed, or the penetration prevention section 90 is inserted into a hole formed in the wall 83. Alternatively, the penetration prevention section 90 and the wall 83 may be integrated. Note that a shape of the organizing section 89 and a shape of the penetration prevention section 90 may be appropriately adjusted so that chloride mounted on the penetration prevention section 90 can be taken down by the organizing section 89.

As described above, the sterilizer 76 may generate electrolyzed water to become a sterilizing liquid by generating an aqueous chloride solution from supplied water and chloride supplied separately from the water and electrolyzing the aqueous chloride solution. As described above, since the sterilizer 76 can generate a sterilizing liquid, there is an advantage that the manager need not prepare the sterilizing liquid.

In the present disclosure, a liquid accumulated in the drain tank 9 may be referred to as "drain water". The drain water collects as a result of water droplets having been adhered to a hand or a sterilizing liquid passing through the drain outlet 8 and flowing into the drain tank 9. The hand-drying system 75 may include drain water detection means 98 for detecting drain water having accumulated in the drain tank 9.

The drain water detection means 98 may detect that an amount of drain water in the drain tank 9 has reached a predetermined threshold or has exceeded the threshold. The drain water detection means 98 may directly or indirectly detect that a predetermined amount of drain water has accumulated in the drain tank 9. "Directly detect" means, for example, the drain water detection means 98 detecting an amount of the drain water or, in other words, a volume of the drain water accumulated in the drain tank 9. "Indirectly detect" means, for example, the drain water detection means 98 detecting that a weight of the drain tank 9 or, in other words, a weight of the drain water accumulated in the drain tank 9 has reached or exceeded a predetermined weight. "Indirectly detect" means, for example, a sensor configured to detect a contact made with a liquid detecting a contact made with a liquid for a predetermined amount of time or longer. The predetermined amount of time is preferably, for example, one second or longer in order to prevent an erroneous detection due to vibration. As described above, the drain water detection means 98 may detect an amount of liquid, a liquid level of the liquid, a weight of the liquid, or the like accumulated in the drain tank 9. In addition, the drain water detection means 98 may detect a presence or absence of a weight, a distance, or a signal, a presence or absence of reception of a signal, or the like.

The hand-drying system 75 may include a drain water notification section for notifying a detection result of the drain water detection means 98. For example, the display section 4 may correspond to the drain water notification section or the terminal 103 to be described later may correspond to the drain water notification section. With a general hand dryer, as long as the hand dryer is cleaned in accordance with a frequency of use such as once a day or once a week as regular care of the hand dryer, drain water does not overflow from the drain tank 9. However, with the hand-drying system 75 including the sterilizer 76, a part of the sterilizing liquid discharged from the sterilizer 76 may flow into the drain tank 9 from the drain outlet 8 in addition to water removed from a hand. Accordingly, the drain water may accumulate in the drain tank 9 faster than an ordinary hand dryer. As a result, the drain tank 9 may become full. By detecting drain water with the drain water detection means 98 and notifying the manager with the drain water notification section, the drain water can be prevented from excessively accumulating in the drain tank 9 and from overflowing from the drain tank 9. Accordingly, the manager can appropriately manage the hand-drying system 75.

Note that the drain outlet 8 of the hand dryer may be configured to be connected to a flow path of a sewer instead of being connected to the drain tank 9. Accordingly, the sterilizing liquid discharged from the sterilizer 76 and water removed from a hand flow as-is from the drain outlet 8 into the sewer. Therefore, a large amount of the sterilizing liquid can be used at a location where the hand dryer is installed. For example, even in a case of a sterilizing liquid with a high safety level but low germicidal power, hygiene can be improved by using the sterilizing liquid in large amounts.

The liquid volume detection means 93, the water volume detection means 96, the drain water detection means 98, and the chloride volume detection means 100 each directly or indirectly detects that a volume of an object has reached a predetermined threshold or has exceeded the predetermined threshold. These detection means are not limited to means for detecting a volume of an object itself. These detection means may detect a volume of an object by any available principle. "Indirectly detect" corresponds to, for example, detecting a volume by applying conversion, provisional calculation, estimation, or approximation to an index or a value that differs from the volume without directly detecting the volume. These detection means may detect an object by detecting anything regarding a specific object such as a weight, a distance, a variation, a presence or absence of a signal, or a presence or absence of a reception of a signal. These detections means may perform detection using displacement of, for example, a load cell or a spring. The chloride volume detection means 100 may be means called a chloride detection section, the liquid volume detection means 93 may be means called a liquid detection section, and the water volume detection means 96 may be means called a water detection section.

The hand-drying system according to the present disclosure may include a plurality of hand dryers and may be configured such that the plurality of hand dryers cooperate with each other. For example, the hand-drying system 75 may independently determine hygienic conditions of the hand insertion section 3 of each hand dryer based on an irradiation time of the UV irradiator 23, a time of atomization of a sterilizing liquid by the sterilizer 76, or the like in each hand dryer. A configuration may be adopted in which each of the plurality of hand dryers includes the control section 22 and the control sections 22 of the respective hand dryers communicate with each other. Alternatively, a single control section 22 may be configured to control operations of the plurality of hand dryers.

The control sections 22 of the plurality of hand dryers may share information on the hygienic conditions of the hand insertion section 3 of each hand dryer. Alternatively, information on the hygienic conditions of the hand insertion section 3 of each hand dryer may be consolidated to the terminal 103 to be described later. In this case, the terminal 103 may transmit information related to a result of an analysis of the hygienic conditions of the hand insertion section 3 to each of the hand dryers. Due to the above, a hand dryer of which present hygienic conditions are most favorable can be discerned or a plurality of hand dryers of which present hygienic conditions are favorable can be discerned from the plurality of hand dryers.

Which hand dryer corresponds to the one or the plurality of hand dryers of which present hygienic conditions are favorable can be notified by the hygienic condition notification means described earlier. Alternatively, the hand-drying system 75 may use monitors or displays arranged in spaces where the hand dryers are arranged as hygienic condition notification means to notify which hand dryer is a hand dryer of which present hygienic conditions are favorable. Accordingly, since the user can readily comprehend which hand dryer is a hand dryer of which present hygienic conditions are favorable, the user can select and use a hand dryer of which present hygienic conditions are favorable. As a result, hygiene improves.

The hand-drying system which coordinates a plurality of hand dryers may control operation timings of the UV irradiators 23 of the respective hand dryers so that the UV irradiators 23 of all of the hand dryers do not emit ultraviolet light at the same time. For example, when there is a hand dryer that is currently emitting ultraviolet light from the UV irradiator 23 among the plurality of hand dryers, the hand-drying system which coordinates the plurality of hand dryers may perform control so that ultraviolet light is not emitted from the UV irradiator 23 of at least one of the other hand dryers. Accordingly, since an occurrence of an event where all of the hand dryers become unusable at the same time can be prevented, convenience for the user is improved.

In the present disclosure, a person, a vendor, a company, or the like who manages or performs maintenance of a hand dryer, a hand-drying system, or the sterilizer 76 may be simply described as a "manager". In the present disclosure, the hand-drying system 75 or a hand dryer may cooperate with the terminal 103 used by the manager by communicating with the terminal 103. The terminal 103 may have a display for displaying data or information. The terminal 103 may have an operating section which can be operated by a person corresponding to a manager. The terminal 103 may be configured such that, by having a person corresponding to a manager operate the terminal 103 and externally input information to the hand dryer or the sterilizer 76, the person can operate or control the hand dryer or the sterilizer 76.

For example, the terminal 103 may be constituted by a mobile terminal such as a smartphone or a tablet or configured as a display of a personal computer. The terminal 103 may be installable so as to be freely detachable from a hand dryer.

In the following description, detection results of the chloride volume detection means 100, the liquid volume detection means 93, the water volume detection means 96, the drain water detection means 98, the pH detection means 95, the concentration detection means 94, or other detection means, measurement results of other measurement sections, information related to hygienic conditions of the hand insertion section 3, data regarding timings of a forcible termination in the sterilizing step or the drying step, information related to the UV irradiator 23, information related to the hand dryer, and information related to the sterilizer 76 may be collectively referred to as "various kinds of data". The terminal 103 may display data or information corresponding to at least one kind of data among the various kinds of data on a display.

Data regarding timings of a forcible termination in the sterilizing step or the drying step is data regarding at what timing a hand has been extracted from the hand insertion section 3. For example, information related to the UV irradiator 23 may include at least one piece of information among a total lighting time of the light source 24, an hourly lighting time of the light source 24, a daily lighting time of the light source 24, a monthly lighting time of the light source 24, and a yearly lighting time of the light source 24.

The terminal 103 may acquire the various kinds of data via a communication interface device and display the data on a display. The terminal 103 may regularly acquire the various kinds of data. In this case, "regularly" includes extremely short intervals such as intervals of less than one second.

For example, the hand dryer or the sterilizer 76 can transmit various kinds of data to an external terminal 103 via a communication interface device and cause the data to be displayed on a display of the terminal 103. Accordingly, for example, a third party such as a manager who manages the hand dryer or the sterilizer 76 can confirm various kinds of data related to the hand dryer or the sterilizer 76 even from outside of a space such as a room that includes the hand dryer.

The terminal 103 can acquire or confirm various kinds of data with respect to a plurality of hand dryers or a plurality of sterilizers 76. In addition, the terminal 103 capable of acquiring or confirming various kinds of data with respect to a plurality of hand dryers or a plurality of sterilizers 76 may be provided in plurality. Accordingly, the various kinds of data can be more readily managed and utilized.

In the example described above, the manager can comprehend the various kinds of data by proactively confirming the display of the terminal 103 when necessary. In addition to such an example, the hand-drying system 75 may include notification means for more reliably notifying, using the terminal 103, the manager of information related to the various kinds of data. The notification means may notify the manager when any of the values of the various kinds of data reaches a predetermined threshold, exceeds the predetermined threshold, or falls below the predetermined threshold. The notification means may notify the manager using one of or a combination of two or more of a sound, a vibration, a display, a light, and the like generated by the terminal 103. For example, when the total lighting time of the light source 24 of the UV irradiator 23 exceeds a predetermined amount of time, the terminal 103 may notify the manager using the notification means. Notification information may be transmitted from the hand dryer or the sterilizer 76, the terminal 103 may receive the notification information, and the notification means may perform notification. Alternatively, the terminal 103 may receive various kinds of data from the hand dryer or the sterilizer 76, and the notification means may perform notification in accordance with a determination made by the terminal 103 based on the various kinds of received data.

The hand dryer or the sterilizer 76 may include a reception section for accepting an input from outside. The reception section includes an external input acceptance section that accepts external input. The reception section accepts input from outside according to, for example, an existing technique. For example, a method of receiving the input may involve using radio, infrared light, a sensor, or the like. For example, the hand dryer or the sterilizer 76 may perform, by having the external input acceptance section of the reception section accept an input from outside, switching between on/off states of the switch of the electrolyzed water generation means 92, switching between on/off states of the continuous operation switching section, switching between on/off states of detection switching means or, in other words, switching between on/off states of the sensing function of the hand detection section 21 or the human detection means, switching between supply and suspension of supply of chloride from the chloride storage section 79 to the storage, switching among operations of the blower 10, and detection by the various detection sections described earlier. According to such a configuration, various operations of the hand dryer or the sterilizer 76 can be controlled according to external input.

In addition, the terminal 103 can also provide a plurality of hand dryers or a plurality of the sterilizers 76 with external input and cause the external input acceptance sections of the plurality of hand dryers or the plurality of the sterilizers 76 to accept input from outside. Accordingly, the plurality of hand dryers or the plurality of the sterilizers 76 can be more readily handled.

Furthermore, based on various kinds of data acquired by the terminal 103, external input may be automatically performed to the hand dryer or the sterilizer 76 and the external input acceptance section of the hand dryer or the sterilizer 76 may accept input from outside.

In addition, based on various kinds of data regarding a plurality of hand dryers or a plurality of the sterilizers 76 acquired by the terminal 103, external input may be automatically performed to the plurality of hand dryers or the plurality of the sterilizers 76 and the external input acceptance sections of the plurality of hand dryers or the plurality of the sterilizers 76 may accept input from outside.

Accordingly, for example, when there are a space in which a plurality of hand dryers are arranged, the user may be guided to a hand dryer with higher hygiene due to drive by the UV irradiator 23 or the sterilizer 76. For example, the user may be guided to a hand dryer with a longer amount of time having elapsed from its last use and with higher hygiene. As a method of guiding the user, display enabling the user to understand that hygiene is high may be output to the display section 4 of the hand dryer with higher hygiene. Alternatively, information indicating which hand dryer should be used may be displayed on a display, a monitor, or the like installed in a space where a plurality of hand dryers are present. The display, the monitor, or the like includes a reception section. The display, the monitor, or the like is capable of displaying the display contents described above in accordance with information automatically sent from the terminal 103.

Each function of the hand dryer or the sterilizer 76 may be achieved by a processing circuit. For example, a processing circuit for performing control for causing the terminal 103 to display data acquired by each detection means may be provided. The processing circuit may be an exclusive hardware product or a processing circuit including a CPU (Central Processing Unit) for executing a program stored in a memory.

Note that, among the plurality of embodiments described above, two or more combinable embodiments may be implemented in combination with each other.

### Reference Signs List

1...hand dryer, 2... main body enclosure, 2a...front cover, 2b...rear cover, 2c...side cover, 3...hand insertion section, 3a... insertion bottom section, 3b...insertion front section, 3c... insertion rear section, 3d... insertion side section, 3e...front projecting section, 3f...rear projecting section, 3g...insertion left section, 3h... insertion right section, 3i...crest section, 3j...opening, 3k...boss, 3m...projecting section, 3n...arrangement section, 3p...arrangement surface, 3q...arrangement side wall section, 3r... insertion upper section, 4...display section, 5...hand insertion space, 6...air hole, 7...hand insertion opening, 8...drain outlet, 9...drain tank, 10...blower, 11...motor, 12...turbo fan, 13...blowout air path, 14...front blowout air path, 15...rear blowout air path, 16...air inlet, 17...filter section, 18...inlet air path, 19...first blowout port section, 20... second blowout port section, 21... hand detection section, 21a... electrode, 21b... electrode, 21c...electrode, 21d...electrode, 22...control section, 23...UV irradiator, 24...light source, 25...case, 26...windows, 26a...first window, 26b...second window, 27...substrate, 28...heat sink, 29...spacer, 30...sealing member, 31...fixing member, 32...wiring, 33...screw, 34... bushing, 35...two-sided adhesive tape, 36...reflection section, 36a... reflection surface, 36b... rear surface, 36c...reflection side wall section, 37...assembly, 37a... assembly bottom section, 37b... assembly front section, 37c... assembly rear section, 37g... assembly left section, 37h... assembly right section, 37i...opening, 38... assembly sheet, 38a...protruding main body section, 38b...convex shape section, 38c...recessed main body section, 38d...concave shape sections, 38e...position, 39...human detection section, 40...hand dryer, 41...suction port, 42... suction air path, 43...first water droplet removing means, 44... second water droplet removing means, 44a...plate-like members, 45... hand dryer, 46... shielding body, 46a... rotary shaft, 46b...wall section, 46c...support section, 47... hand dryer, 48 ... first shielding body, 48a... wall section, 48b... gear section, 48c...support section, 49...second shielding body, 49a...wall section, 49b... gear section, 49c...support section, 50...hand dryer, 51...shielding body, 51a... wall section, 51b...hinge section, 51c...projecting section, 52...hand dryer, 53...shielding body, 53a...rotary shaft, 54...hand dryer, 55...shielding body, 55a...wall section, 55b...hinge section, 56...pedal, 57...mover, 57a...lever, 57b... push rod, 57c...fulcrum, 58...hand dryer, 59... hand dryer, 60...human recognition section, 61... heater, 62... hand dryer, 63... second light source, 64... hand-drying system, 65...presence/absence detection means, 66... hand-drying system, 67... second light source, 68... hand-drying system, 69... switching section, 70...proximity detection means, 71... hand-drying system, 72... human recognition means, 73... hand-drying system, 74... human count detection means, 75... hand-drying system, 76... sterilizer, 77... discharge means, 78... chloride supplying section, 79... chloride storage section, 80...rotating section, 81... closed section, 82... discharge port, 83...wall, 84...outer frame, 85...shaft section, 86...gear, 87...partition section, 88...gap, 89... organizing section, 90...penetration prevention section, 91...penetration prevention support section, 92... electrolyzed water generation means, 93...liquid volume detection means, 94... concentration detection means, 95 ...pH detection means, 96...water volume detection means, 97...water volume adjustment means, 98... drain water detection means, 99... chloride adjustment means, 100... chloride volume detection means, 101...processor, 102...memory, 103...terminal, 421...suction air path, 422...suction air path, 423...suction air path

## Claims

1. A hand dryer comprising:
a hand insertion section defining a hand insertion space and having an air hole that opens to the hand insertion space;
a blower in fluid communication with the air hole, the blower being configured to blow air from the air hole into the hand insertion space or configured to suck air from the hand insertion space into the air hole; and
a UV irradiator including a light source, the UV irradiator being configured to irradiate the hand insertion space with ultraviolet light produced by the light source.

2. The hand dryer according to claim 1, wherein the blower is configured to blow air from the air hole into the hand insertion space.

3. The hand dryer according to claim 2, further comprising
a suction port that opens to the hand insertion space, wherein
the blower is configured to suck air from the hand insertion space into the suction port.

4. The hand dryer according to any one of claims 1 to 3, further comprising:
one or both of hand detection means for detecting a hand inserted into the hand insertion section or a hand placed on the hand insertion section and human detection means for detecting a human body having approached the hand dryer; and
output change means configured to reduce an output of the light source or change the output of the light source to zero in accordance with a detection by the hand detection means or a detection by the human detection means.

5. The hand dryer according to claim 4, wherein the output change means is configured to reduce an output of the light source or change the output of the light source to zero when a duration of a state where the hand detection means does not detect a hand or a duration of a state where the human detection means does not detect a person exceeds a criterion.

6. The hand dryer according to claim 4, comprising both the hand detection means and the human detection means, wherein
the output change means is configured to reduce an output of the light source or change the output of the light source to zero when a duration of a state where the hand detection means does not detect a hand and the human detection means does not detect a person exceeds a criterion.

7. The hand dryer according to any one of claims 1 to 6, wherein the hand dryer is configured to reduce an output of the light source or change the output of the light source to zero when a duration of emission of the ultraviolet light by the UV irradiator exceeds a criterion.

8. The hand dryer according to any one of claims 1 to 7, further comprising irradiation notification means for notifying, when the UV irradiator is emitting the ultraviolet light, emission of the ultraviolet light by the UV irradiator.

9. The hand dryer according to any one of claims 1 to 8, further comprising hygienic condition notification means for notifying hygienic conditions of the hand insertion section.

10. The hand dryer according to any one of claims 1 to 9, wherein the light source is a light-emitting diode capable of generating ultraviolet light with a wavelength in a UVC range.

11. The hand dryer according to any one of claims 1 to 10, wherein the light source is a light-emitting diode, and
a wavelength with highest radiant intensity among light generated by the light source ranges from 220 nm to 280 nm.

12. The hand dryer according to any one of claims 1 to 11, further comprising
a window which prevents a hand from coming into contact with the light source, wherein
the hand insertion space is irradiated with the ultraviolet light from the light source through the window, and
the window is detachable.

13. The hand dryer according to any one of claims 1 to 11, wherein the UV irradiator further includes a window which prevents a hand from coming into contact with the light source, wherein
the hand insertion space is irradiated with the ultraviolet light from the light source through the window, and
the UV irradiator is detachable.

14. The hand dryer according to any one of claims 1 to 11, wherein the UV irradiator further includes a substrate on which the light source is installed, a window which covers the light source from an opposite side to the substrate and which transmits the ultraviolet light, and a spacer arranged between the substrate and the window, wherein
the spacer is configured to reflect at least light of which a wavelength has highest radiant intensity among light generated by the light source.

15. The hand dryer according to any one of claims 1 to 11, further comprising:
a first window which covers the light source; and
a second window which covers the first window, wherein
the light source, the first window, and the second window are arranged on a straight line.

16. The hand dryer according to any one of claims 1 to 15, wherein
the light source generates visible light together with the ultraviolet light, and
a color of the hand insertion section or a color of light emitted from the UV irradiator when the light source is turned on differs from a color of the hand insertion section or a color of light emitted from the UV irradiator when the light source is turned off.

17. The hand dryer according to any one of claims 1 to 16, further comprising
a main body enclosure which defines an outer appearance of the hand dryer, wherein
the light source is covered by the main body enclosure in any of a front view, a rear view, a side view, a top view, and a bottom view in the outer appearance.

18. The hand dryer according to any one of claims 1 to 17, further comprising
a main body enclosure which defines an outer appearance of the hand dryer, wherein
the light source is covered by the main body enclosure even when the hand dryer is viewed from any direction perpendicular to a vertical line.

19. The hand dryer according to any one of claims 1 to 18, wherein at least a part of a surface facing the hand insertion space has a reflectance of 70% or higher with respect to light of which a wavelength has highest radiant intensity among light generated by the light source.

20. The hand dryer according to any one of claims 1 to 19, wherein
a surface facing the hand insertion space has a first region and a second region,
the first region has a reflectance of 70% or higher with respect to light of which a wavelength has highest radiant intensity among light generated by the light source, and
the second region has a reflectance of 30% or lower with respect to light of which a wavelength has highest radiant intensity among light generated by the light source.

21. The hand dryer according to any one of claims 1 to 20, further comprising
a reflection section provided so as to face the hand insertion space, wherein
the reflection section is made of a fluorine resin or a metal.

22. The hand dryer according to any one of claims 1 to 21, further comprising
a reflection section provided so as to face the hand insertion space, wherein
a reflection surface of the reflection section is on a same plane as a surface of the hand insertion section in a periphery of the reflection section or recessed with respect to the surface of the hand insertion section in a periphery of the reflection section.

23. The hand dryer according to any one of claims 1 to 22, further comprising
a reflection section provided so as to face the hand insertion space, wherein
an assembly formed by three-dimensionally assembling a sheet is provided as the reflection section.

24. The hand dryer according to any one of claims 1 to 23, further comprising a shielding body, wherein
the hand dryer is configured to be capable of switching between a first state and a second state,
the second state is a state where the shielding body covers at least a part of a hand insertion opening to be an inlet of a hand into the hand insertion space, and
the first state is a state where a ratio being covered by the shielding body among the hand insertion opening is either smaller than in the second state or zero.

25. The hand dryer according to claim 24, further comprising output change means configured to reduce an output of the light source or change the output of the light source to zero when a switch is made from the second state to the first state.

26. The hand dryer according to claim 24 or 25, further comprising:
a pedal; and
a mover which moves the shielding body so as to be switched from the second state to the first state when the pedal is stepped on with a foot and which moves the shielding body so as to be switched from the first state to the second state when the foot on the pedal is removed.

27. The hand dryer according to any one of claims 24 to 26, wherein the shielding body is provided as a detachable attachment.

28. The hand dryer according to any one of claims 1 to 27, further comprising
a main body enclosure which defines an outer appearance of the hand dryer, wherein
the light source and the UV irradiator are covered by the main body enclosure so that the light source and the UV irradiator are not visually recognized when the hand dryer is viewed by gazes in all directions in a three-dimensional space from outside of the main body enclosure.

29. The hand dryer according to any one of claims 1 to 28, further comprising
a main body enclosure which defines an outer appearance of the hand dryer, wherein
the light source and the UV irradiator are covered by the main body enclosure so that there is no light beam which directly exits the main body enclosure from the light source when it is assumed that light beams are emitted in all directions in a three-dimensional space from the light source or the UV irradiator.

30. The hand dryer according to any one of claims 1 to 29, comprising
hand detection means for detecting a presence or absence of a hand inserted into the hand insertion section or a hand placed on the hand insertion section, wherein
the UV irradiator is configured to emit the ultraviolet light when it is detected that the hand is absent.

31. The hand dryer according to any one of claims 1 to 30, comprising
a reception section which receives a signal for controlling on/off states of emission by the UV irradiator.

32. The hand dryer according to any one of claims 1 to 31, wherein
the light source is a first light source,
the hand dryer comprising:
a second light source having a dominant wavelength which is longer than a dominant wavelength of the first light source; and
hand detection means for detecting a hand inserted into the hand insertion section or a hand placed on the hand insertion section, and
the hand dryer is configured to turn on the second light source without turning on the first light source when the hand detection means detects a hand.

33. The hand dryer according to any one of claims 1 to 32, comprising:
hand detection means for detecting a hand inserted into the hand insertion section or a hand placed on the hand insertion section; and
a non-insertion time drive mode which drives the blower when the hand detection means has not detected a hand, wherein
the non-insertion time drive mode is an operation which produces an air volume or an air speed equivalent to a minimum air volume or a minimum air speed when driving the blower in order to dry a hand or an operation which produces an air volume or an air speed which is smaller or lower than the minimum air volume or the minimum air speed.

34. The hand dryer according to any one of claims 1 to 33, wherein
the hand insertion section includes an insertion side section, and
the hand dryer comprises insertion side section adjustment means for adjusting a height of an upper end of the insertion side section.

35. A hand-drying system, comprising:
the hand dryer according to any one of claims 1 to 34 being arranged in a user region that is a region which a user enters; and
presence/absence detection means for detecting whether or not a person is present in the user region.

36. The hand-drying system according to claim 35, wherein the UV irradiator is configured to emit the ultraviolet light when it is detected that a person is not present in the user region.

37. The hand-drying system according to claim 35 or 36, wherein the hand-drying system is configured to reduce an output of the light source of the UV irradiator or change the output of the light source to zero when it is detected that a person is present in the user region.

38. The hand-drying system according to any one of claims 35 to 37, wherein the hand-drying system is configured to reduce an output of the light source or change the output of the light source to zero when a duration of emission of the ultraviolet light by the UV irradiator exceeds a criterion when it is detected that a person is not present in the user region.

39. The hand-drying system according to any one of claims 35 to 38, wherein
the light source is a first light source,
the hand dryer comprises a second light source having a dominant wavelength which is longer than a dominant wavelength of the first light source, and
the hand-drying system is configured to turn off the first light source and turn on the second light source when a duration of lighting of the first light source exceeds a criterion when it is detected that a person is not present in the user region.

40. The hand-drying system according to any one of claims 35 to 39, wherein the presence/absence detection means detects whether or not a person is present in the user region by detecting at least one of a brightness of the user region and an on/off state of an illuminator which illuminates the user region.

41. The hand-drying system according to any one of claims 35 to 39, wherein
the presence/absence detection means detects that a person is present in the user region when a brightness of the user region is higher than a threshold but detects that a person is not present in the user region when the brightness of the user region is lower than the threshold, and
the hand-drying system comprises threshold adjustment means capable of adjusting the threshold.

42. A hand-drying system, comprising:
the hand dryer according to any one of claims 1 to 23;
a cover section capable of covering at least one face of the hand insertion space of the hand dryer;
a switching section capable of switching between a closed state where the cover section covers the at least one face of the hand insertion space and an open state where a hand can be inserted into the hand insertion space; and
proximity detection means capable of detecting that a hand or a person is in proximity to the hand insertion section in the closed state,
the hand-drying system being configured to enter the open state when the proximity detection means detects that a hand or a person is in proximity to the hand insertion section.

43. A hand-drying system, comprising:
the hand dryer according to any one of claims 1 to 34 being arranged in a user region being a region which a user enters; and
human count detection means for detecting a number of people in the user region,
the UV irradiator being configured to emit the ultraviolet light when the number of people is zero,
the hand-drying system being configured to stop emission of the ultraviolet light by the UV irradiator when the number of people is one or more.

44. A hand-drying system, comprising:
the hand dryer according to any one of claims 1 to 34;
human recognition means for recognizing a same user;
human storage means for counting a use frequency by a same user; and
output variable means which changes an output of emission of the ultraviolet light by the UV irradiator in accordance with a daily use frequency by a same user.

45. A hand-drying system, comprising:
the hand dryer according to any one of claims 1 to 34; and
a sterilizer which discharges a sterilizing liquid being a liquid with a sterilization effect.

46. The hand-drying system according to claim 45 performing, in this order: a drying step of drying a hand by driving the blower; and a sterilizing step of discharging the sterilizing liquid from the sterilizer so that the sterilizing liquid adheres to the hand.

47. The hand-drying system according to claim 45 capable of performing a drying step of drying a hand by driving the blower and a sterilizing step of discharging the sterilizing liquid from the sterilizer so that the sterilizing liquid adheres to the hand, wherein
the hand-drying system performs the sterilizing step for a first time, the drying step for a first time, the sterilizing step for a second time, and the drying step for a second time in this order.

48. The hand-drying system according to any one of claims 45 to 47, wherein the sterilizer generates the sterilizing liquid by generating an aqueous chloride solution from supplied water and chloride supplied separately from the water and electrolyzing the aqueous chloride solution.

49. The hand-drying system according to claim 48, wherein the sterilizer includes a storage capable of storing water and a chloride supplying section which automatically supplies the chloride to the storage.

50. The hand-drying system according to any one of claims 45 to 49, wherein
the sterilizer is capable of discharging the sterilizing liquid by atomization, and
the hand-drying system is capable of operating so as to convey the sterilizing liquid discharged by atomization by an airflow generated by the blower.

51. The hand-drying system according to any one of claims 45 to 50, comprising:
a drain tank for storing water removed from a hand and the sterilizing liquid discharged from the sterilizer; and
drain water detection means for detecting drain water being a liquid accumulated in the drain tank.

52. A hand-drying system, comprising
the hand dryer according to any one of claims 1 to 34 in plurality,
the hand-drying system cooperatively operating the plurality of hand dryers.

53. A hand-drying system, comprising:
the hand dryer according to any one of claims 1 to 34; and
a terminal used by a manager who manages the hand dryer,
the hand-drying system transmitting data detected by the hand dryer to the terminal.
